# EUROPEAN PATENT SPECIFICATION

(11) **EP 2 682 097 B1**
(45) Date of publication and mention of the grant of the patent: **05.10.2016**
(21) Application number: 13175077.0
(22) Date of filing: 04.07.2013
(51) Int. Cl.: A61K 9/00, A61K 9/14, A61K 31/40, A61K 31/439, A61K 31/4704

(54) **Dry Powder Inhalers Comprising A Carrier Other Than Lactose**
Trockenpulverinhalatoren mit einem anderen Träger als Lactose
Inhalateurs de poudre sèche comprenant un support autre que le lactose

(30) Priority: 05.07.2012 TR 201207842
(43) Date of publication of application: 08.01.2014
(73) Proprietor: Arven Ilac Sanayi Ve Ticaret A.S., Istanbul 34460 (TR)
(72) Inventor: Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- EP-A1- 1 944 018
- EP-A1- 2 100 599
- EP-A1- 2 191 821
- WO-A1-2005/097126
- WO-A1-2008/000482
- WO-A1-2012/106575

## Description

### Field of the Invention

This invention relates to novel pharmaceutical compositions for inhalation comprising separately, sequentially or together, drugs having amine in the form of a dry powder in admixture with a pharmaceutically acceptable carrier, other than lactose, and its use in the treatment of respiratory condition selected from asthma, chronic obstructive pulmonary disease (COPD) and other obstructive airways diseases.

In addition, the present invention relates to novel pharmaceutical compositions for inhalation based on combinations of long acting muscarinic antagonists, long acting beta agonists, short acting beta-2 agonists, corticosteroids or a combination of two or more of them with drugs having amine.

### Background of the Invention

Amines are organic compounds and functional groups that contain basic nitrogen atom with alone pair. Amines are derivatives of ammonia, wherein one or more hydrogen atoms have been replaced by a substituent such as an alkyl or aryl group. Therefore drugs having amines can be selected from the group comprising aclidinium, glycopyrronium, daratropium, indacaterol, vilanterol, carmeterol, olodaterol or pharmaceutically acceptable salts, or esters thereof, or in enantiomerically pure form or as a racemic mixture.

Most dry powder inhaler (DPI) formulations rely on lactose as a carrier. However, lactose cannot be used for compounds that interact with the reducing sugar function of the lactose. Such drugs are the ones having amine groups, as described above, especially the ones having primary or secondary amine.

Another disadvantage can be the Maillard reaction which results from a chemical reaction between an amine group and a reducing sugar. Water content (humidty) and temperature are found to influence the degradation.

Thus, there is a need in the art to look for alternative carriers, other than lactose, that still possess the positive aspects but overcome the above mentioned disadvantages of lactose.

Other sugars may comprise but not limited to mannitol, glucose, trehalose, cellobiose, sorbitol and maltitol as potential carriers; especially it is mannitol.

In prior art, there are several patents and compounds used for the treatment of asthma but all these compounds include lactose as a carrier. None of them comprise mannitol as carrier.

Additionally, carriers are used as a flow aid and facilitate the dose of the active into the lungs. Therefore the properties of the particles of the carrier play an important role in the formulation of dry powder inhaler (DPI). Thus, carriers should be carefully selected, designed and controlled for the use in a dry powder inhalation formulation.

Accordingly, formulations of dry powder Inhalers (DPI) must fulfill a set of requirements, whereby in particular the following are to be considered:

### content uniformity of the active drug:

In a single dose system, each capsule or blister needs to contain the same amount of drug. In a multi dose system, the same amount of drug must be released every time it is administered, to guarantee that the patient receives the same dose each time. The presence of carrier, should promote the content uniformity even in a low-dosage medication.

### flowability:

The design of the device, the characteristics of the active and the filling platform to be used will determine the appropriate characteristics of the carrier that will be needed. The flow properties of the formulation will be important to ensure that the overall device functions in the correct way and provides consistent performance. The choice of carrier is essential in ensuring that the device works correctly and delivers the right amount of active to the patient. Therefore to use mannitol as a carrier in two different particle sizes (fine and coarse) is essential.

### dose consistency:

DPI devices have to show a consistent dose uniformity in order to guarantee that all doses from the device contain the correct quantitiy of the active. Regardless of a patient's inhalation ability, it is essential that the dose released by the DPI device is exactly the same every time. Therefore, using mannitol as a carrier with the right properties in the formulation assists dose-consistent delivery.

Accordingly, in order to penetrate into the deep lungs the active particles will have a particle size less than 10 microns and often lower than 4 microns. These small drug particles will have a tendency to agglomerate. By using the appropriate carrier (such as mannitol) this drug to drug agglomeration can be prevented. Furthermore, the carrier (such as mannitol) will help to control the flowability, the drug release from the device and helps to ensure the correct and consistent dosage of the active that reaches the lungs.

Additionally, the formulation should be a homogeneous mixture where the drug particles adhere to the carrier. The adhesion should not be too strong as the drug will not be able to release from the carrier particle during inhalation. Furthermore, a low dose of powder should be filled into the device and the drug should always be released in the same way. One of the main important parameters for the formulation is the particle size of the carrier. Therefore, it is found that using the right ratio of the fine (small) and coarse (large) particles of the selected carrier in the present formulations of the invention is essential.

To fulfill all these requirements the formulations of DPIs needs to be adapted in particular by a careful selection of the carriers used. In order to meet these requirements, the inhalable, fine or microfine particles of active compounds are mixed with carriers. By means of the mixing process, the particle size of the carrier can also be changed such that a certain proportion is inhalable. The particle size of the carrier employed depends on the requirements and specifications of the powder inhaler which is intended for the administration of the formulation. It is true for these mixtures that during all required processing, transport, storage and dosage operations no segregation must take place, i.e. the active compound particles must not detach from their carrier particles. During dispersion in the inhaler, induced by the respiratory flow of the patient, the active compound particles, however, must be detached as effectively as possible, i.e. as quantitatively as possible, in order to be inhaled.

Thus, there is still a need for carriers that are able to overcome the problems mentioned above and the problems related with interaction of carrier between the drugs having amine and furthermore the problems related to pulmonary administration of drugs. This invention also proposes the possibility to obtain different compositions and composition of combinations for pulmonary administration having satisfactory properties in terms of increasing drug deposition or accelerating drug release rate in a safe and effective way.

### The Detailed Description of the Invention

This invention relates to novel pharmaceutical compositions for inhalation comprising separately, sequentially or together, drugs having amine in the form of dry powder in admixture with a pharmaceutically acceptable carrier, other than lactose which doesn't interact with the drugs especially having an amine group. The present invention further relates its use in the treatment of respiratory condition selected from asthma and chronic obstructive pulmonary disease (COPD) and other obstructive airways diseases.

Accordingly, the main object of the present invention is to provide pharmaceutical compositions for inhalation which is stable throughout the shelflife, in other words, which prevents any chemical reaction between an amine group and reducing sugar which may cause degredation of the active and furthermore resistant to humidty and temperature which may occur during the manufacturing process.

Another main object of the present invention is to provide pharmaceutical compositions for inhalation having an adequate content uniformity of the active in order to guarantee that the patient receives the same dose each time even in low-dosage formulations.

Another object of the present invention is to obtain the dose consistency of the active in order to guarantee that all doses from the device contain the correct quantitiy of the active. Using the carrier with the right properties and the right ratio in the formulation asissts dose-consistent delivery. According to this preffered embodiment, the weight ratio of the fine carrier particles to coarse carrier carrier particles, is between 0.01 - 0.25 by weight, preferably it is between 0.05 - 0.20 by weight.

According to a further embodiment of the invention, the fine carrier particles of the said pharmaceutically acceptable carrier have a particle diameter of d₁₀ between 1.0 - 4.0 µm, d₅₀ between 4.0 - 7.0 µm and d₉₀ between 7.0 - 15.0 µm. The coarse carrier particles of the said pharmaceutically acceptable carrier have a particle diameter of d₁₀ between 10.0 - 50.0 µm, d₅₀ between 50.0 - 75.0 µm and d₉₀ between 75.0 - 250.0 µm.

The coarse carrier particles are used to prevent (re)agglomeration of the fine particles of active. To provide this effect, carrier with a particle size of approximately ten times that of the active is used. Generally, a monolayer of the active particles is formed on the larger carrier particles. Since the active and carrier will have to be separated during inhalation, the shape and the surface roughness of the carrier particles is of significant importance. Carrier particles with a smooth surface will separate from the active more easily than highly porous particles of equal size.

The fine carrier particles are used to help the active to reach the lungs in a safer way and higher doses. Because the surface energy is normally not equally spread over the carrier particle, the active will tend to concentrate on higher energy sites. This can make separation of the active from the carrier following pulmonary delivery more difficult, especially for low dose formulations. The presence of fine carrier paticles, smaller than 10.0 micron or 5.0 micron, will help to prevent this, as the high energy sites will be occupied by the fine carrier particles and the active will tend to attach to the low energy sites. It is found that lung deposition will increase with an increasing fraction of fine carrier particles. Accordingly a reduction in particle size (having finer particles) increases the fluidization energy and this enhances the increase of the amount of drug particles that will get into the lung.

In this invention, the term "fine particles" means the fraction of active particles being less than 5.0µm when assessed by a cumulative volume size distrubition as tested by any conventionally accepted method such as the laser diffraction method.

The drug particles will then adhere to the lower adhesion sites and will be easier released during inhalation. With the addition of fines also the surface area increases significantly and the payload will be reduced. When the fine carrier particles are slightly coarser then the drug particles it could eliminate the friction forces between drug and carrier/mannitol in the mixing process.

Another object of the present invention is to obtain good flowability of the formulations to ensure that the right amount of the active is delivered by the devices for DPIs. In other words, in order to guarantee consistent production of the formulations, mechanical filling of the powder inhaler, correct dosage and release by the powder inhaler the present invention provides free-flowing formulations by selecting the right carrier.

Another object of the present invention is to prevent agglomeration by using appropriate carrier, other than lactose. Active particles have fine or sometimes microfine particles in order to penetrate into the deep lungs. Thus, these small drug particles will have a tendency to agglomerate.

In a preferred embodiment according to the present invention, the pharmaceutically acceptable carrier, other than lactose, is selected from the group comprising mannitol, glucose, trehalose, cellobiose, sorbitol, maltitol or a combination of two or more of them.

As a further embodiment, the carrier may be a combination of mannitol and glucose, or mannitol and trehalose, or mannitol and sorbitol, or mannitol and cellobiose, or mannitol and maltitol.

In a more preffered embodiment, the invention suggests to use mannitol as a carrier, more specifically to use spray-dried mannitol to achieve the best results.

In an ideal drug-carrier system, the adhesion of the active to the carriers is strong enough to prevent demixing during filling, handling and storage, but not so strong, since the active and carrier will have to be separated during inahalation. Therefore the shape and the surface roughness of the carrier particles is of significant importance. It is found that spray-dried mannitol particles will separate from the active more easily than highly porous particles of equal size. Because spray dried mannitol produces more spherical particles and a smooth surface. Such particles are characterized by a lower area of contact and a smaller, more homogeneous particle size distribution that result in a higher respirable fraction than mechanically micronized carriers. One of the advantages for using spray-dried mannitol is to achieve particle diameters of several micrometers with a narrow particle size distribution. This ensures, assuming an appropriate diameter, a maximum deposition of the embedded drug in the tracheo-bronchial and deep alveoli regions at normal inhalation rates. Moreover, spray-dried mannitol exhibited a narrow particle size distribution which means the ratio between the median particle size (d₅₀) and d₉₀ which is equal to or greater than 0.40. Preferably, the ratio between the median particle size and d d₉₀ is between 0.45 and 0.50, more preferably it is 0.50 and 0.70.

Additionaly, this narrow particle size distribution also applies to mannitol in the compositions of the present invention which is equal to or greater than 0.40. Preferably, the ratio of narrow particle size distribution is between 0.45 and 0.50, more preferably it is 0.50 and 0.70.

According to a preferred embodiment of the present invention, the average particle diameter (d₅₀) of the drugs having amine is between 1.5 - 2.5 µm, and the amine is primary amine and/or secondary amine.

According to a preferred embodiment of the present invention, the drug having amine is aclidinium or a pharmaceutically acceptable salt or ester thereof, or in enantiomerically pure form or as a racemic mixture. Preferably it can be aclidinium bromide.

According to a preferred embodiment of the present invention, the drug having amine is glycopyrronium or a pharmaceutically acceptable salt or ester thereof, or in enantiomerically pure form or as a racemic mixture. Preferably it can be glycopyrronium bromide or glycopyrronium acetate.

According to a preferred embodiment of the present invention, the drug having amine is darotropium or a pharmaceutically acceptable salt or ester thereof, or in enantiomerically pure form or as a racemic mixture. Preferably it can be darotropium bromide.

According to a preferred embodiment of the present invention, the drug having amine is indacaterol or a pharmaceutically acceptable salt or ester thereof, or in enantiomerically pure form or as a racemic mixture. Preferably it can be indacaterol maleate.

According to a preferred embodiment of the present invention, the drug having amine is vilanterol or a pharmaceutically acceptable salt or ester thereof, or in enantiomerically pure form or as a racemic mixture. Preferably it can be vilanterol trifenatate.

According to a preferred embodiment of the present invention, the drug having amine is carmoterol or a pharmaceutically acceptable salt or ester thereof, or in enantiomerically pure form or as a racemic mixture. Preferably it can be carmoterol hydrochloride.

According to a preferred embodiment of the present invention, the drug having amine is olodaterol or a pharmaceutically acceptable salt or ester thereof, or in enantiomerically pure form or as a racemic mixture. Preferably it can be olodaterol hydrochloride.

Asthma, chronic obstructive pulmonary disease and other related disorders have been known to be treated with beta-2 adrenergic receptor agonists as they provide a bronchodilator effect to the patients, resulting in relief from the symptoms of breathlessness. Beta-2 adrenergic receptor agonists can be short acting for immediate relief, or long acting for long-term prevention, of asthma symptoms. Long actings are long acting beta agonists (LABA) whose effect lasts for 12 hours or more. In a preferred embodiment, LABAs are selected from the group comprising salmeterol, formoterol, arformoterol, indacaterol, olodaterol, vilanterol, carmoterol, bambuterol or a pharmaceutically acceptable salt or ester thereof, or in enantiomerically pure form or as a racemic mixture or a combination of two or more of them. Preferably LABAs can be salmeterol xinofoate, arformoterol tartarate, indacaterol tartarate, olodaterol hydrochloride, vilanterol trifenatate, carmoterol hydrochloride, bambuterol hydrochloride, formoterol fumarate or a combination of two or more of them.

Short actings are short acting beta-2 agonists (SABA). They are bronchodilators. They relax the muscles lining the airways that carry air to the lungs within 5 minutes, increasing airflow and making it easier to breathe. They relieve asthma symptoms for 3 to 6 hours. They do not control the inflammation. In a preferred embodiment, SABAs are selected from the group comprising salbutamol, levosalbutamol, terbutaline, pirbuterol, procaterol, fenoterol, biltolterol, ritodrine, metaproterenol or a pharmaceutically acceptable salt or ester thereof, or in enantiomerically pure form or as a racemic mixture or a combination of two or more of them. Preferably SABAs can be salbutamol sulphate, salbutamol hemisulphate, levosalbutamol sulphate, terbutaline sulfate, pirbuterol hydrochloride, pirbuterol acetate, procaterol hydrochloride, fenoterol hydrobromide, bitolterol mesylate, ritodrine hydrochloride, metaproterenol sulfate or a combination of two or more of them.

Whilst it is also known that, beta-2 agonists provide symptomatic relief of bronchoconstriction in patients, another component of asthma, i. e. inflammation, often requires separate treatment. According to this, involves treatment with a steroid. Treatment with an inhaled corticosteroid is considered one of the most potent and effective therapies currently available for persistent asthma. In a preffered embodiment, inhaled corticosteroids are selected from the group comprising fluticasone, ciclesonide, budesonide, mometasone, beclomethasone, triamcinolone, flunisolide, dexamethasone or a pharmaceutically acceptable salt or ester thereof, or in enantiomerically pure form or as a racemic mixture or a combination of two or more of them. Preferably, the corticosteroids can be fluticasone propionate, fluticasone furoate, ciclesonide, budesonide, mometasone furoate, beclomethasone dipropionate, triamcinolone acetonide, flunisolide acetate, dexamethasone sodium phosphate or a combination of two or more of them.

Bronchoconstriction and inflammation are also associated with bronchial plugging with secretions, which may be treated with long acting muscarinic antagonists (LAMA). In a preffered embodiment LAMAs are selected from the group comprising tiotropium, glycopyrronium, ipratropium, aclidinium, oxitropium or a pharmaceutically acceptable salt or ester thereof, or in enantiomerically pure form or as a racemic mixture or a combination of two or more of them. Preferably the LAMAs can be tiotropium bromide, glycopyrronium bromide, glycopyrronium acetate, ipratropium bromide, aclidinium bromide, oxitropium bromide or a combination of two or more of them.

According to this preferred embodiment of the present invention, the said pharmaceutical compositions may further comprise one or more additional active agents selected from long acting muscarinic antagonists, long acting beta agonists, short acting beta-2 agonists, inhaled corticosteroids or a combination of two or more of them.

To assist better patient compliance, combination products are still needed. It would be highly desirable, however, to provide a combination therapy suitable to reduce bronchial inflammation, bronchial constriction and bronchial secretions in a single product or dosage form. It would also be desirable to provide such a combination product or composition in a form whereby the correct dosage of the various components is easily and safely administered.

Therefore, in a preffered embodiment of the invention, the pharmaceutical compositions comprise the drugs having amine and long acting muscarinic antagonists, or comprise the drugs having amine and long acting beta agonists, or comprise the drugs having amine and short acting beta-2 agonists, or comprise the drugs having amine and inhaled corticosteroids.

According to another embodiment, the pharmaceutical compositions comprise any of the following combinations which are suitable for administration separately, sequentially or together in effective amounts of aclidinium and tiotropium, aclidinium and glycopyrronium, aclidinum and ipratropium, aclidinum and oxitropium, glycopyrronium and tiotropium, glycopyrronium and ipratropium, glycopyrronium and oxitropium, darotropium and tiotropium, darotropium and glycopyrronium, darotropium and ipratropium, darotropium and aclidinium, darotropium and oxitropium, indacaterol and tiotropium, indacaterol and glycopyrronium, indacaterol and ipratropium, indacaterol and aclidinium, indacaterol and oxitropium, vilanterol and tiotropium, vilanterol and glycopyrronium, vilanterol and ipratropium, vilanterol and aclidinium, vilanterol and oxitropium, carmoterol and tiotropium, carmoterol and glycopyrronium, carmoterol and ipratropium, carmoterol and aclidinium, carmoterol and oxitropium, olodaterol and tiotropium, olodaterol and ipratropium, olodaterol and glycopyrronium, olodaterol and aclidinium, olodaterol and oxitropium wherein the above therapeutic agents can be present as a pharmaceutically acceptable salt or ester thereof, or in enantiomerically pure form or as a racemic mixture. According to another embodiment, the pharmaceutical compositions comprise any of the following combinations which are suitable for administration separately, sequentially or together in effective amounts of aclidinium and salmeterol, aclidinum and formoterol, aclidinium and arformoterol, aclidinium and indacaterol, aclidinium and olodaterol, aclidinium and vilanterol, aclidinium and carmoterol, aclidinium and bambuterol, glycopyrronium and salmeterol, glycopyrronium and formoterol, glycopyrronium and arformoterol, glycopyrronium and indacaterol, glycopyrronium and olodaterol, glycopyrronium and vilanterol, glycopyrronium and carmoterol, glycopyrronium and bambuterol, darotropium and salmeterol, darotropium and formoterol, darotropium and arformoterol, darotropium and indacaterol, darotropium and olodaterol, darotropium and vilanterol, darotropium and carmoterol, darotropium and bambuterol, indacaterol and salmeterol, indacaterol and formoterol, indacaterol and arformoterol, indacaterol and olodaterol, indacaterol and vilanterol, indacaterol and carmoterol, indacaterol and bambuterol, vilanterol and salmeterol, vilanterol and formoterol, vilanterol and arformoterol, vilanterol and olodaterol, vilanterol and carmoterol, vilanterol and bambuterol, carmoterol and salmeterol, carmoterol and formoterol, carmoterol and arformoterol, carmoterol and olodaterol, carmoterol and bambuterol, olodaterol and salmeterol, olodaterol and formoterol, olodaterol and arformoterol, olodaterol and bambuterol wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of aclidinium and salbutamol, aclidinium and levosalbutamol, aclidinium and terbutaline, aclidinium and pirbuterol, aclidinium and procaterol, aclidinium and fenoterol, aclidinium and bitolterol, aclidinium and ritodrine, aclidinium and metaproterenol, glycopyrronium and salbutamol, glycopyrronium and levosalbutamol, glycopyrronium and terbutaline, glycopyrronium and pirbuterol, glycopyrronium and procaterol, glycopyrronium and fenoterol, glycopyrronium and bitolterol, glycopyrronium and ritodrine, glycopyrronium and metaproterenol, darotropium and salbutamol, darotropium and levosalbutamol, darotropium and terbutaline, darotropium and pirbuterol, darotropium and procaterol, darotropium and fenoterol, darotropium and bitolterol, darotropium and ritodrine, darotropium and metaproterenol, indacaterol and salbutamol, indacaterol and levosalbutamol, indacaterol and terbutaline, indacaterol and pirbuterol, indacaterol and procaterol, indacaterol and fenoterol, indacaterol and bitolterol, indacaterol and ritodrine, indacaterol and metaproterenol, vilanterol and salbutamol, vilanterol and levosalbutamol, vilanterol and terbutaline, vilanterol and pirbuterol, vilanterol and procaterol, vilanterol and fenoterol, vilanterol and bitolterol, vilanterol and ritodrine, vilanterol and metaproterenol, carmoterol and salbutamol, carmoterol and levosalbutamol, carmoterol and terbutaline, carmoterol and pirbuterol, carmoterol and procaterol, carmoterol and fenoterol, carmoterol and bitolterol, carmoterol and ritodrine, carmoterol and metaproterenol, olodaterol and salbutamol, olodaterol and levosalbutamol, olodaterol and terbutaline, olodaterol and pirbuterol, olodaterol and procaterol, olodaterol and fenoterol, olodaterol and bitolterol, olodaterol and ritodrine, olodaterol and metaproterenol wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of aclidinium and fluticasone, aclidinium and ciclesonide, aclidinium and budesonide, aclidinium and mometasone, aclidinium and beclomethasone, aclidinium and triamcinolone, aclidinium and flunisolide, aclidinium and dexamethasone, glycopyrronium and fluticasone, glycopyrronium and ciclesonide, glycopyrronium and budesonide, glycopyrronium and mometasone, glycopyrronium and beclomethasone, glycopyrronium and triamcinolone, glycopyrronium and flunisolide, glycopyrronium and dexamethasone, darotropium and fluticasone, darotropium and ciclesinide, darotropium and budesonide, darotropium and mometasone, darotropium and beclomethasone, darotropium and triamcinolone, darotropium and flunisolide, darotropium and dexamethasone, indacaterol and fluticasone, indacaterol and ciclesonide, indacaterol and budesonide, indacaterol and mometasone, indacaterol and beclomethasone, indacaterol and triamcinolone, indacaterol and flunisolide, indacaterol and dexamethasone, vilanterol and fluticasone, vilanterol and ciclesonide, vilanterol and budesonide, vilanterol and mometasone, vilanterol and beclomethasone, vilanterol and triamcinolone, vilanterol and flunisolide, vilanterol and dexamethasone, carmoterol and fluticasone, carmoterol and ciclesonide, carmoterol and budesonide, carmoterol and mometasone, carmoterol and beclomethasone, carmoterol and triamcinolone, carmoterol and flunisolide, carmoterol and dexamethasone, olodaterol and fluticasone, olodaterol and ciclesonide, olodaterol and budesonide, olodaterol and mometasone, olodaterol and beclamethasone, olodaterol and triamcinolone, olodaterol and flunisolide, olodaterol and dexamethasone wherein the above therapeutic agents can be present as a pharmaceutically acceptable salt or ester thereof, or in enantiomerically pure form or as a racemic mixture.

We have also found that certain therapeutic three-in-one combinations further comprising specific, LABAs, SABAs, LAMAs and/or inhaled corticosteroids surprisingly provide an enhanced, synergistic, effect in terms of treatment of bronchoconstriction, inflammation and mucous secretions of airways. Also the three-in-one combination therapy as provided by the present invention is an extremely patient-friendly combination, which results in maximum patient compliance and better control of asthma and chronic obstructive pulmonary disease than the known combinations or single therapies.

It will also be appreciated from the above that the respective therapeutic agents of the combined preparations can be administered simultaneously, either in the same or different pharmaceutical formulations, or separately or sequentially. If there is separate or sequential administration, it will also be appreciated that the subsequently administered therapeutic agents should be administered to a patient within a time scale so as to achieve, or more particularly optimise, the above referred to advantageous synergistic therapeutic effect of a combined preparation as present in a pharmaceutical product according to the present invention.

Therefore, in a further embodiment, the pharmaceutical compositions of the invention comprise the drugs having amine, long acting muscarinic antagonists and long acting beta agonists, or comprise the drugs having amine, long acting muscarinic antagonists and short acting beta-2 agonists, or comprise the drugs having amine, long acting muscarinic antagonists and inhaled corticosteorids, or comprise the drugs having amine, long acting beta angonists and short acting beta-2 agonists, or comprise the drugs having amine, long acting beta angonists and inhaled corticosteorids, or comprise the drugs having amine, short acting beta-2 agonists and inhaled corticosteorids.

According to another embodiment, the pharmaceutical compositions comprise any of the following combinations which are suitable for administration separately, sequentially or together in effective amounts of;
i. Aclidinum, tiotropium and salmeterol
ii. Aclidinum, tiotropium and formoterol
iii. Aclidinum, tiotropium and arformoterol
iv. Aclidinum, tiotropium and indacaterol
v. Aclidinum, tiotropium and olodaterol
vi. Aclidinum, tiotropium and vilanterol
vii. Aclidinum, tiotropium and carmoterol
viii. Aclidinum, tiotropium and bambuterol
ix. Aclidinum, glycopyrronium and salmeterol
x. Aclidinum, glycopyrronium and formoterol
xi. Aclidinum, glycopyrronium and arformoterol
xii. Aclidinum, glycopyrronium and indacaterol
xiii. Aclidinum, glycopyrronium and olodaterol
xiv. Aclidinum, glycopyrronium and vilanterol
xv. Aclidinum, glycopyrronium and carmoterol
xvi. Aclidinum, glycopyrronium and bambuterol
xvii. Aclidinum, oxitropium and salmeterol
xviii. Aclidinum, oxitropium and formoterol
xix. Aclidinum, oxitropium and arformoterol
xx. Aclidinum, oxitropium and indacaterol
xxi. Aclidinum, oxitropium and olodaterol
xxii. Aclidinum, oxitropium and vilanterol
xxiii. Aclidinum, oxitropium and carmoterol
xxiv. Aclidinum, oxitropium and bambuterol
xxv. Glycopyrronium, tiotropium and salmeterol
xxvi. Glycopyrronium, tiotropium and formoterol
xxvii. Glycopyrronium, tiotropium and arformoterol
xxviii. Glycopyrronium, tiotropium and indacaterol
xxix. Glycopyrronium, tiotropium and olodaterol
xxx. Glycopyrronium, tiotropium and vilanterol
xxxi. Glycopyrronium, tiotropium and carmoterol
xxxii. Glycopyrronium, tiotropium and bambuterol
xxxiii. Glycopyrronium, oxitropium and salmeterol
xxxiv. Glycopyrronium, oxitropium and formoterol
xxxv. Glycopyrronium, oxitropium and arformoterol
xxxvi. Glycopyrronium, oxitropium and indacaterol
xxxvii. Glycopyrronium, oxitropium and olodaterol
xxxviii. Glycopyrronium, oxitropium and vilanterol
xxxix. Glycopyrronium, oxitropium and carmoterol
xl. Glycopyrronium, oxitropium and bambuterol
xli. Daratropium, tiotropium and salmeterol
xlii. Daratropium, tiotropium and formoterol
xliii. Daratropium, tiotropium and arformoterol
xliv. Daratropium, tiotropium and indacaterol
xlv. Daratropium, tiotropium and olodaterol
xlvi. Daratropium, tiotropium and vilanterol
xlvii. Daratropium, tiotropium and carmoterol
xlviii. Daratropium, tiotropium and bambuterol
xlix. Daratropium, gycopyrronium and salmeterol
I. Daratropium, gycopyrronium and formoterol
li. Daratropium, gycopyrronium and arformoterol
lii. Daratropium, gycopyrronium and indacaterol
liii. Daratropium, gycopyrronium and olodaterol
liv. Daratropium, gycopyrronium and vilanterol
lv. Daratropium, gycopyrronium and carmoterol
Ivi. Daratropium, gycopyrronium and bambuterol
Ivii. Daratropium, aclidinium and salmeterol
Iviii. Daratropium, aclidinium and formoterol
lix. Daratropium, aclidinium and arformoterol
Ix. Daratropium, aclidinium and indacaterol
Ixi. Daratropium, aclidinium and olodaterol
Ixii. Daratropium, aclidinium and vilanterol
Ixiii. Daratropium, aclidinium and carmoterol
Ixiv. Daratropium, aclidinium and bambuterol
lxv. Daratropium, oxitropium and salmeterol
lxvi. Daratropium, oxitropium and formoterol
lxvii. Daratropium, oxitropium and arformoterol
lxviii. Daratropium, oxitropium and indacaterol
Ixix. Daratropium, oxitropium and olodaterol
lxx. Daratropium, oxitropium and vilanterol
lxxi. Daratropium, oxitropium and carmoterol
lxxii. Daratropium, oxitropium and bambuterol
lxxiii. Indacaterol, tirotropium and salmeterol
lxxiv. Indacaterol, tirotropium and formoterol
lxxv. Indacaterol, tirotropium and arformoterol
lxxvi. Indacaterol, tirotropium and olodaterol
lxxvii. Indacaterol, tirotropium and vilanterol
lxxviii. Indacaterol, tirotropium and carmoterol
lxxix. Indacaterol, tirotropium and bambuterol
lxxx. Indacaterol, glycopyrronium and salmeterol
lxxxi. Indacaterol, glycopyrronium and formoterol
lxxxii. Indacaterol, glycopyrronium and arformoterol
lxxxiii. Indacaterol, glycopyrronium and olodaterol
lxxxiv. Indacaterol, glycopyrronium and vilanterol
lxxxv. Indacaterol, glycopyrronium and carmoterol
lxxxvi. Indacaterol, glycopyrronium and bambuterol
lxxxvii. Indacaterol, aclidinium and salmeterol
lxxxviii. Indacaterol, aclidinium and formoterol
lxxxix. Indacaterol, aclidinium and arformoterol
xc. Indacaterol, aclidinium and olodaterol
xci. Indacaterol, aclidinium and vilanterol
xcii. Indacaterol, aclidinium and carmoterol
xciii. Indacaterol, aclidinium and bambuterol
xciv. Indacaterol, oxitropium and salmeterol
xcv. Indacaterol, oxitropium and formoterol
xcvi. Indacaterol, oxitropium and arformoterol
xcvii. Indacaterol, oxitropium and olodaterol
xcviii. Indacaterol, oxitropium and vilanterol
xcix. Indacaterol, oxitropium and carmoterol
c. Indacaterol, oxitropium and bambuterol
ci. Vilanterol, tiotropium and salmeterol
cii. Vilanterol, tiotropium and formoterol
ciii. Vilanterol, tiotropium and arformoterol
civ. Vilanterol, tiotropium and indacaterol
cv. Vilanterol, tiotropium and olodaterol
cvi. Vilanterol, tiotropium and carmoterol
cvii. Vilanterol, tiotropium and bambuterol
cviii. Vilanterol, glycopyrronium and salmeterol
cix. Vilanterol, glycopyrronium and formoterol
cx. Vilanterol, glycopyrronium and arformoterol
cxi. Vilanterol, glycopyrronium and indacaterol
cxii. Vilanterol, glycopyrronium and olodaterol
cxiii. Vilanterol, glycopyrronium and carmoterol
cxiv. Vilanterol, glycopyrronium and bambuterol
cxv. Vilanterol, aclidinium and salmeterol
cxvi. Vilanterol, aclidinium and formoterol
cxvii. Vilanterol, aclidinium and arformoterol
cxviii. Vilanterol, aclidinium and indacaterol
cxix. Vilanterol, aclidinium and olodaterol
cxx. Vilanterol, aclidinium and carmoterol
cxxi. Vilanterol, aclidinium and bambuterol
cxxii. Vilanterol, oxitropium and salmeterol
cxxiii. Vilanterol, oxitropium and formoterol
cxxiv. Vilanterol, oxitropium and arformoterol
cxxv. Vilanterol, oxitropium and indacaterol
cxxvi. Vilanterol, oxitropium and olodaterol
cxxvii. Vilanterol, oxitropium and carmoterol
cxxviii. Vilanterol, oxitropium and bambuterol
cxxix. Carmoterol, tiotropium and salmeterol
cxxx. Carmoterol, tiotropium and formoterol
cxxxi. Carmoterol, tiotropium and arformoterol
cxxxii. Carmoterol, tiotropium and indacaterol
cxxxiii. Carmoterol, tiotropium and olodaterol
cxxxiv. Carmoterol, tiotropium and vilanterol
cxxxv. Carmoterol, tiotropium and bambuterol
cxxxvi. Carmoterol, glycopyrronium and salmeterol
cxxxvii.Carmoterol, glycopyrronium and formoterol
cxxxviii. Carmoterol, glycopyrronium and arformoterol
cxxxix. Carmoterol, glycopyrronium and indacaterol
cxl. Carmoterol, glycopyrronium and olodaterol
cxli. Carmoterol, glycopyrronium and vilanterol
cxlii. Carmoterol, glycopyrronium and bambuterol
cxliii. Carmoterol, aclidinium and salmeterol
cxliv. Carmoterol, aclidinium and formoterol
cxlv. Carmoterol, aclidinium and arformoterol
cxlvi. Carmoterol, aclidinium and indacaterol
cxlvii. Carmoterol, aclidinium and olodaterol
cxlviii. Carmoterol, aclidinium and vilanterol
cxlix. Carmoterol, aclidinium and bambuterol
cl. Carmoterol, oxitropium and salmeterol
cli. Carmoterol, oxitropium and formoterol
clii. Carmoterol, oxitropium and arformoterol
cliii. Carmoterol, oxitropium and indacaterol
cliv. Carmoterol, oxitropium and olodaterol
clv. Carmoterol, oxitropium and vilanterol
clvi. Carmoterol, oxitropium and bambuterol
clvii. Olodaterol, tiotropium and salmeterol
clviii. Olodaterol, tiotropium and formoterol
clix. Olodaterol, tiotropium and arformoterol
clx. Olodaterol, tiotropium and indacaterol
clxi. Olodaterol, tiotropium and vilanterol
clxii. Olodaterol, tiotropium and bambuterol
clxiii. Olodaterol, glycopyrronium and salmeterol
clxiv. Olodaterol, glycopyrronium and formoterol
clxv. Olodaterol, glycopyrronium and arformoterol
clxvi. Olodaterol, glycopyrronium and indacaterol
clxvii. Olodaterol, glycopyrronium and vilanterol
clxviii. Olodaterol, glycopyrronium and bambuterol
clxix. Olodaterol, aclidinium and salmeterol
clxx. Olodaterol, aclidinium and formoterol
clxxi. Olodaterol, aclidinium and arformoterol
clxxii. Olodaterol, aclidinium and indacaterol
clxxiii. Olodaterol, aclidinium and vilanterol
clxxiv. Olodaterol, aclidinium and bambuterol
clxxv. Olodaterol, oxitropium and salmeterol
clxxvi. Olodaterol, oxitropium and formoterol
clxxvii. Olodaterol, oxitropium and arformoterol
clxxviii.Olodaterol, oxitropium and indacaterol
clxxix. Olodaterol, oxitropium and vilanterol
clxxx. Olodaterol, oxitropium and bambuterol
wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of;
i. Aclidinum, tiotropium and salbutamol
ii. Aclidinum, tiotropium and levosalbutamol
iii. Aclidinum, tiotropium and terbutaline
iv. Aclidinum, tiotropium and pirbutarol
v. Aclidinum, tiotropium and procaterol
vi. Aclidinum, tiotropium and fenoterol
vii. Aclidinum, tiotropium and ritodrine
viii. Aclidinum, tiotropium and bitolterol
ix. Aclidinum, tiotropium and metaproterenol
x. Aclidinum, glycopyrronium and salbutamol
xi. Aclidinum, glycopyrronium and levosalbutamol
xii. Aclidinum, glycopyrronium and terbutaline
xiii. Aclidinum, glycopyrronium and pirbuterol
xiv. Aclidinum, glycopyrronium and procaterol
xv. Aclidinum, glycopyrronium and fenoterol
xvi. Aclidinum, glycopyrronium and bitolterol
xvii. Aclidinum, glycopyrronium and ritodrine
xviii. Aclidinum, glycopyrronium and metaproterenol
xix. Aclidinum, ipratropium and salbutamol
xx. Aclidinum, ipratropium and levosalbutamol
xxi. Aclidinum, ipratropium and terbutaline
xxii. Aclidinum, ipratropium and pirbuterol
xxiii. Aclidinum, ipratropium and procaterol
xxiv. Aclidinum, ipratropium and fenoterol
xxv. Aclidinum, ipratropium and bitolterol
xxvi. Aclidinum, ipratropium and ritodrine
xxvii. Aclidinum, ipratropium and metaproterenol
xxviii. Aclidinum, oxitropium and salbutamol
xxix. Aclidinum, oxitropium and levosalbutamol
xxx. Aclidinum, oxitropium and terbutaline
xxxi. Aclidinum, oxitropium and pirbuterol
xxxii. Aclidinum, oxitropium and procaterol
xxxiii. Aclidinum, oxitropium and fenoterol
xxxiv. Aclidinum, oxitropium and bitolterol
xxxv. Aclidinum, oxitropium and ritodrine
xxxvi. Aclidinum, oxitropium and metaproterenol
xxxvii. Glycopyrronium, tiotropium and salbutamol
xxxviii. Glycopyrronium, tiotropium and levosalbutamol
xxxix. Glycopyrronium, tiotropium and terbutaline
xl. Glycopyrronium, tiotropium and pirbuterol
xli. Glycopyrronium, tiotropium and procaterol
xlii. Glycopyrronium, tiotropium and fenoterol
xliii. Glycopyrronium, tiotropium and bitolterol
xliv. Glycopyrronium, tiotropium and ritodrine
xlv. Glycopyrronium, tiotropium and metaproterenol
xlvi. Glycopyrronium, ipratropium and salbutamol
xlvii. Glycopyrronium, ipratropium and levosalbutamol
xlviii. Glycopyrronium, ipratropium and terbutaline
xlix. Glycopyrronium, ipratropium and pirbuterol
l. Glycopyrronium, ipratropium and procaterol
li. Glycopyrronium, ipratropium and fenoterol
lii. Glycopyrronium, ipratropium and bitolterol
liii. Glycopyrronium, ipratropium and ritodrine
liv. Glycopyrronium, ipratropium and metaproterenol
lv. Glycopyrronium, oxitropium and salbutamol
lvi. Glycopyrronium, oxitropium and levosalbutamol
lvii. Glycopyrronium, oxitropium and terbutaline
lviii. Glycopyrronium, oxitropium and pirbuterol
lix. Glycopyrronium, oxitropium and procaterol
lx. Glycopyrronium, oxitropium and fenoterol
lxi. Glycopyrronium, oxitropium and bitolterol
lxii. Glycopyrronium, oxitropium and ritodrine
lxiii. Glycopyrronium, oxitropium and metaproterenol
lxiv. Daratropium, tiotropium and salbutamol
lxv. Daratropium, tiotropium and levosalbutamol
lxvi. Daratropium, tiotropium and terbutaline
lxvii. Daratropium, tiotropium and pirbuterol
lxviii. Daratropium, tiotropium and procaterol
lxix. Daratropium, tiotropium and fenoterol
lxx. Daratropium, tiotropium and bitolterol
lxxi. Daratropium, tiotropium and ritodrine
lxxii. Daratropium, tiotropium and metaproterenol
lxxiii. Daratropium, aclidinium and salbutamol
lxxiv. Daratropium, aclidinium and levosalbutamol
lxxv. Daratropium, aclidinium and terbutaline
lxxvi. Daratropium, aclidinium and pirbuterol
lxxvii. Daratropium, aclidinium and procaterol
lxxviii. Daratropium, aclidinium and fenoterol
lxxix. Daratropium, aclidinium and bitolterol
lxxx. Daratropium, aclidinium and ritodrine
lxxxi. Daratropium, aclidinium and metaproterenol
lxxxii. Daratropium, glycopyrronium and salbutamol
lxxxiii. Daratropium, glycopyrronium and levosalbutamol
lxxxiv. Daratropium, glycopyrronium and terbutaline
lxxxv. Daratropium, glycopyrronium and pirbuterol
lxxxvi. Daratropium, glycopyrronium and procaterol
lxxxvii. Daratropium, glycopyrronium and fenoterol
lxxxviii. Daratropium, glycopyrronium and bitolterol
lxxxix. Daratropium, glycopyrronium and ritodrine
xc. Daratropium, glycopyrronium and metaproterenol
xci. Daratropium, ipratropium and salbutamol
xcii. Daratropium, ipratropium and levosalbutamol
xciii. Daratropium, ipratropium and terbutaline
xciv. Daratropium, ipratropium and pirbuterol
xcv. Daratropium, ipratropium and procaterol
xcvi. Daratropium, ipratropium and fenoterol
xcvii. Daratropium, ipratropium and bitolterol
xcviii. Daratropium, ipratropium and ritodrine
xcix. Daratropium, ipratropium and metaproterenol
c. Daratropium, oxitropium and salbutamol
ci. Daratropium, oxitropium and levosalbutamol
cii. Daratropium, oxitropium and terbutaline
ciii. Daratropium, oxitropium and pirbuterol
civ. Daratropium, oxitropium and procaterol
cv. Daratropium, oxitropium and fenoterol
cvi. Daratropium, oxitropium and bitolterol
cvii. Daratropium, oxitropium and ritodrine
cviii. Daratropium, oxitropium and metaproterenol
cix. Indacaterol, tirotropium and salbutamol
cx. Indacaterol, tirotropium and levosalbutamol
cxi. Indacaterol, tirotropium and terbutaline
cxii. Indacaterol, tirotropium and pirbuterol
cxiii. Indacaterol, tirotropium and procaterol
cxiv. Indacaterol, tirotropium and fenoterol
cxv. Indacaterol, tirotropium and bitolterol
cxvi. Indacaterol, tirotropium and ritodrine
cxvii. Indacaterol, tirotropium and metaproterenol
cxviii. Indacaterol, glycopyrronium and salbutamol
cxix. Indacaterol, glycopyrronium and levosalbutamol
cxx. Indacaterol, glycopyrronium and terbutaline
cxxi. Indacaterol, glycopyrronium and pirbuterol
cxxii. Indacaterol, glycopyrronium and procaterol
cxxiii. Indacaterol, glycopyrronium and fenoterol
cxxiv. Indacaterol, glycopyrronium and bitolterol
cxxv. Indacaterol, glycopyrronium and ritodrine
cxxvi. Indacaterol, glycopyrronium and metaproterenol
cxxvii. Indacaterol, aclidinium and salbutamol
cxxviii. Indacaterol, aclidinium and levosalbutamol
cxxix. Indacaterol, aclidinium and terbutaline
cxxx. Indacaterol, aclidinium and pirbuterol
cxxxi. Indacaterol, aclidinium and procaterol
cxxxii. Indacaterol, aclidinium and fenoterol
cxxxiii. Indacaterol, aclidinium and bitolterol
cxxxiv. Indacaterol, aclidinium and ritodrine
cxxxv. Indacaterol, aclidinium and metaproterenol
cxxxvi. Indacaterol, ipratropium and salbutamol
cxxxvii. Indacaterol, ipratropium and levosalbutamol
cxxxviii. Indacaterol, ipratropium and terbutaline
cxxxix. Indacaterol, ipratropium and pirbuterol
cxl. Indacaterol, ipratropium and procaterol
cxli. Indacaterol, ipratropium and fenoterol
cxlii. Indacaterol, ipratropium and bitolterol
cxliii. Indacaterol, ipratropium and ritodrine
cxliv. Indacaterol, ipratropium and metaproterenol
cxlv. Indacaterol, oxitropium and salbutamol
cxlvi. Indacaterol, oxitropium and levosalbutamol
cxlvii. Indacaterol, oxitropium and terbutaline
cxlviii. Indacaterol, oxitropium and pirbuterol
cxlix. Indacaterol, oxitropium and procaterol
cl. Indacaterol, oxitropium and fenoterol
cli. Indacaterol, oxitropium and bitolterol
clii. Indacaterol, oxitropium and ritodrine
cliii. Indacaterol, oxitropium and metaproterenol
cliv. Vilanterol, tiotropium and salbutamol
clv. Vilanterol, tiotropium and levosalbutamol
clvi. Vilanterol, tiotropium and terbutaline
clvii. Vilanterol, tiotropium and pirbuterol
clviii. Vilanterol, tiotropium and procaterol
clix. Vilanterol, tiotropium and fenoterol
clx. Vilanterol, tiotropium and bitolterol
clxi. Vilanterol, tiotropium and ritodrine
clxii. Vilanterol, tiotropium and metaproterenol
clxiii. Vilanterol, glycopyrronium and salbutamol
clxiv. Vilanterol, glycopyrronium and levosalbutamol
clxv. Vilanterol, glycopyrronium and terbutaline
clxvi. Vilanterol, glycopyrronium and pirbuterol
clxvii. Vilanterol, glycopyrronium and procaterol
clxviii. Vilanterol, glycopyrronium and fenoterol
clxix. Vilanterol, glycopyrronium and bitolterol
clxx. Vilanterol, glycopyrronium and ritodrine
clxxi. Vilanterol, glycopyrronium and metaproterenol
clxxii. Vilanterol, ipratropium and salbutamol
clxxiii. Vilanterol, ipratropium and levosalbutamol
clxxiv. Vilanterol, ipratropium and terbutaline
clxxv. Vilanterol, ipratropium and pirbuterol
clxxvi. Vilanterol, ipratropium and procaterol
clxxvii. Vilanterol, ipratropium and fenoterol
clxxviii. Vilanterol, ipratropium and bitolterol
clxxix. Vilanterol, ipratropium and ritodrine
clxxx. Vilanterol, ipratropium and metaproterenol
clxxxi. Vilanterol, aclidinium and salbutamol
clxxxii. Vilanterol, aclidinium and levosalbutamol
clxxxiii. Vilanterol, aclidinium and terbutaline
clxxxiv.Vilanterol, aclidinium and pirbuterol
clxxxv. Vilanterol, aclidinium and procaterol
clxxxvi.Vilanterol, aclidinium and fenoterol
clxxxvii. Vilanterol, aclidinium and bitolterol
clxxxviii. Vilanterol, aclidinium and ritodrine
clxxxix.Vilanterol, aclidinium and metaproterenol
cxc. Vilanterol, oxitropium and salbutamol
cxci. Vilanterol, oxitropium and levosalbutamol
cxcii. Vilanterol, oxitropium and terbutaline
cxciii. Vilanterol, oxitropium and pirbuterol
cxciv. Vilanterol, oxitropium and procaterol
cxcv. Vilanterol, oxitropium and fenoterol
cxcvi. Vilanterol, oxitropium and bitolterol
cxcvii. Vilanterol, oxitropium and ritodrine
cxcviii. Vilanterol, oxitropium and metaproterenol
cxcix. Carmoterol, tiotropium and salbutamol
cc. Carmoterol, tiotropium and levosalbutamol
cci. Carmoterol, tiotropium and terbutaline
ccii. Carmoterol, tiotropium and pirbuterol
cciii. Carmoterol, tiotropium and procaterol
cciv. Carmoterol, tiotropium and fenoterol
ccv. Carmoterol, tiotropium and bitolterol
ccvi. Carmoterol, tiotropium and ritodrine
ccvii. Carmoterol, tiotropium and metaproterenol
ccviii. Carmoterol, ipratropium and levosalbutamol
ccix. Carmoterol, ipratropium and salbutamol
ccx. Carmoterol, ipratropium and terbutaline
ccxi. Carmoterol, ipratropium and pirbuterol
ccxii. Carmoterol, ipratropium and procaterol
ccxiii. Carmoterol, ipratropium and fenoterol
ccxiv. Carmoterol, ipratropium and bitolterol
ccxv. Carmoterol, ipratropium and ritodrine
ccxvi. Carmoterol, ipratropium and metaproterenol
ccxvii. Carmoterol, aclidinum and levosalbutamol
ccxviii. Carmoterol, aclidinum and salbutamol
ccxix. Carmoterol, aclidinum and terbutaline
ccxx. Carmoterol, aclidinum and pirbuterol
ccxxi. Carmoterol, aclidinum and procaterol
ccxxii. Carmoterol, aclidinum and fenoterol
ccxxiii. Carmoterol, aclidinum and bitolterol
ccxxiv. Carmoterol, aclidinum and ritodrine
ccxxv. Carmoterol, aclidinum and metaproterenol
ccxxvi. Carmoterol, oxitropium and salbutamol
ccxxvii.Carmoterol, oxitropium and levosalbutamol
ccxxviii. Carmoterol, oxitropium and terbutaline
ccxxix. Carmoterol, oxitropium and pirbuterol
ccxxx. Carmoterol, oxitropium and procaterol
ccxxxi. Carmoterol, oxitropium and fenoterol
ccxxxii.Carmoterol, oxitropium and bitolterol
ccxxxiii. Carmoterol, oxitropium and ritodrine
ccxxxiv. Carmoterol, oxitropium and metaproterenol
ccxxxv. Olodaterol, tiotropium and salbutamol
ccxxxvi. Olodaterol, tiotropium and levosalbutamol
ccxxxvii. Olodaterol, tiotropium and terbutaline
ccxxxviii. Olodaterol, tiotropium and pirbuterol
ccxxxix. Olodaterol, tiotropium and procaterol
ccxl. Olodaterol, tiotropium and fenoterol
ccxli. Olodaterol, tiotropium and bitolterol
ccxlii. Olodaterol, tiotropium and ritodrine
ccxliii. Olodaterol, tiotropium and metaproterenol
ccxliv. Olodaterol, ipratropium and salbutamol
ccxlv. Olodaterol, ipratropium and levosalbutamol
ccxlvi. Olodaterol, ipratropium and terbutaline
ccxlvii. Olodaterol, ipratropium and pirbuterol
ccxlviii.Olodaterol, ipratropium and procaterol
ccxlix. Olodaterol, ipratropium and fenoterol
ccl. Olodaterol, ipratropium and bitolterol
ccli. Olodaterol, ipratropium and ritodrine
cclii. Olodaterol, ipratropium and metaproterenol
ccliii. Olodaterol, aclidinum and salbutamol
ccliv. Olodaterol, aclidinum and levosalbutamol
cclv. Olodaterol, aclidinum and terbutaline
cclvi. Olodaterol, aclidinum and pirbuterol
cclvii. Olodaterol, aclidinum and procaterol
cclviii. Olodaterol, aclidinum and fenoterol
cclix. Olodaterol, aclidinum and bitolterol
cclx. Olodaterol, aclidinum and ritodrine
cclxi. Olodaterol, aclidinum and metaproterenol
cclxii. Olodaterol, glycopyrronium and salbutamol
cclxiii. Olodaterol, glycopyrronium and levosalbutamol
cclxiv. Olodaterol, glycopyrronium and terbutaline
cclxv. Olodaterol, glycopyrronium and pirbuterol
cclxvi. Olodaterol, glycopyrronium and procaterol
cclxvii. Olodaterol, glycopyrronium and fenoterol
cclxviii.Olodaterol, glycopyrronium and bitolterol
cclxix. Olodaterol, glycopyrronium and ritodrine
cclxx. Olodaterol, glycopyrronium and metaproterenol
cclxxi. Olodaterol, oxitropium and salbutamol
cclxxii. Olodaterol, oxitropium and levosalbutamol
cclxxiii. Olodaterol, oxitropium and terbutaline
cclxxiv.Olodaterol, oxitropium and pirbuterol
cclxxv. Olodaterol, oxitropium and procaterol
cclxxvi.Olodaterol, oxitropium and fenoterol
cclxxvii. Olodaterol, oxitropium and bitolterol
cclxxviii. Olodaterol, oxitropium and ritodrine
cclxxix. Olodaterol, oxitropium and metaproterenol
wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of;
i. Aclidinum, tiotropium and fluticasone
ii. Aclidinum, tiotropium and ciclesonide
iii. Aclidinum, tiotropium and budesonide
iv. Aclidinum, tiotropium and mometasone
v. Aclidinum, tiotropium and beclomethasone
vi. Aclidinum, tiotropium and triamcinolone
vii. Aclidinum, tiotropium and flunisolide
viii. Aclidinum, tiotropium and dexomethasone
ix. Daratropium, tiotropium and fluticasone
x. Daratropium, tiotropium and ciclesonide
xi. Daratropium, tiotropium and budesonide
xii. Daratropium, tiotropium and mometasone
xiii. Daratropium, tiotropium and beclamethasone
xiv. Daratropium, tiotropium and triamcinolone
xv. Daratropium, tiotropium and flunisolide
xvi. Daratropium, tiotropium and dexomethasone
xvii. Indacaterol, tiotropium and fluticasone
xviii. Indacaterol, tiotropium and budesonide
xix. Indacaterol, tiotropium and ciclesonide
xx. Indacaterol, tiotropium and mometasone
xxi. Indacaterol, tiotropium and beclamethasone
xxii. Indacaterol, tiotropium and triamcinolone
xxiii. Indacaterol, tiotropium and flunisolide
xxiv. Indacaterol, tiotropium and dexomethasone
xxv. Vilanterol, tiotropium and ciclesonide
xxvi. vilanterol, tiotropium and fluticasone
xxvii. vilanterol, tiotropium and budesonide
xxviii. vilanterol, tiotropium and mometasone
xxix. vilanterol, tiotropium and beclamethasone
xxx. vilanterol, tiotropium and triamcinolone
xxxi. vilanterol, tiotropium and flunisolide
xxxii. vilanterol, tiotropium and dexomethasone
xxxiii. carmoterol, tiotropium and budesonide
xxxiv. carmoterol, tiotropium and ciclesonide
xxxv. carmoterol, tiotropium and fluticasone
xxxvi. carmoterol, tiotropium and mometasone
xxxvii. carmoterol, tiotropium and beclamethasone
xxxviii. carmoterol, tiotropium and triamcinolone
xxxix. carmoterol, tiotropium and flunisolide
xl. carmoterol, tiotropium and dexomethasone
xli. Olodaterol, tiotropium and ciclesonide
xlii. Olodaterol, tiotropium and fluticasone
xliii. Olodaterol, tiotropium and budesonide
xliv. Olodaterol, tiotropium and mometasone
xlv. Olodaterol, tiotropium and beclamethasone
xlvi. Olodaterol, tiotropium and triamcinolone
xlvii. Olodaterol, tiotropium and flunisolide
xlviii. Olodaterol, tiotropium and dexomethasone
wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of;
i. Aclidinium, salmeterol and salbutamol
ii. Aclidinium, salmeterol and levosalbutamol
iii. Aclidinium, formoterol and salbutamol
iv. Aclidinium, formoterol and levosalbutamol
v. Aclidinium, arformoterol and salbutamol
vi. Aclidinium, arformoterol and levosalbutamol
vii. Aclidinium, indacaterol and salbutamol
viii. Aclidinium, indacaterol and levosalbutamol
ix. Aclidinium, olodaterol and salbutamol
x. Aclidinium, olodaterol and levosalbutamol
xi. Aclidinium, vilanterol and salbutamol
xii. Aclidinium, vilanterol and levosalbutamol
xiii. Aclidinium, carmoterol and salbutamol
xiv. Aclidinium, carmoterol and levosalbutamol
xv. Aclidinium, bambuterol and salbutamol
xvi. Aclidinium, bambuterol and levosalbutamol
xvii. Glycopyrronium, indacaterol and salbutamol
xviii. Glycopyrronium, indacaterol and levosalbutamol
xix. Glycopyrronium, salmeterol and salbutamol
xx. Glycopyrronium, salmeterol and levosalbutamol
xxi. Glycopyrronium, formoterol and salbutamol
xxii. Glycopyrronium, formoterol and levosalbutamol
xxiii. Glycopyrronium, arformoterol and salbutamol
xxiv. Glycopyrronium, arformoterol and levosalbutamol
xxv. Glycopyrronium, carmoterol and salbutamol
xxvi. Glycopyrronium, carmoterol and levosalbutamol
xxvii. Glycopyrronium, olodaterol and salbutamol
xxviii. Glycopyrronium, olodaterol and levosalbutamol
xxix. Glycopyrronium, vilanterol and salbutamol
xxx. Glycopyrronium, vilanterol and levosalbutamol
xxxi. Glycopyrronium, bambuterol and salbutamol
xxxii. Glycopyrronium, bambuterol and levosalbutamol
xxxiii. Daratropium, indacaterol and salbutamol
xxxiv. Daratropium, indacaterol and levosalbutamol
xxxv. Daratropium, salmeterol and salbutamol
xxxvi. Daratropium, salmeterol and levosalbutamol
xxxvii. Daratropium, formoterol and salbutamol
xxxviii. Daratropium, formoterol and levosalbutamol
xxxix. Daratropium, carmoterol and salbutamol
xl. Daratropium, carmoterol and levosalbutamol
xli. Daratropium, olodaterol and salbutamol
xlii. Daratropium, olodaterol and levosalbutamol
xliii. Daratropium, vilanterol and salbutamol
xliv. Daratropium, vilanterol and levosalbutamol
xlv. Daratropium, bambuterol and salbutamol
xlvi. Daratropium, bambuterol and levosalbutamol
xlvii. Daratropium, arformoterol and salbutamol
xlviii. Daratropium, arformoterol and levosalbutamol
wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of;
i. Aclidinium, salmeterol and mometasone
ii. Aclidinium, salmeterol and fluticasone
iii. Aclidinium, salmeterol and budesonide
iv. Aclidinium, formoterol and mometasone
v. Aclidinium, formoterol and fluticasone
vi. Aclidinium, formoterol and budesonide
vii. Aclidinium, arformoterol and mometasone
viii. Aclidinium, arformoterol and fluticasone
ix. Aclidinium, arformoterol and budesonide
x. Aclidinium, indacaterol and mometasone
xi. Aclidinium, indacaterol and fluticasone
xii. Aclidinium, indacaterol and budesonide
xiii. Aclidinium, olodaterol and mometasone
xiv. Aclidinium, olodaterol and fluticasone
xv. Aclidinium, olodaterol and budesonide
xvi. Aclidinium, vilanterol and mometasone
xvii. Aclidinium, vilanterol and fluticasone
xviii. Aclidinium, vilanterol and budesonide
xix. Aclidinium, carmoterol and mometasone
xx. Aclidinium, carmoterol and fluticasone
xxi. Aclidinium, carmoterol and budesonide
xxii. Aclidinium, bambuterol and mometasone
xxiii. Aclidinium, bambuterol and fluticasone
xxiv. Aclidinium, bambuterol and budesonide
xxv. Glycopyrronium, indacaterol and mometasone
xxvi. Glycopyrronium, indacaterol and fluticasone
xxvii. Glycopyrronium, indacaterol and budesonide
xxviii. Glycopyrronium, salmeterol and mometasone
xxix. Glycopyrronium, salmeterol and fluticasone
xxx. Glycopyrronium, salmeterol and budesonide
xxxi. Glycopyrronium, formoterol and mometasone
xxxii. Glycopyrronium, formoterol and fluticasone
xxxiii. Glycopyrronium, formoterol and budesonide
xxxiv. Glycopyrronium, arformoterol and mometasone
xxxv. Glycopyrronium, arformoterol and fluticasone
xxxvi. Glycopyrronium, arformoterol and budesonide
xxxvii. Glycopyrronium, carmoterol and mometasone
xxxviii. Glycopyrronium, carmoterol and fluticasone
xxxix. Glycopyrronium, carmoterol and budesonide
xl. Glycopyrronium, olodaterol and mometasone
xli. Glycopyrronium, olodaterol and fluticasone
xlii. Glycopyrronium, olodaterol and budesonide
xliii. Glycopyrronium, vilanterol and mometasone
xliv. Glycopyrronium, vilanterol and fluticasone
xlv. Glycopyrronium, vilanterol and budesonide
xlvi. Glycopyrronium, bambuterol and mometasone
xlvii. Glycopyrronium, bambuterol and fluticasone
xlviii. Glycopyrronium, bambuterol and budesonide
xlix. Daratropium, indacaterol and mometasone
l. Daratropium, indacaterol and fluticasone
li. Daratropium, indacaterol and budesonide
lii. Daratropium, salmeterol and mometasone
liii. Daratropium, salmeterol and fluticasone
liv. Daratropium, salmeterol and budesonide
lv. Daratropium, formoterol and mometasone
lvi. Daratropium, formoterol and fluticasone
lvii. Daratropium, formoterol and budesonide
lviii. Daratropium, carmoterol and mometasone
lix. Daratropium, carmoterol and fluticasone
lx. Daratropium, carmoterol and budesonide
lxi. Daratropium, olodaterol and mometasone
lxii. Daratropium, olodaterol and fluticasone
lxiii. Daratropium, olodaterol and budesonide
lxiv. Daratropium, vilanterol and mometasone
lxv. Daratropium, vilanterol and fluticasone
lxvi. Daratropium, vilanterol and budesonide
lxvii. Daratropium, bambuterol and mometasone
lxviii. Daratropium, bambuterol and fluticasone
lxix. Daratropium, bambuterol and budesonide
lxx. Daratropium, arformoterol and mometasone
lxxi. Daratropium, arformoterol and fluticasone
lxxii. Daratropium, arformoterol and budesonide
lxxiii. Indacaterol, salmeterol and mometasone
lxxiv. Indacaterol, salmeterol and fluticasone
lxxv. Indacaterol, salmeterol and budesonide
lxxvi. Indacaterol, formoterol and mometasone
lxxvii. Indacaterol, formoterol and fluticasone
lxxviii. Indacaterol, formoterol and budesonide
lxxix. Indacaterol, arformoterol and mometasone
lxxx. Indacaterol, arformoterol and fluticasone
lxxxi. Indacaterol, arformoterol and budesonide
lxxxii. Indacaterol, olodaterol and mometasone
lxxxiii. Indacaterol, olodaterol and fluticasone
lxxxiv. Indacaterol, olodaterol and budesonide
lxxxv. Indacaterol, vilanterol and mometasone
lxxxvi. Indacaterol, vilanterol and fluticasone
lxxxvii. Indacaterol, vilanterol and budesonide
lxxxviii. Indacaterol, carmeterol and mometasone
lxxxix. Indacaterol, carmeterol and fluticasone
xc. Indacaterol, carmeterol and budesonide
xci. Indacaterol, bambuterol and mometasone
xcii. Indacaterol, bambuterol and fluticasone
xciii. Indacaterol, bambuterol and budesonide
xciv. Vilanterol, salmeterol and mometasone
xcv. Vilanterol, salmeterol and fluticasone
xcvi. Vilanterol, salmeterol and budesonide
xcvii. Vilanterol, formoterol and mometasone
xcviii. Vilanterol, formoterol and fluticasone
xcix. Vilanterol, formoterol and budesonide
c. Vilanterol, arformoterol and mometasone
ci. Vilanterol, arformoterol and fluticasone
cii. Vilanterol, arformoterol and budesonide
ciii. Vilanterol, olodaterol and mometasone
civ. Vilanterol, olodaterol and fluticasone
cv. Vilanterol, olodaterol and budesonide
cvi. Vilanterol, carmeterol and mometasone
cvii. Vilanterol, carmeterol and fluticasone
cviii. Vilanterol, carmeterol and budesonide
cix. Vilanterol, bambuterol and mometasone
cx. Vilanterol, bambuterol and fluticasone
cxi. Vilanterol, bambuterol and budesonide
cxii. Vilanterol, indacaterol and mometasone
cxiii. Vilanterol, indacaterol and fluticasone
cxiv. Vilanterol, indacaterol and budesonide
cxv. Carmeterol, salmeterol and mometasone
cxvi. Carmeterol, salmeterol and fluticasone
cxvii. Carmeterol, salmeterol and budesonide
cxviii. Carmeterol, formoterol and mometasone
cxix. Carmeterol, formoterol and fluticasone
cxx. Carmeterol, formoterol and budesonide
cxxi. Carmeterol, arformoterol and mometasone
cxxii. Carmeterol, arformoterol and fluticasone
cxxiii. Carmeterol, arformoterol and budesonide
cxxiv. Carmeterol, indaceterol and mometasone
cxxv. Carmeterol, indaceterol and fluticasone
cxxvi. Carmeterol, indaceterol and budesonide
cxxvii. Carmeterol, olodaterol and mometasone
cxxviii. Carmeterol, olodaterol and fluticasone
cxxix. Carmeterol, olodaterol and budesonide
cxxx. Carmeterol, vilanterol and mometasone
cxxxi. Carmeterol, vilanterol and fluticasone
cxxxii. Carmeterol, vilanterol and budesonide
cxxxiii. Carmeterol, bambuterol and mometasone
cxxxiv. Carmeterol, bambuterol and fluticasone
cxxxv. Carmeterol, bambuterol and budesonide
cxxxvi. Olodaterol, salmeterol and mometasone
cxxxvii. Olodaterol, salmeterol and fluticasone
cxxxviii. Olodaterol, salmeterol and budesonide
cxxxix. Olodaterol, formoterol and mometasone
cxl. Olodaterol, formoterol and fluticasone
cxli. Olodaterol, formoterol and budesonide
cxlii. Olodaterol, arformoterol and mometasone
cxliii. Olodaterol, arformoterol and fluticasone
cxliv. Olodaterol, arformoterol and budesonide
cxlv. Olodaterol, indaceterol and mometasone
cxlvi. Olodaterol, indaceterol and fluticasone
cxlvii. Olodaterol, indaceterol and budesonide
cxlviii. Olodaterol, vilanterol and mometasone
cxlix. Olodaterol, vilanterol and fluticasone
cl. Olodaterol, vilanterol and budesonide
cli. Olodaterol, carmeterol and mometasone
clii. Olodaterol, carmeterol and fluticasone
cliii. Olodaterol, carmeterol and budesonide
cliv. Olodaterol, bambuterol and mometasone
clv. Olodaterol, bambuterol and fluticasone
clvi. Olodaterol, bambuterol and budesonide
wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of;
i. Aclidinium, salbutamol and fluticasone
ii. Aclidinium, levosalbutamol and fluticasone
iii. Aclidinium, salbutamol and ciclesonide
iv. Aclidinium, levosalbutamol and ciclesonide
v. Aclidinium, salbutamol and budesonide
vi. Aclidinium, levosalbutamol and budesonide
vii. Aclidinium, salbutamol and mometasone
viii. Aclidinium, levosalbutamol and mometasone
ix. Aclidinium, salbutamol and beclometahsone
x. Aclidinium, levosalbutamol and beclometahsone
xi. Aclidinium, salbutamol and triamcinolone
xii. Aclidinium, levosalbutamol and triamcinolone
xiii. Aclidinium, salbutamol and flunisolide
xiv. Aclidinium, levosalbutamol and flunisolide
xv. Aclidinium, salbutamol and dexamethasone
xvi. Aclidinium, levosalbutamol and dexamethasone
xvii. Glycopyrronium, salbutamol and fluticasone
xviii. Glycopyrronium, levosalbutamol and fluticasone
xix. Glycopyrronium, salbutamol and ciclesonide
xx. Glycopyrronium, levosalbutamol and ciclesonide
xxi. Glycopyrronium, salbutamol and budesonide
xxii. Glycopyrronium, levosalbutamol and budesonide
xxiii. Glycopyrronium, salbutamol and mometasone
xxiv. Glycopyrronium, levosalbutamol and mometasone
xxv. Glycopyrronium, salbutamol and beclometahsone
xxvi. Glycopyrronium, levosalbutamol and beclometahsone
xxvii. Glycopyrronium, salbutamol and triamcinolone
xxviii. Glycopyrronium, levosalbutamol and triamcinolone
xxix. Glycopyrronium, salbutamol and flunisolide
xxx. Glycopyrronium, levosalbutamol and flunisolide
xxxi. Glycopyrronium, salbutamol and dexamethasone
xxxii. Glycopyrronium, levosalbutamol and dexamethasone
xxxiii. Daratropium, salbutamol and fluticasone
xxxiv. Daratropium, levosalbutamol and fluticasone
xxxv. Daratropium, salbutamol and ciclesonide
xxxvi. Daratropium, levosalbutamol and ciclesonide
xxxvii. Daratropium, salbutamol and budesonide
xxxviii. Daratropium, levosalbutamol and budesonide
xxxix. Daratropium, salbutamol and mometasone
xl. Daratropium, levosalbutamol and mometasone
xli. Daratropium, salbutamol and beclometahsone
xlii. Daratropium, levosalbutamol and beclometahsone
xliii. Daratropium, salbutamol and triamcinolone
xliv. Daratropium, levosalbutamol and triamcinolone
xlv. Daratropium, salbutamol and flunisolide
xlvi. Daratropium, levosalbutamol and flunisolide
xlvii. Daratropium, salbutamol and dexamethasone
xlviii. Daratropium, levosalbutamol and dexamethasone
xlix. Indacaterol, salbutamol and fluticasone
l. Indacaterol, levosalbutamol and fluticasone
li. Indacaterol, salbutamol and ciclesonide
lii. Indacaterol, levosalbutamol and ciclesonide
liii. Indacaterol, salbutamol and budesonide
liv. Indacaterol, levosalbutamol and budesonide
Iv. Indacaterol, salbutamol and mometasone
Ivi. Indacaterol, levosalbutamol and mometasone
Ivii. Indacaterol, salbutamol and beclometahsone
Iviii. Indacaterol, levosalbutamol and beclometahsone
lix. Indacaterol, salbutamol and triamcinolone
Ix. Indacaterol, levosalbutamol and triamcinolone
Ixi. Indacaterol, salbutamol and flunisolide
Ixii. Indacaterol, levosalbutamol and flunisolide
Ixiii. Indacaterol, salbutamol and dexamethasone
Ixiv. Indacaterol, levosalbutamol and dexamethasone
lxv. Vilanterol, salbutamol and fluticasone
lxvi. Vilanterol, levosalbutamol and fluticasone
lxvii. Vilanterol, salbutamol and budesonide
lxviii. Vilanterol, levosalbutamol and budesonide
Ixix. Vilanterol, salbutamol and ciclesonide
lxx. Vilanterol, levosalbutamol and ciclesonide
lxxi. Vilanterol, salbutamol and mometasone
lxxii. Vilanterol, levosalbutamol and mometasone
lxxiii. Vilanterol, salbutamol and beclomethasone
lxxiv. Vilanterol, levosalbutamol and beclomethasone
lxxv. Vilanterol, salbutamol and triamcinolone
lxxvi. Vilanterol, levosalbutamol and triamcinolone
lxxvii. Vilanterol, salbutamol and flunisolide
lxxviii. Vilanterol, levosalbutamol and flunisolide
lxxix. Vilanterol, salbutamol and dexamethasone
lxxx. Vilanterol, levosalbutamol and dexamethasone
lxxxi. Carmeterol, salbutamol and fluticasone
lxxxii. Carmeterol, levosalbutamol and fluticasone
lxxxiii. Carmeterol, salbutamol and ciclesonide
lxxxiv. Carmeterol, levosalbutamol and ciclesonide
lxxxv. Carmeterol, salbutamol and budesonide
lxxxvi. Carmeterol, levosalbutamol and budesonide
lxxxvii. Carmeterol, salbutamol and mometasone
Ixxxviii. Carmeterol, levosalbutamol and mometasone
lxxxix. Carmeterol, salbutamol and beclomethasone
xc. Carmeterol, levosalbutamol and beclomethasone
xci. Carmeterol, salbutamol and triamcinolone
xcii. Carmeterol, levosalbutamol and triamcinolone
xciii. Carmeterol, salbutamol and flunisolide
xciv. Carmeterol, levosalbutamol and flunisolide
xcv. Carmeterol, salbutamol and dexamethasone
xcvi. Carmeterol, levosalbutamol and dexamethasone
xcvii. Olodaterol, salbutamol and fluticasone
xcviii. Olodaterol, levosalbutamol and fluticasone
xcix. Olodaterol, salbutamol and ciclesonide
c. Olodaterol, levosalbutamol and ciclesonide
ci. Olodaterol, salbutamol and budesonide
cii. Olodaterol, levosalbutamol and budesonide
ciii. Olodaterol, salbutamol and mometasone
civ. Olodaterol, levosalbutamol and mometasone
cv. Olodaterol, salbutamol and beclomethasone
cvi. Olodaterol, levosalbutamol and beclomethasone
cvii. Olodaterol, salbutamol and triamcinolone
cviii. Olodaterol, levosalbutamol and triamcinolone
cix. Olodaterol, salbutamol and flunisolide
cx. Olodaterol, levosalbutamol and flunisolide
cxi. Olodaterol, salbutamol and dexamethasone
cxii. Olodaterol, levosalbutamol and dexamethasone wherein the above therapeutic agents can be present as a pharmaceutically acceptable salt or ester thereof, or in enantiomerically pure form or as a racemic mixture.

According to another embodiment, the pharmaceutical compositions comprise any of the following combinations which are suitable for administration separately, sequentially or together in effective amounts of;
i. Formoterol, budesonide and tiotropium
ii. Salmeterol, fluticasone and tiotropium
iii. Carmoterol, tiotropium and fluticasone
iv. Salbutamol, formoterol and budesonide
v. Salbutamol, salmeterol and fluticasone
vi. Salbutamol, arformoterol and fluticasone
wherein the above therapeutic agents can be present as a pharmaceutically acceptable salt or ester thereof, or in enantiomerically pure form or as a racemic mixture.

According to a preffered embodiment of the invention, the therapeutically effective amount of said pharmaceutical compositions are administered once a day and/ or administered twice a day.

According to a preffered embodiment, the pharmaceutical compositions are used for in the treatment of respiratory conditions selected from asthma and chronic obstructive pulmonary disease and other obstructive airways diseases. In particular, the combinations of compounds of the present invention are useful in the treatment of respiratory diseases and conditions comprising, asthma, acute respiratory distress syndrome, chronic pulmonary inflammatory disease, bronchitis, chronic bronchitis, chronic obstructive pulmonary (airway) disease, and silicosis; or immune diseases and conditions comprising: allergic rhinitis and chronic sinusitis.
According to a further embodiment, the pharmaceutical compositions are suitable for administarion separately, sequentially or together in effective amounts, together with a moisture tight and high barrier sealed blister or together with a capsule

In particular, the blister comprises aluminium to prevent ingress of moisture whereby the fine particle fraction (FPF) of the pharmaceutical composition dose is preserved. Furthermore, the blister is a high barrier sealed against moisture. Thus, the blister does not release any water to the dose and ingress of moisture from the exterior into the container is thereby prevented.

In a further preferred embodiment of the invention, the dry powder is in a capsule, which can be a pharmaceutically acceptable natural or synthetic polymer such as gelatin or hydroxypropyl methylcellulose.

In a preferred embodiment, the pharmaceutical compositions are suitable for administration separately, sequentially or together in effective amounts, together with an inhalation device. The device is preferably dry powder inhaler including the blister or the capsule described above.

In a further embodiment, the device in which the pharmaceutical composition is within the blister comprise at least one lock mechanism, enabling the device to remain locked in both positions in which the device is ready for inhalation and the lid is in the closed position, and further enabling the device to setup again automatically, when the lid is closed.

In a further embodiment, the invention relates to a pharmaceutical kit comprising the drugs having amine and one or more additional active agents, in separate unit dosage forms, said forms being suitable for administration separately, sequentially or together in effective amounts, together with one or more inhalation devices for administration of drugs having amine and one or more additional active agents which are LAMAs, LABAs, SABAs and/or inhaled corticosteroids as described in detail above.

In a further embodiment, the process for making the pharmaceutical compositions for inhalation of the present invention comprises the following steps;
To obtain a homogenous mixture first half of the coarse mannitol particules are added to a glass container later on fine mannitol particles are added and active ingredients are added to this mixture and blended in a turbula shaker. Then this mixture is elected. This election is not a milling, the aim of this election is to obtain a homogenous mixture. The rest of the coarse mannitol particels are added to this elected mixture during blending. Final powder mixture is furthermore blended and then filled into blisters or capsules.

This invention is further defined by reference to the following examples. In the following examples the drugs having amine has the ratio between the median particle size (d₅₀) and d₉₀ is about 0.50. Although these examples are not intended to limit the scope of the present invention, it should be considered in the light of the description detailed above.

### Example - 1:

**Table 1.1**

| **Ingredients** | **Amount % (w/w)** |
|---|---|
| drugs having amine | 0.01 - 10.0 |
| fine mannitol | 2.0 - 20.0 |
| coarse mannitol | 20.0 - 98.0 |

### Particle size of the drugs having amine (µm):

d₁₀ : 0.10 - 1.0 d₅₀: 1.0 - 2.5 d₉₀: 2.5 - 5.0

### Particle size of the fine mannitol (µm):

d₁₀ : 1.0 - 4.0 d₅₀: 4.0 - 7.0 d₉₀: 7.0 - 15.0

### Particle size of the coarse mannitol (µm):

d₁₀ : 10 - 50 d₅₀: 50 - 75 d₉₀: 75 - 250

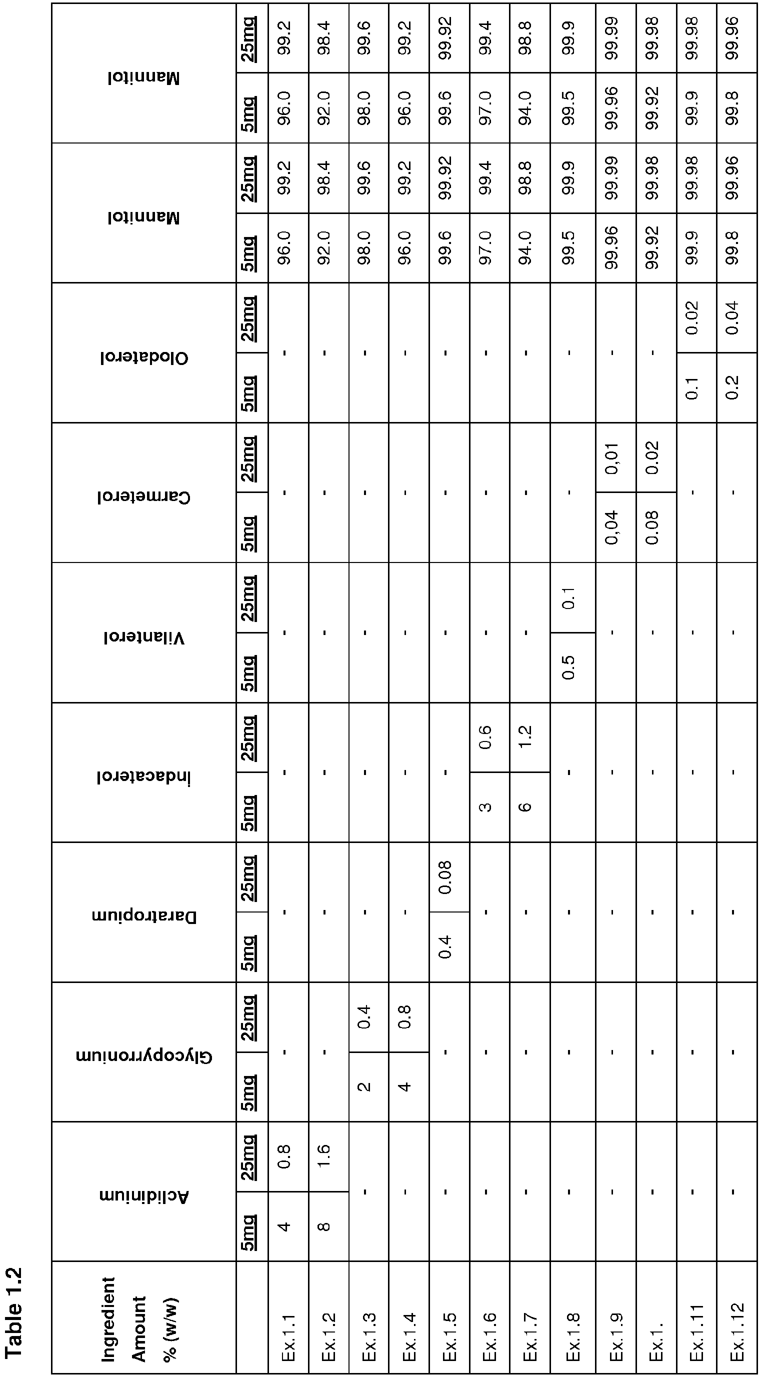

### Example - 2:

**Table 2.1**

| **ingredients** | **Amount % (w/w)** |
|---|---|
| drugs having amine | 0.01 - 10.0 |
| LABAs | 0.01 - 10.0 |
| fine mannitol | 2.0 - 20.0 |
| coarse mannitol | 20.0 - 98.0 |

### Particle size of ;

| | | | |
|---|---|---|---|
| LABAs (µm) | : d₁₀ : 0.10-1.0 | d₅₀: 1.0 - 2.5 | d₉₀: 2.5 - 5.0 |
| the drugs having amine (µm) | : d₁₀ : 0.10 - 1.0 | d₅₀: 1.0 - 2.5 | d₉₀: 2.5 - 5.0 |
| fine mannitol (µm) | : d₁₀ : 1.0-4.0 | d₅₀: 4.0 - 7.0 | d₉₀: 7.0 - 15.0 |
| coarse mannitol (µm) | : d₁₀ : 10-50 | d₅₀: 50 - 75 | d₉₀: 75 - 250 |

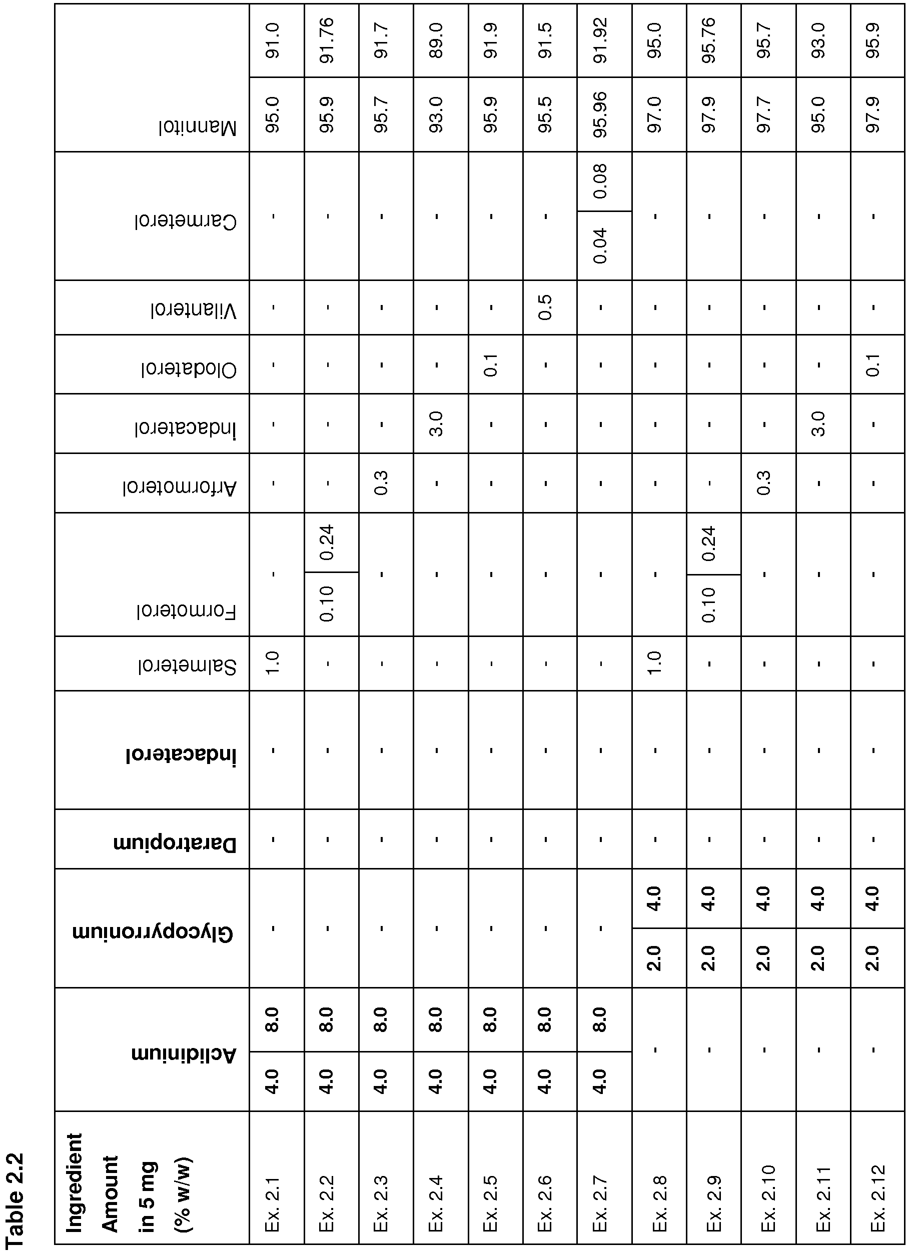

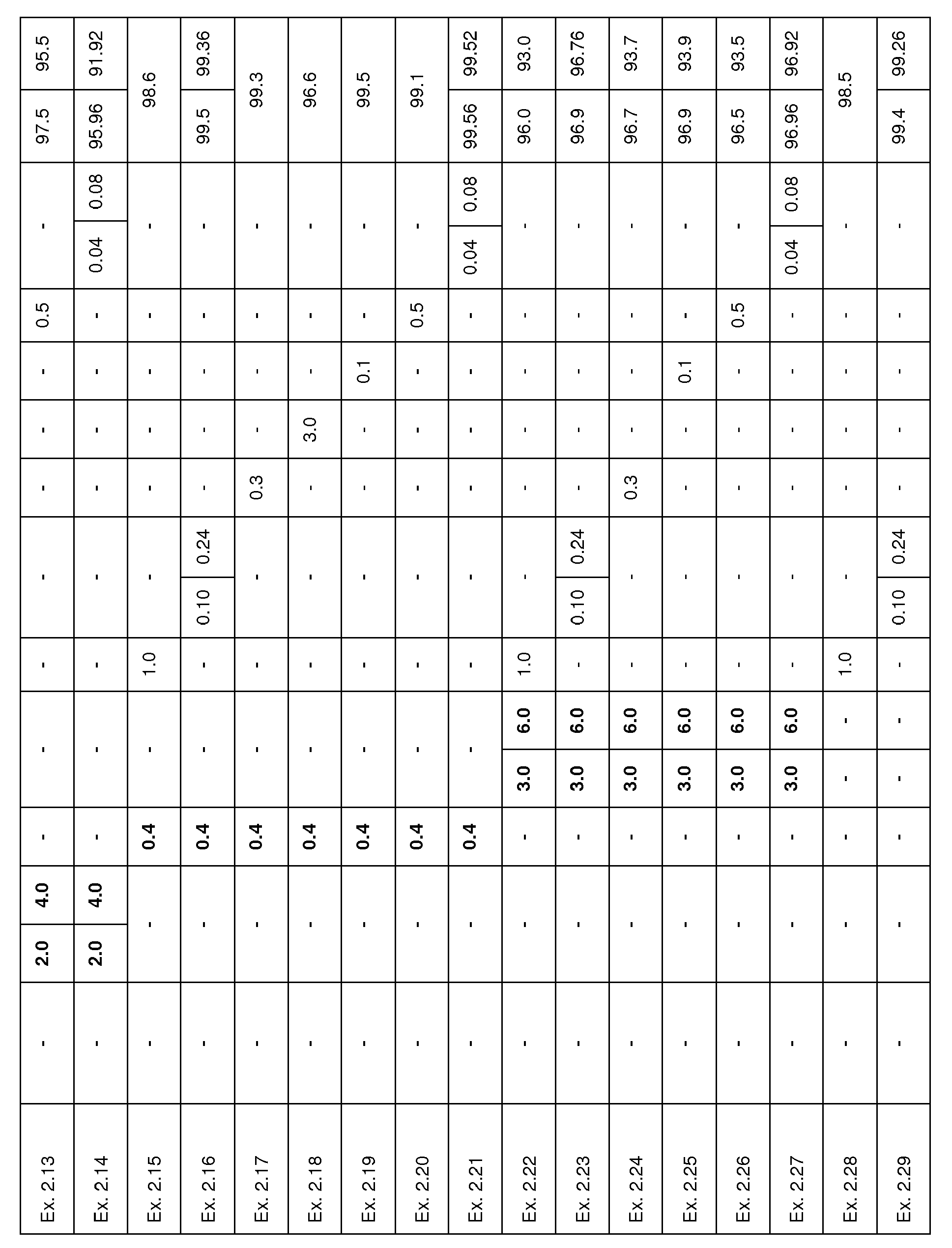

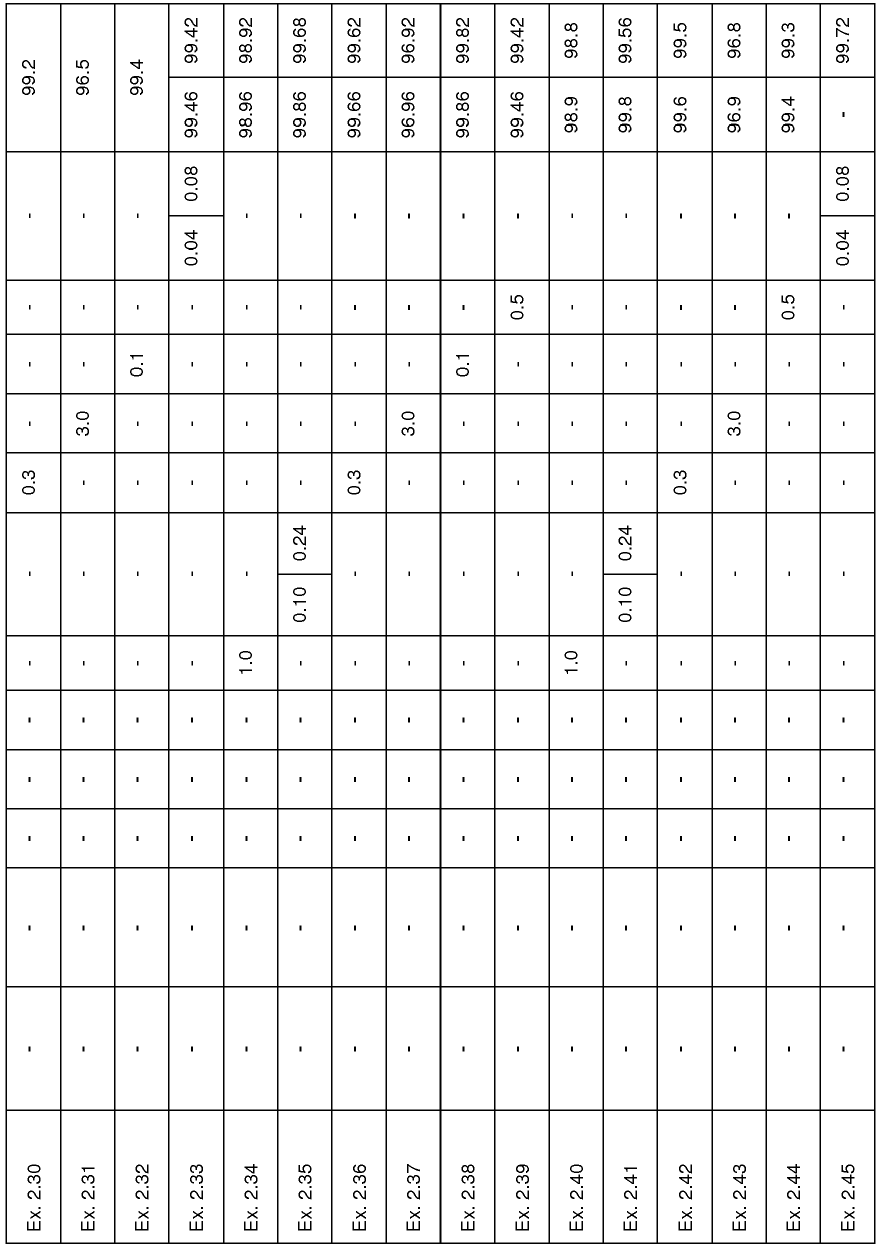

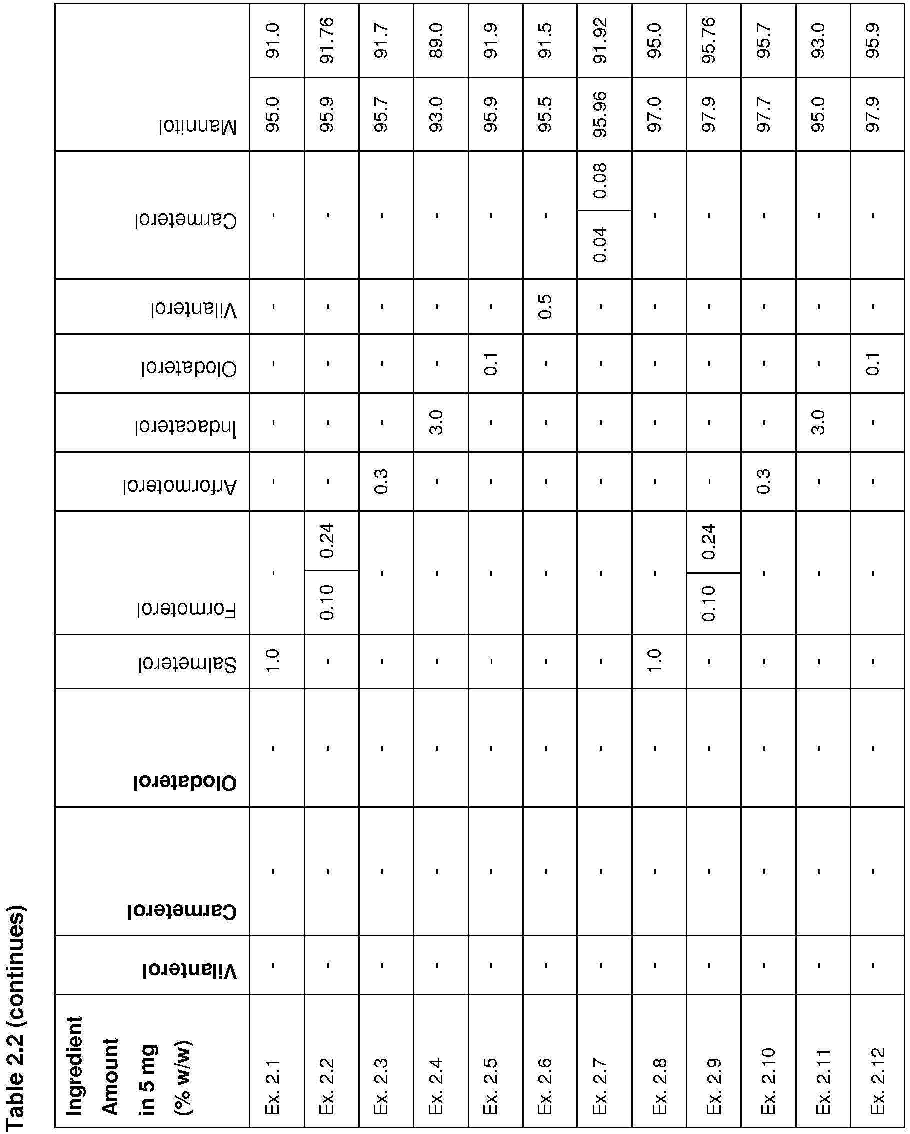

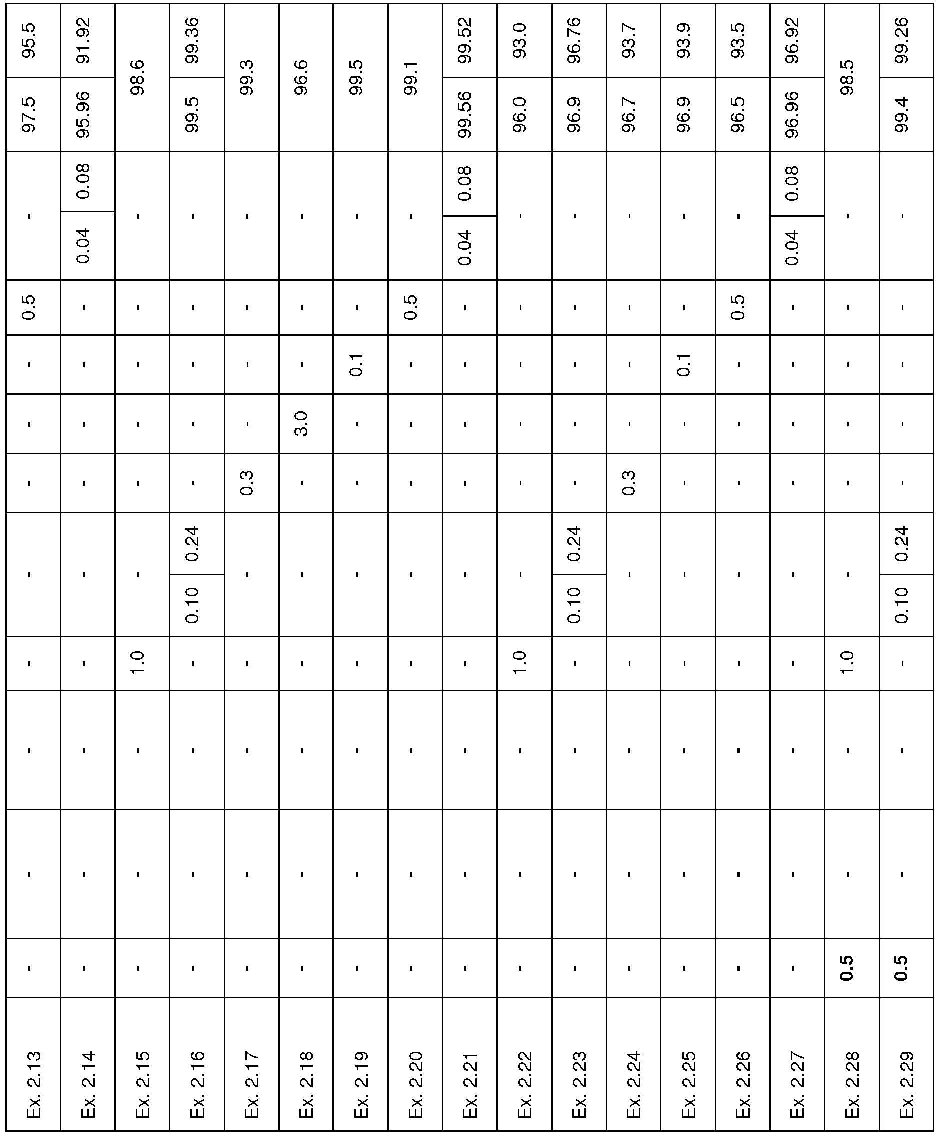

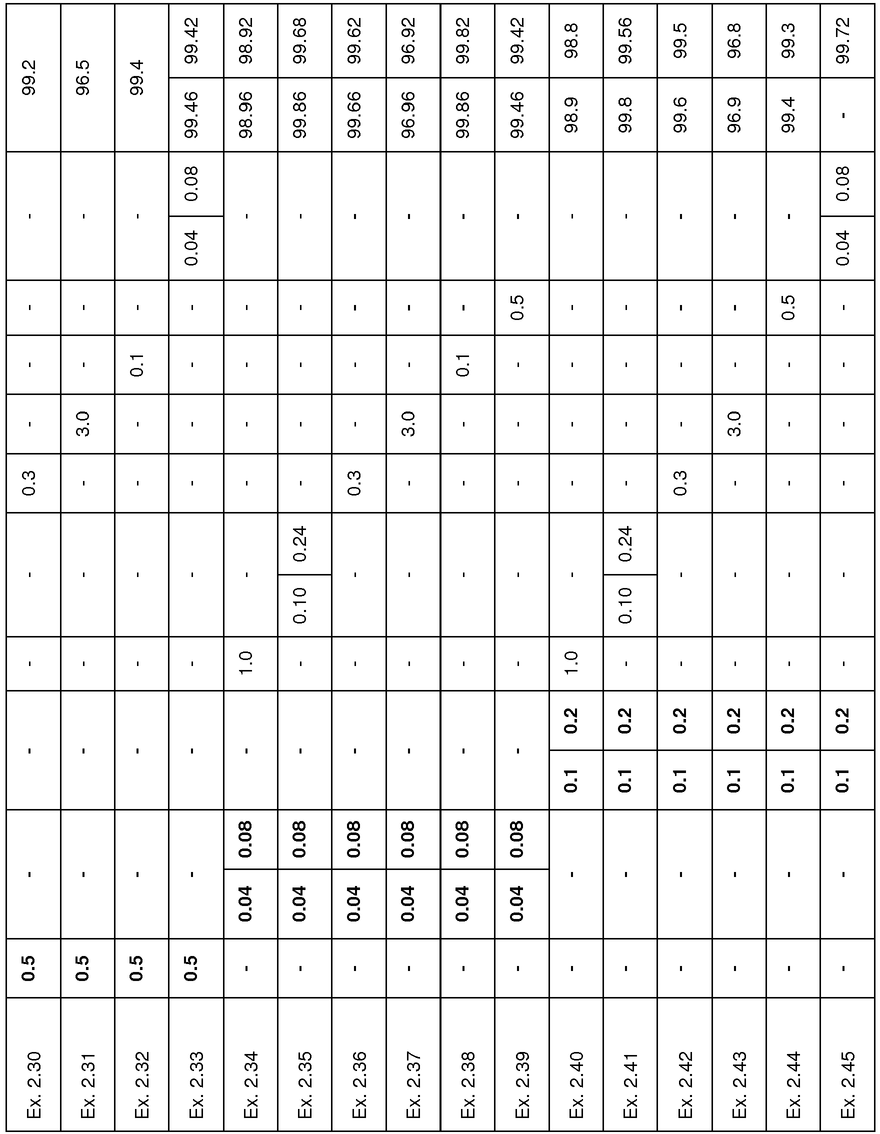

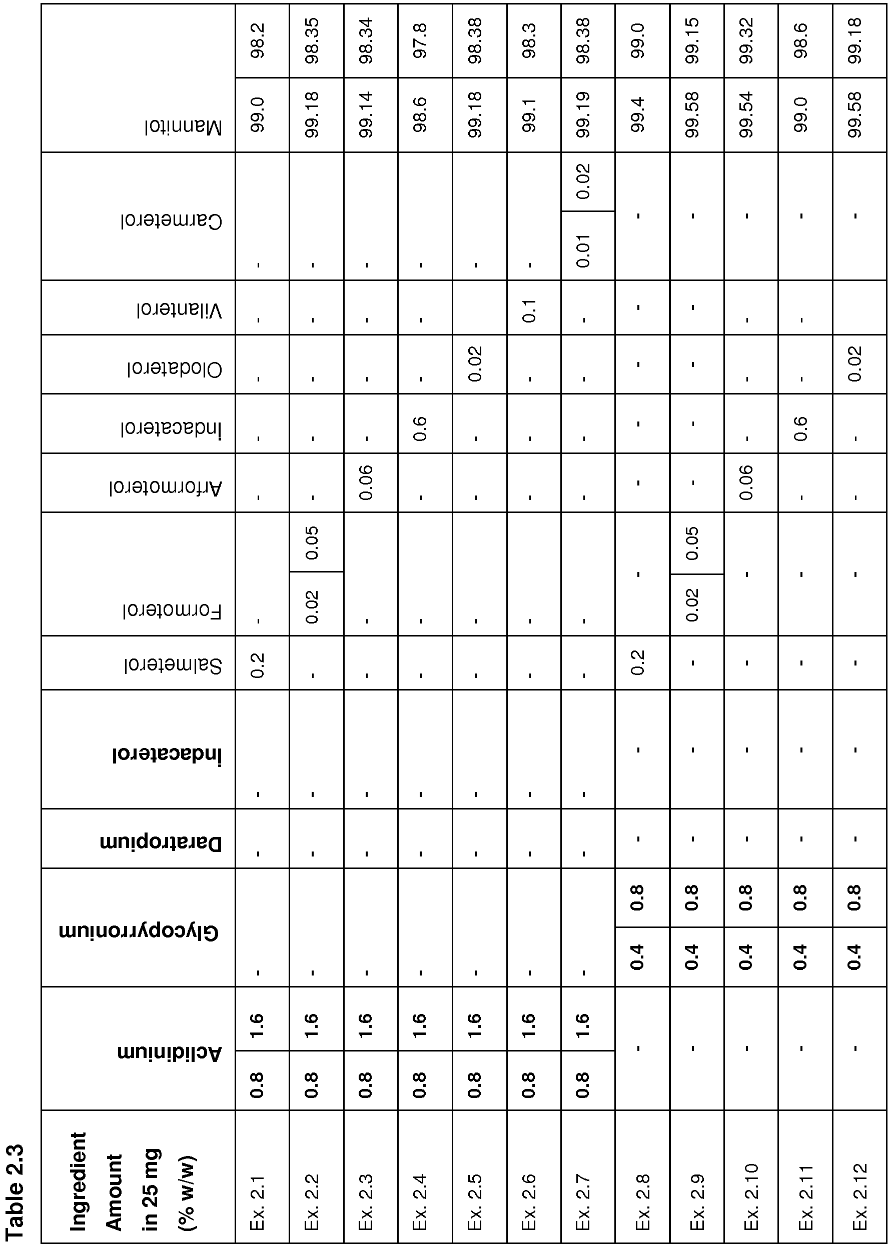

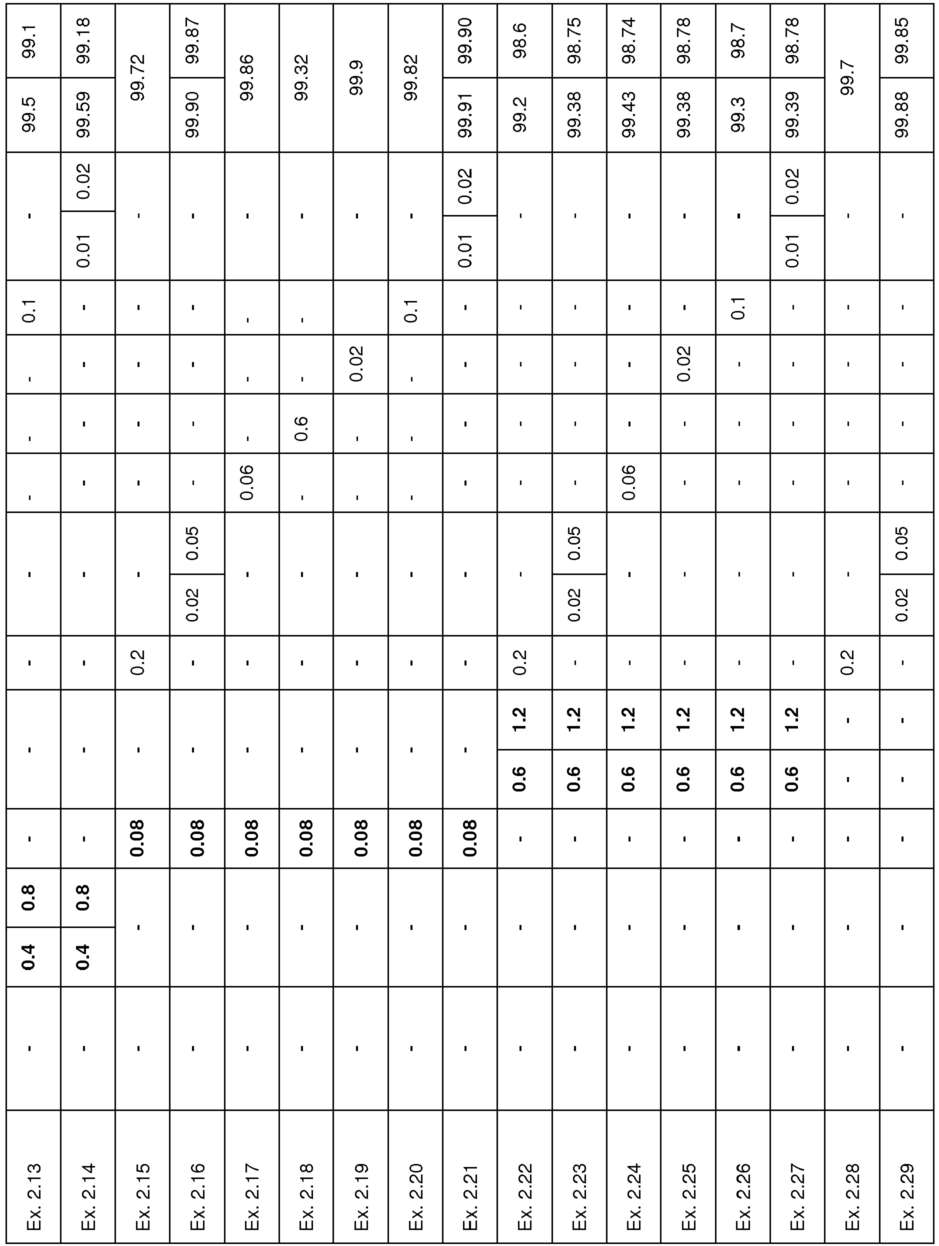

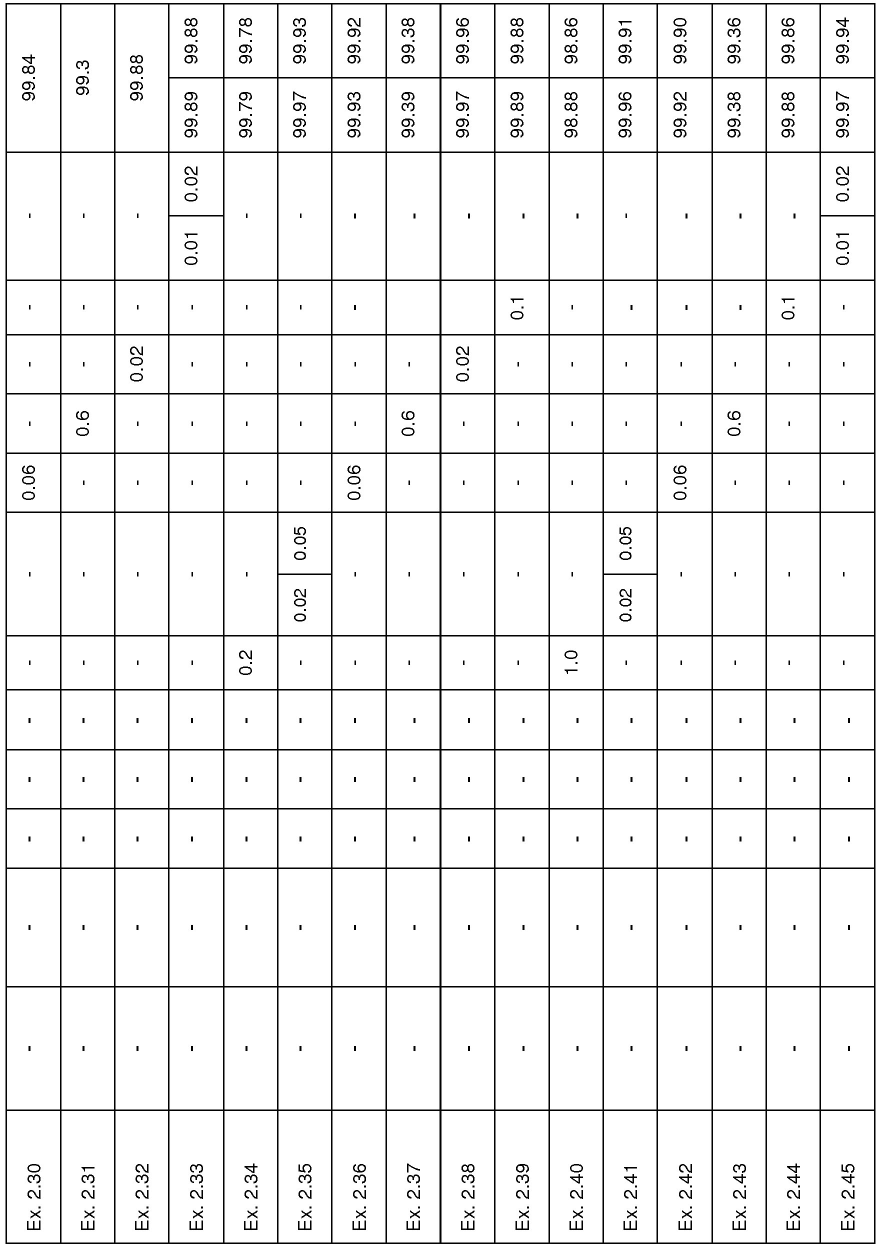

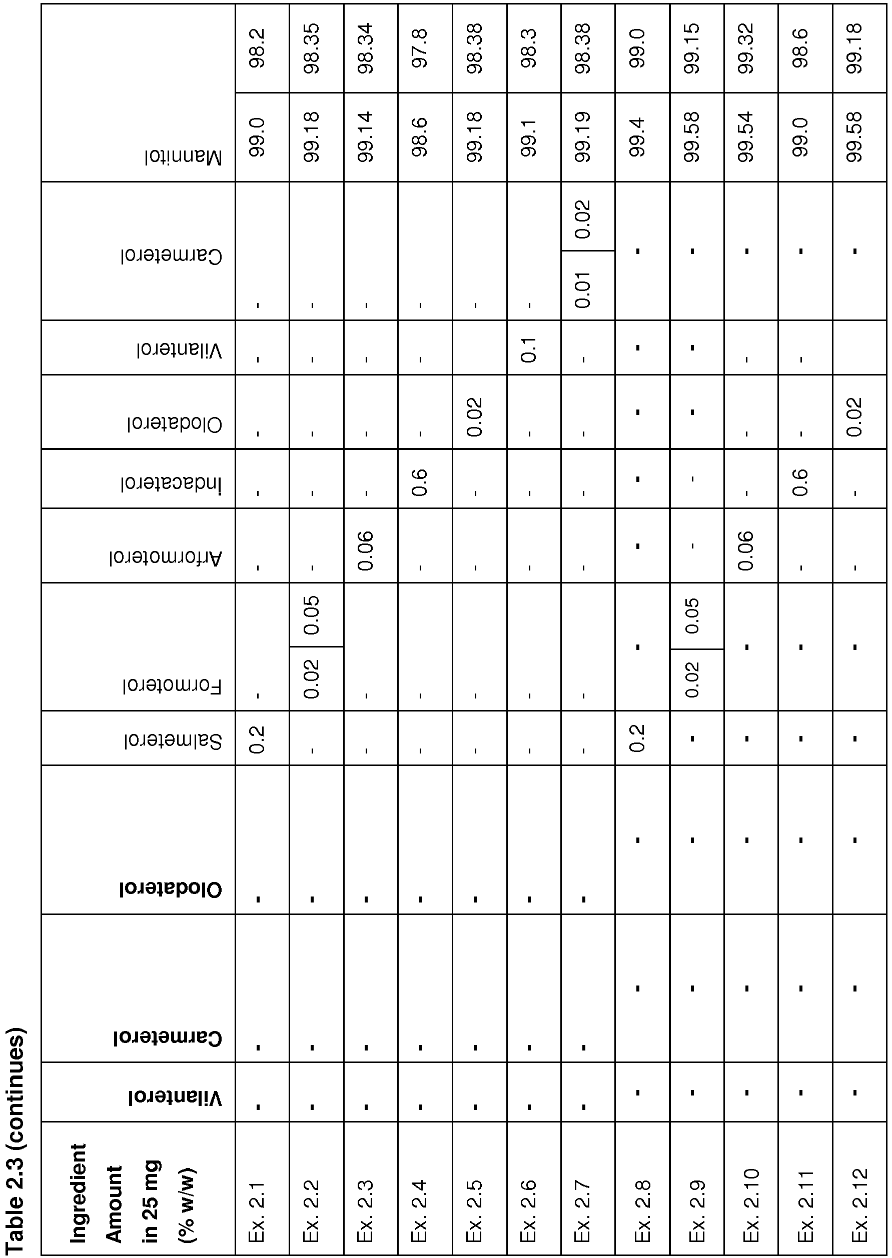

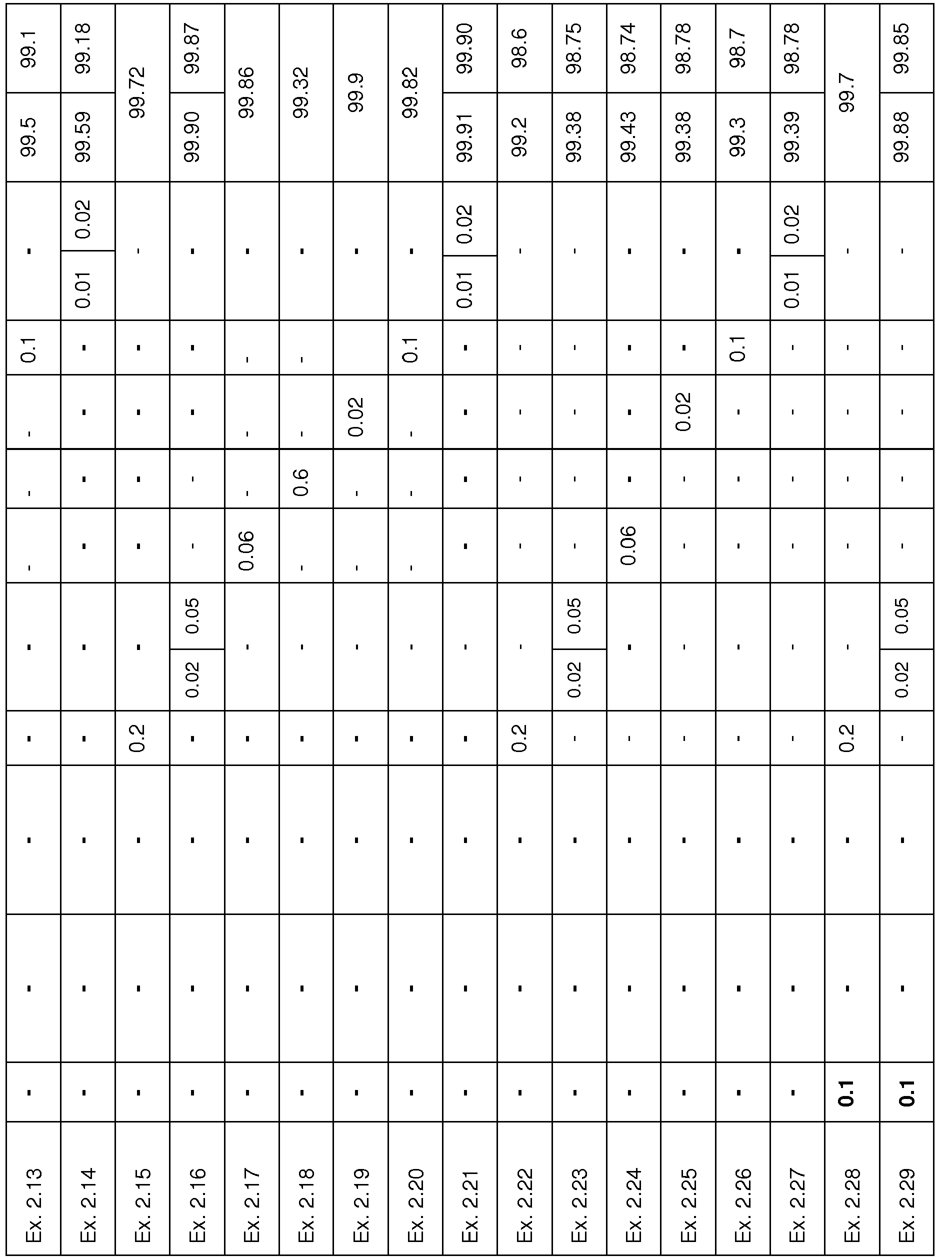

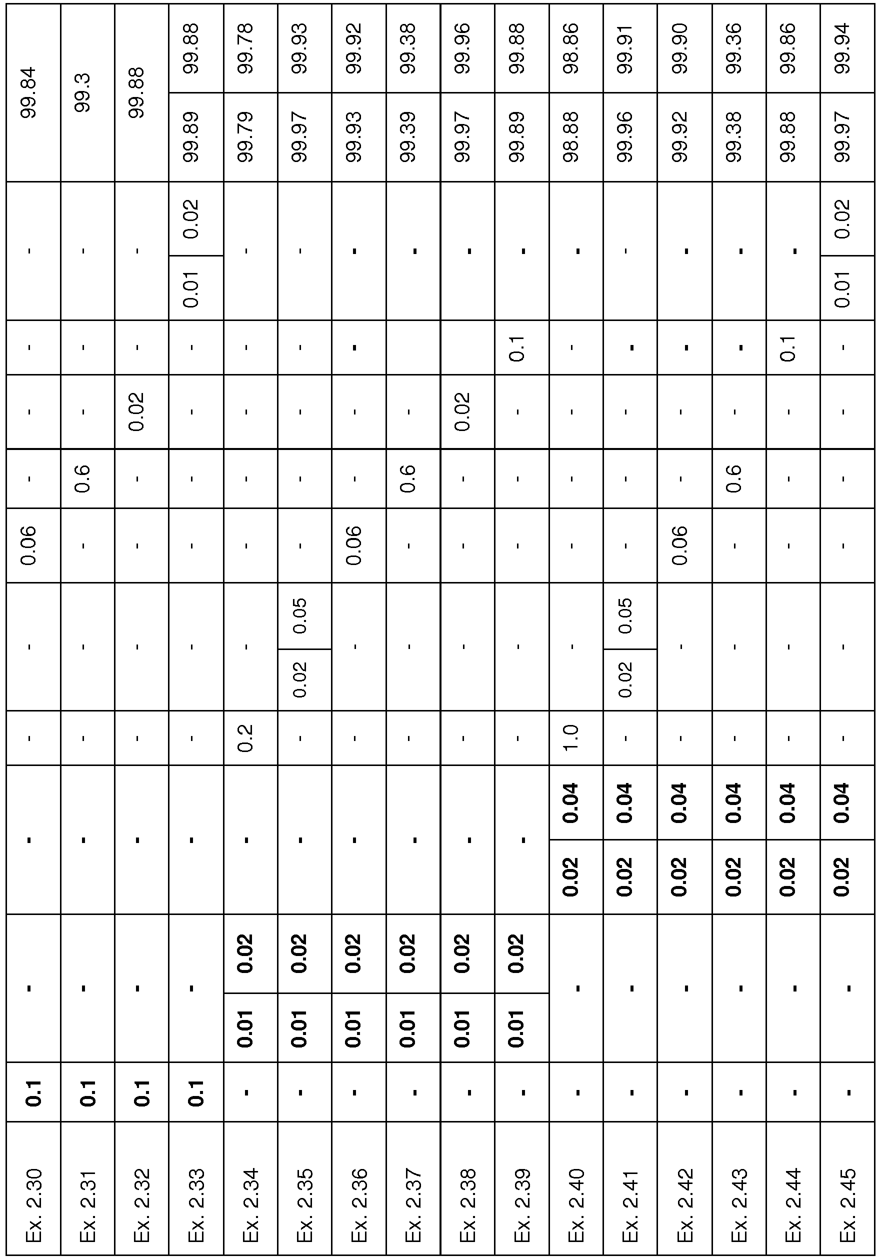

### Examples - 3:

**Table 3.1**

| **ingredients** | **Amount % (w/w)** |
|---|---|
| drugs having amine | 0.01 - 10.0 |
| LAMAs | 0.01 - 10.0 |
| fine mannitol | 2.0 - 20.0 |
| coarse mannitol | 20.0 - 98.0 |

### Particle size of ;

| | | | |
|---|---|---|---|
| the drugs having amine (µm) | : d₁₀ : 0.10 - 1.0 | d₅₀: 1.0 - 2.5 | d₉₀: 2.5 - 5.0 |
| LAMAs(µm) | : d₁₀ : 0.10 - 1.0 | d₅₀: 1.0 - 2.5 | d₉₀: 2.5 - 5.0 |
| fine mannitol (µm) | : d₁₀ : 1.0 - 4.0 | d₅₀: 4.0 - 7.0 | d₉₀: 7.0 - 15.0 |
| coarse mannitol (µm) | : d₁₀ : 10 - 50 | d₅₀: 50 -75 | d₉₀: 75 - 250 |

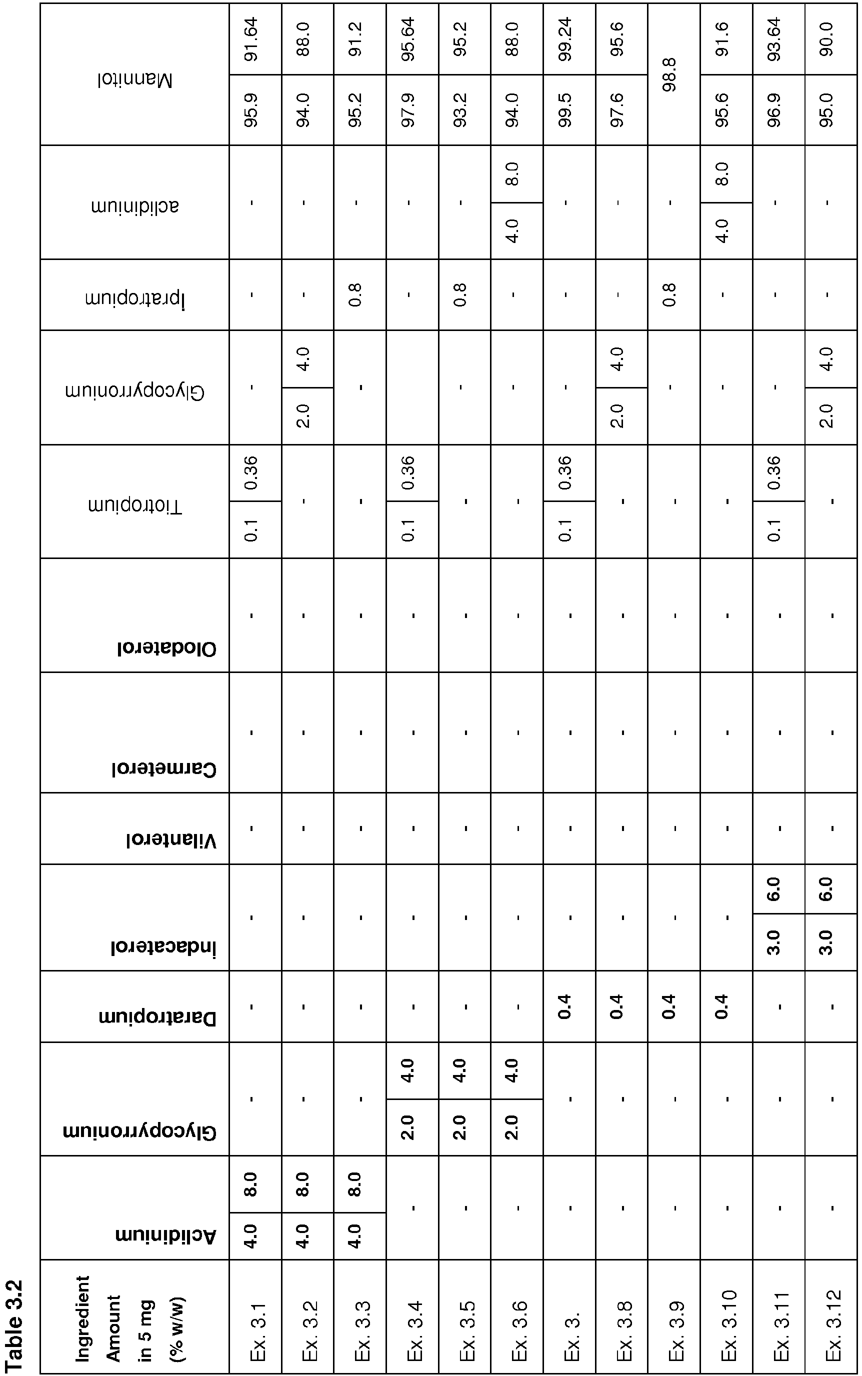

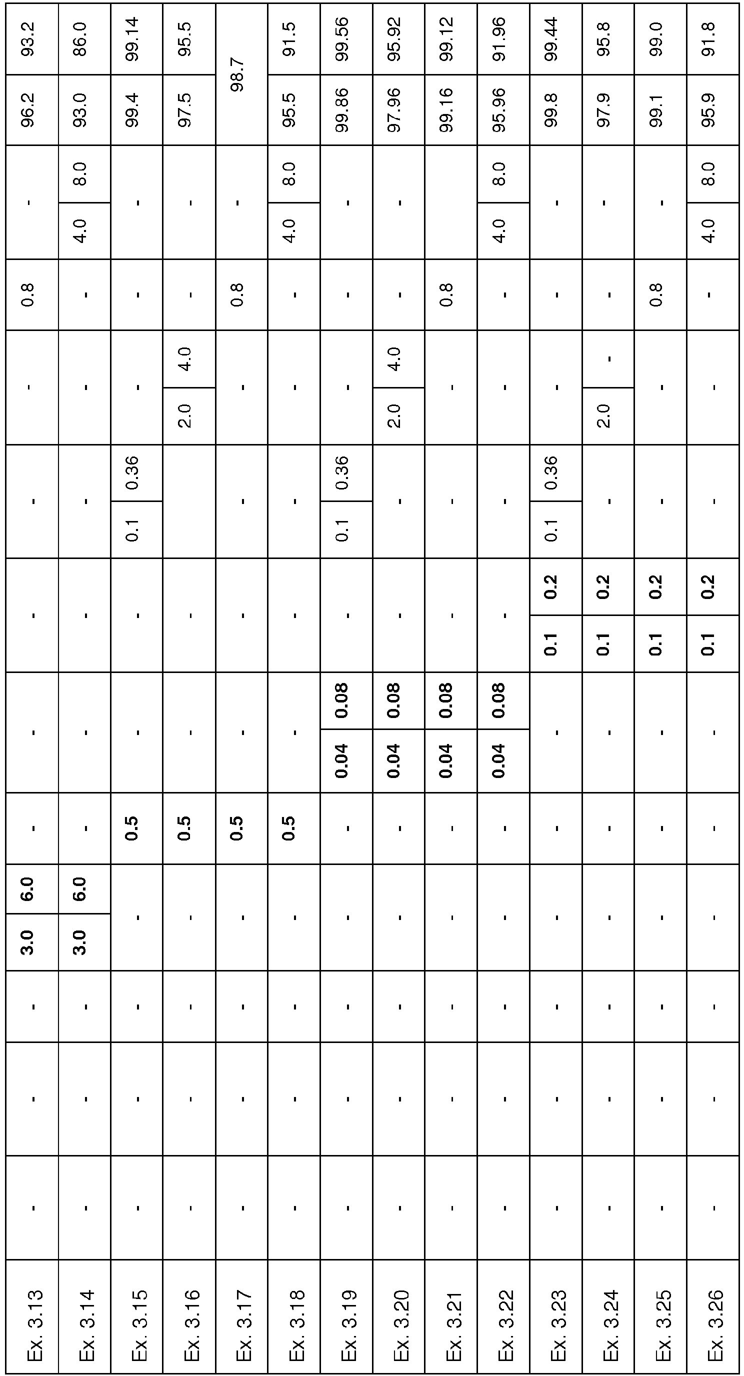

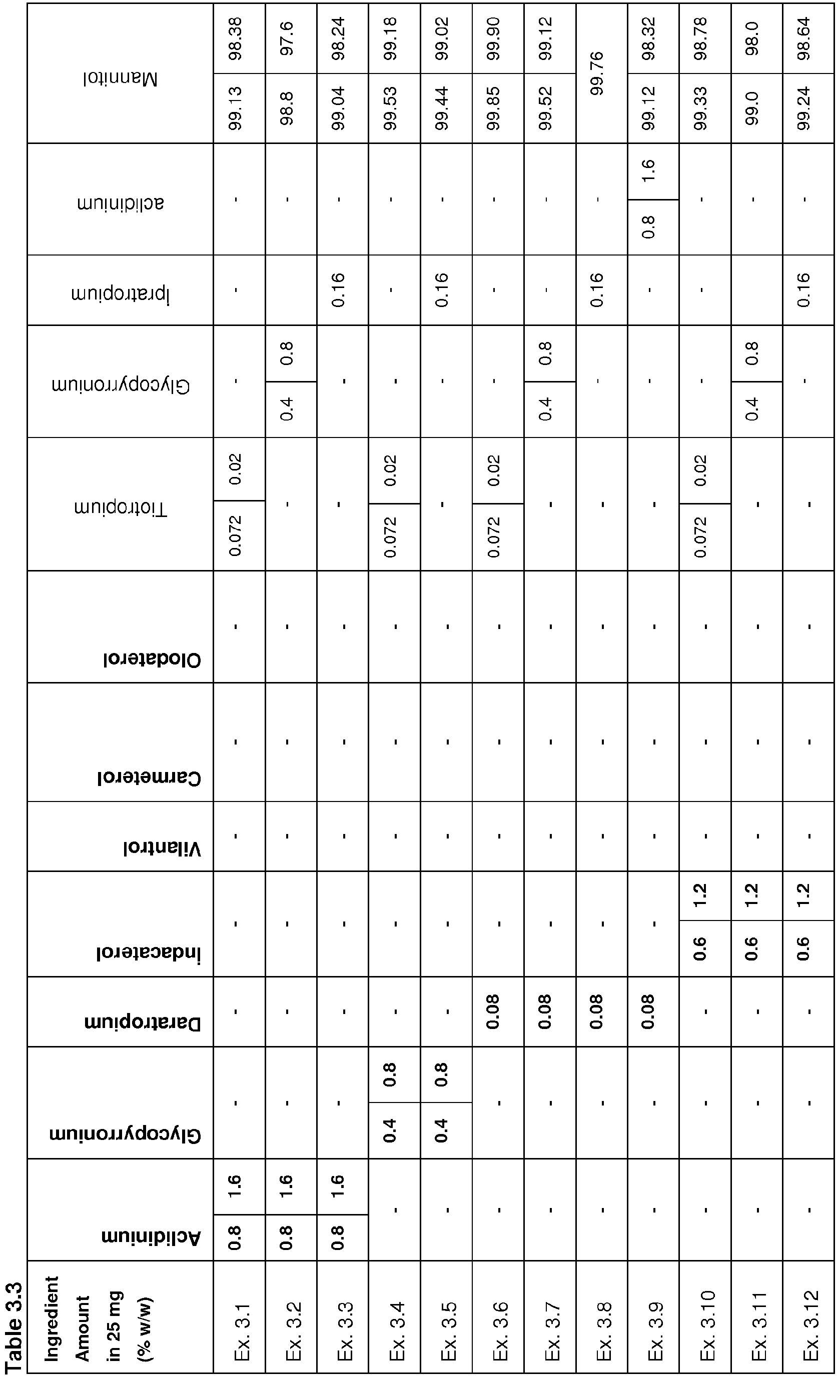

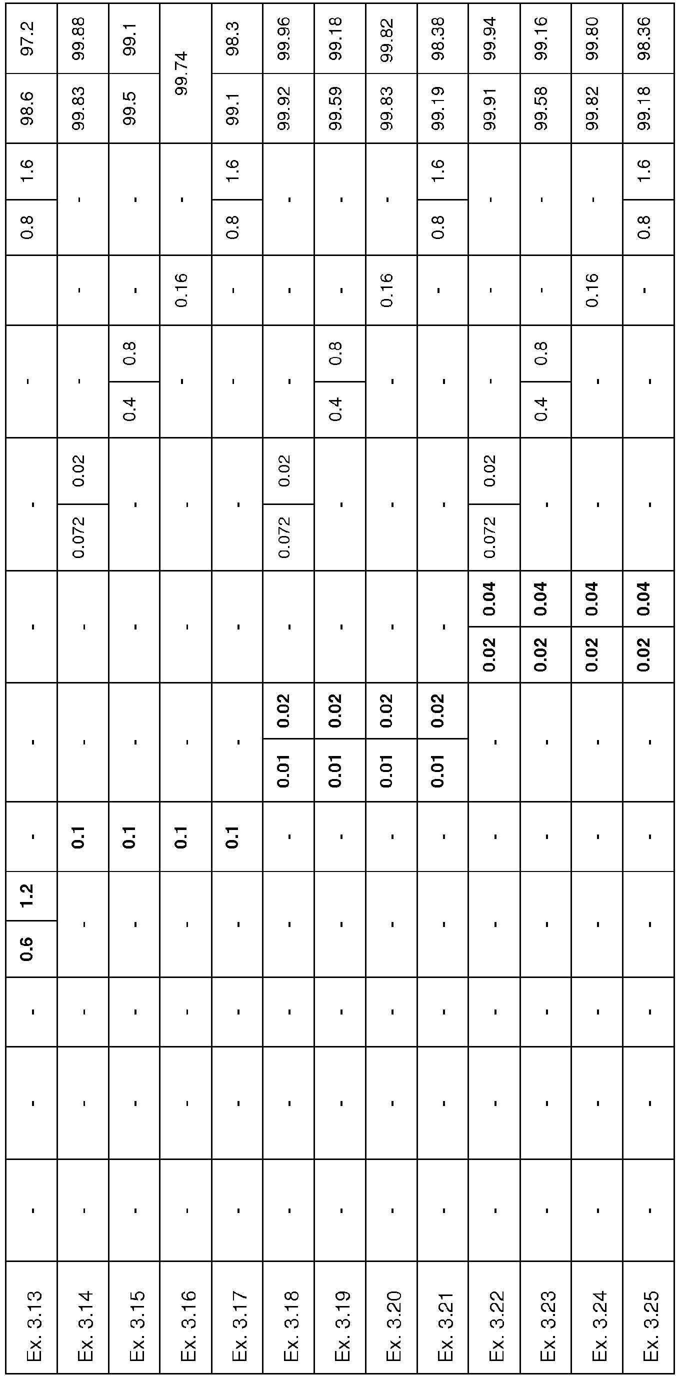

### Examples - 4:

**Table 4.1**

| **Ingredients** | **Amount % (w/w)** |
|---|---|
| drugs having amine | 0.01 - 10.0 |
| SABAs | 0.01 - 10.0 |
| fine mannitol | 2.0 - 20.0 |
| coarse mannitol | 20.0 - 98.0 |

### Particle size of;

| | | | |
|---|---|---|---|
| SABAs (µm) | : d₁₀ : 0.10 - 1.0 | d₅₀: 1.0 - 2.5 | d₉₀: 2.5 - 5.0 |
| the drugs having amine (µm) | : d₁₀ : 0.10 - 1.0 | d₅₀: 1.0 - 2.5 | d₉₀: 2.5 - 5.0 |
| fine mannitol (µm) | : d₁₀ : 1.0 - 4.0 | d₅₀: 4.0 - 7.0 | d₉₀: 7.0 - 15.0 |
| coarse mannitol (µm) | : d₁₀ : 10 - 50 | d₅₀: 50 -75 | d₉₀: 75 - 250 |

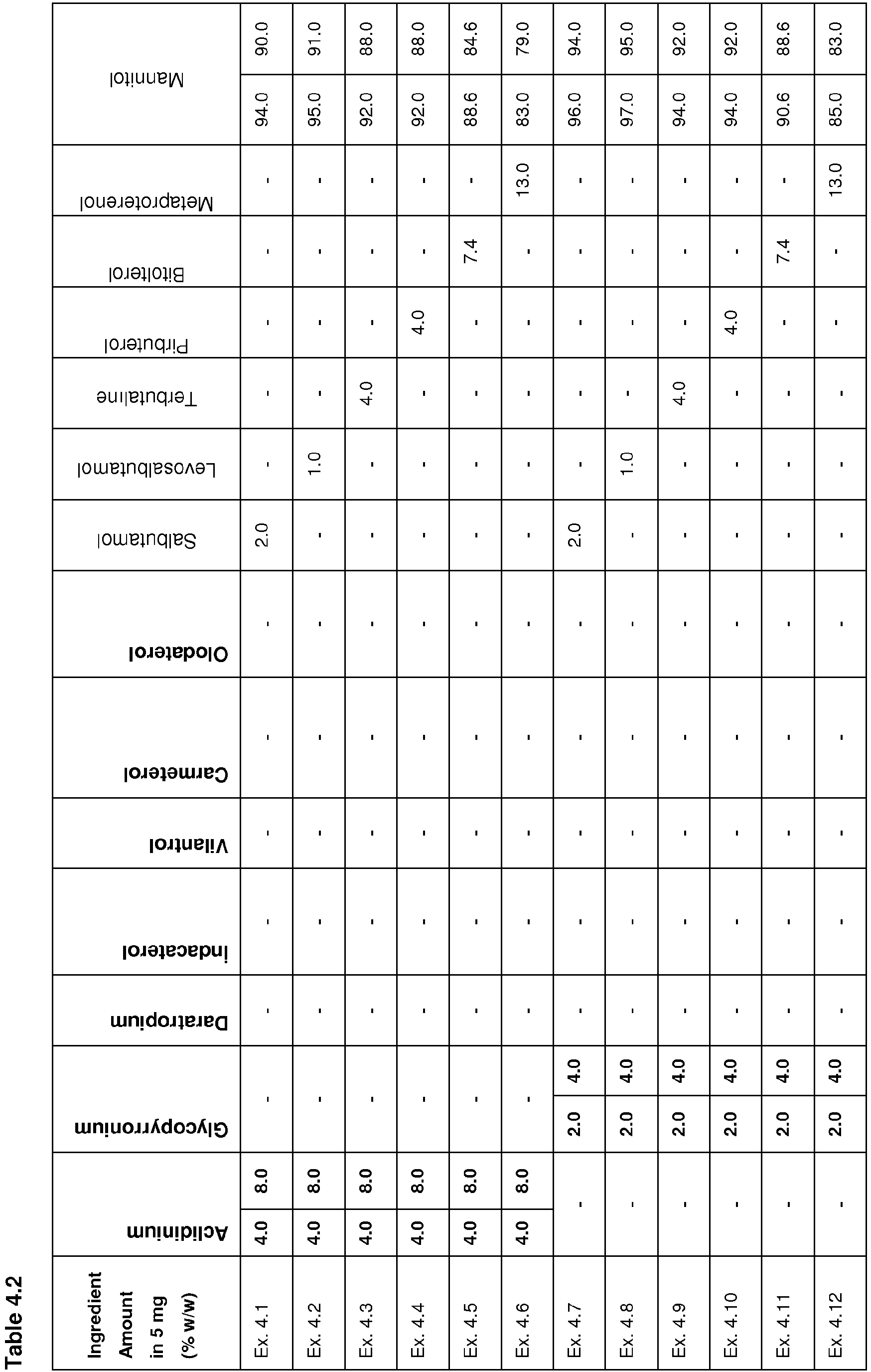

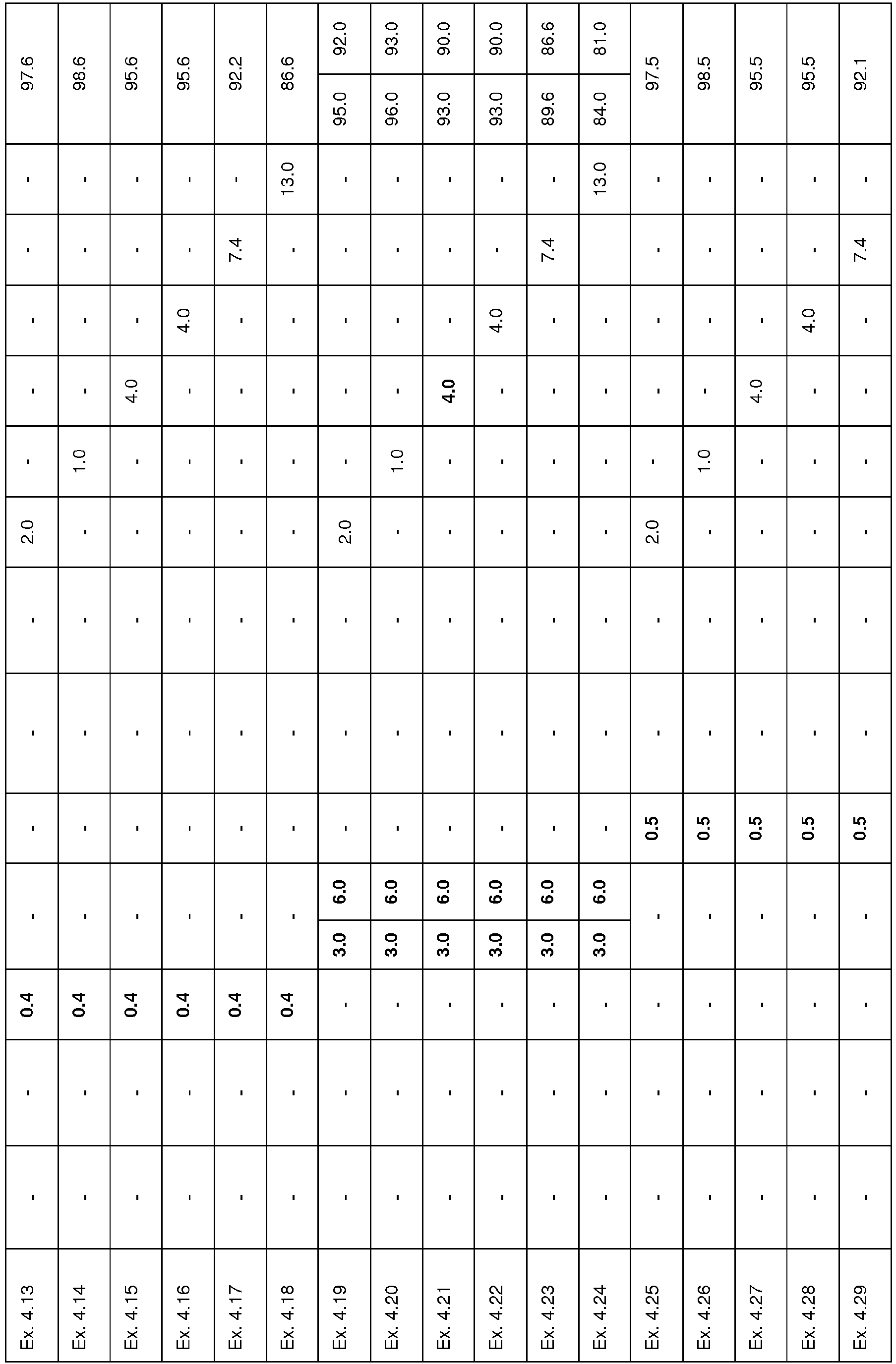

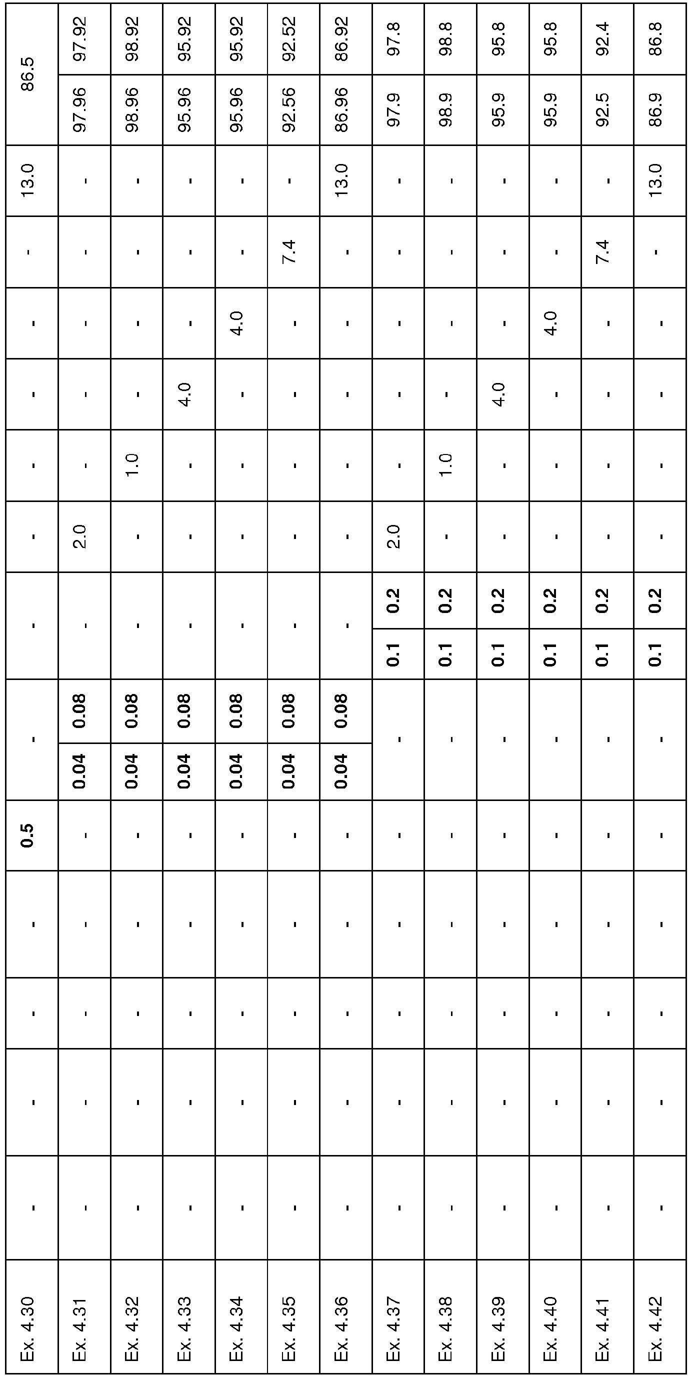

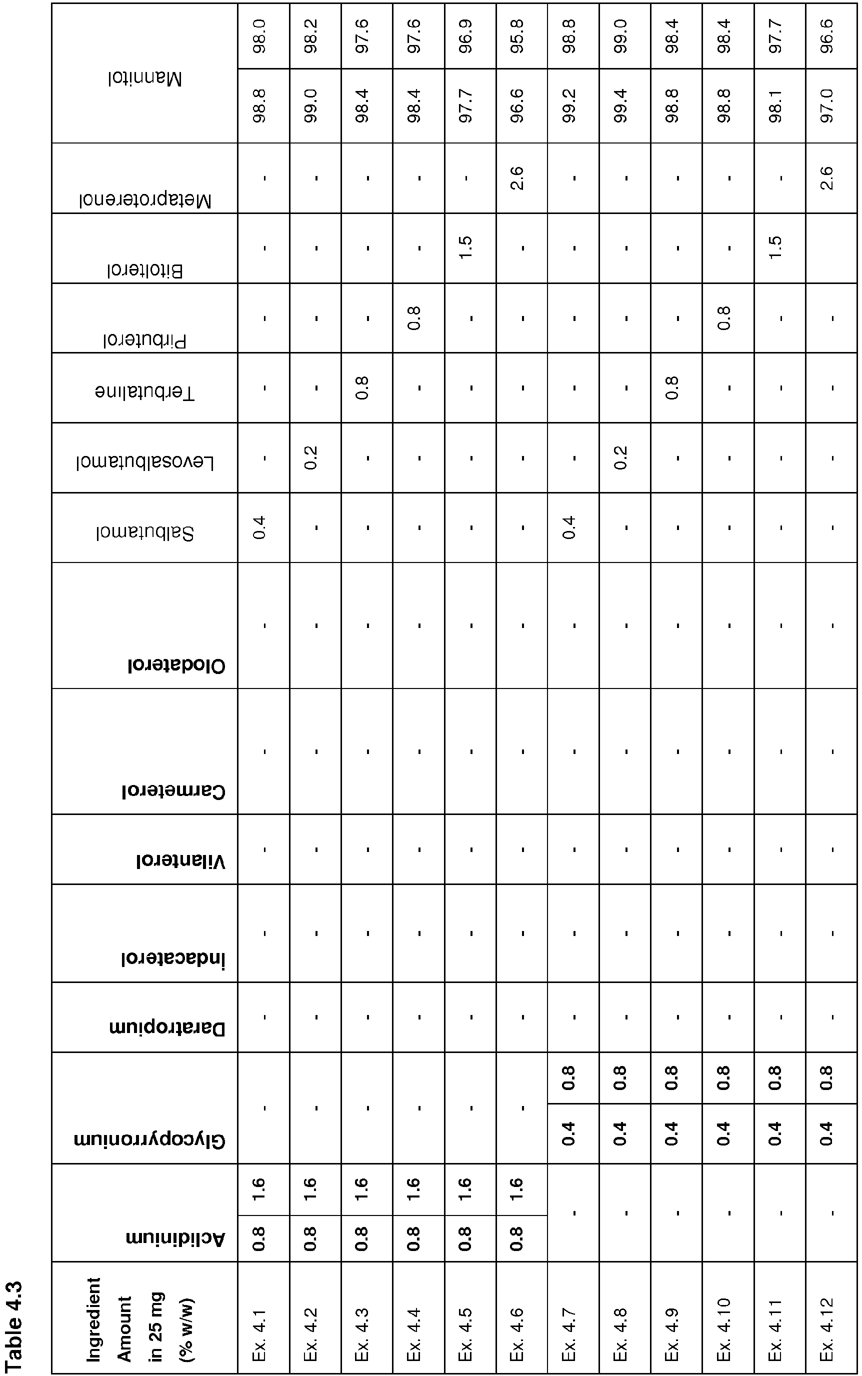

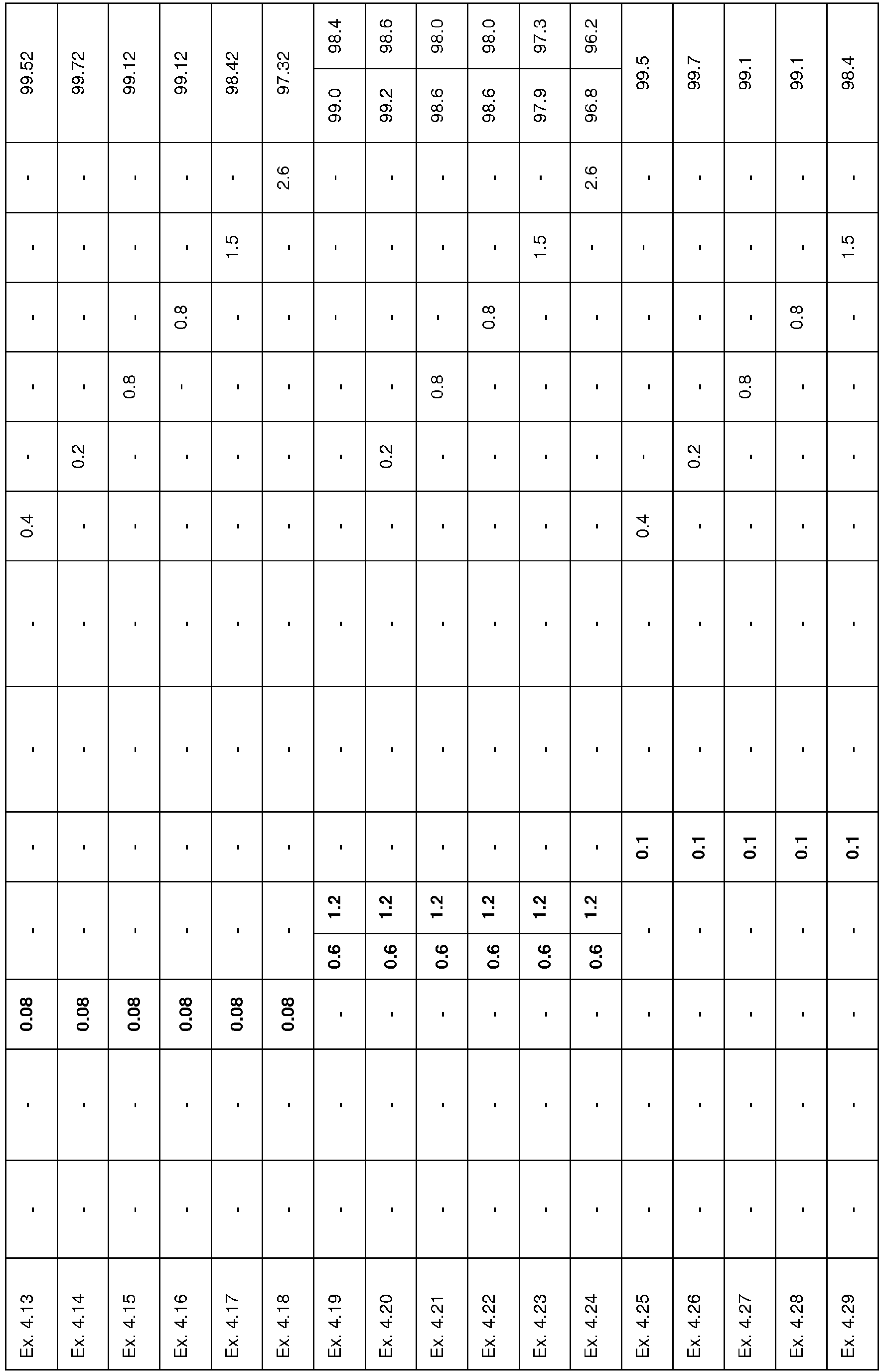

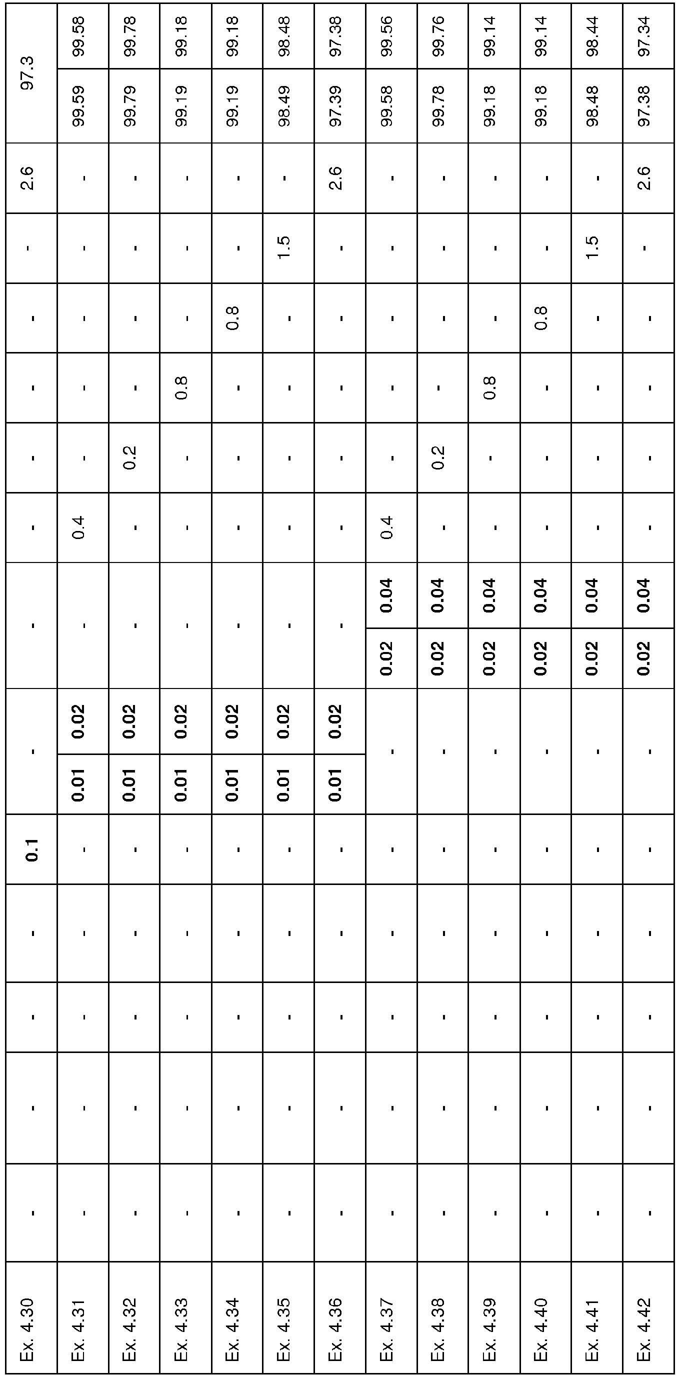

### Examples - 5:

**Table 5.1**

| **ingredients** | **Amount % (w/w)** |
|---|---|
| drugs having amine | 0.01 - 10.0 |
| Corticosteroids | 0.01 - 10.0 |
| fine mannitol | 2.0 - 20.0 |
| coarse mannitol | 20.0 - 98.0 |

### Particle size of;

| | | | |
|---|---|---|---|
| Corticosteroids(µm) | : d₁₀ : 0.1 - 1.0 | d₅₀: 1.0 - 2.5 | d₉₀: 2.5 - 5.0 |
| the drugs having amine (µm) | : d₁₀ : 0.10 - 1.0 | d₅₀: 1.0 - 2.5 | d₉₀: 2.5 - 5.0 |
| fine mannitol (µm) | : d₁₀ : 1.0 - 4.0 | d₅₀: 4.0 - 7.0 | d₉₀: 7.0 - 15.0 |
| coarse mannitol (µm) | : d₁₀ : 10 - 50 | d₅₀: 50 -75 | d₉₀: 75 - 250 |

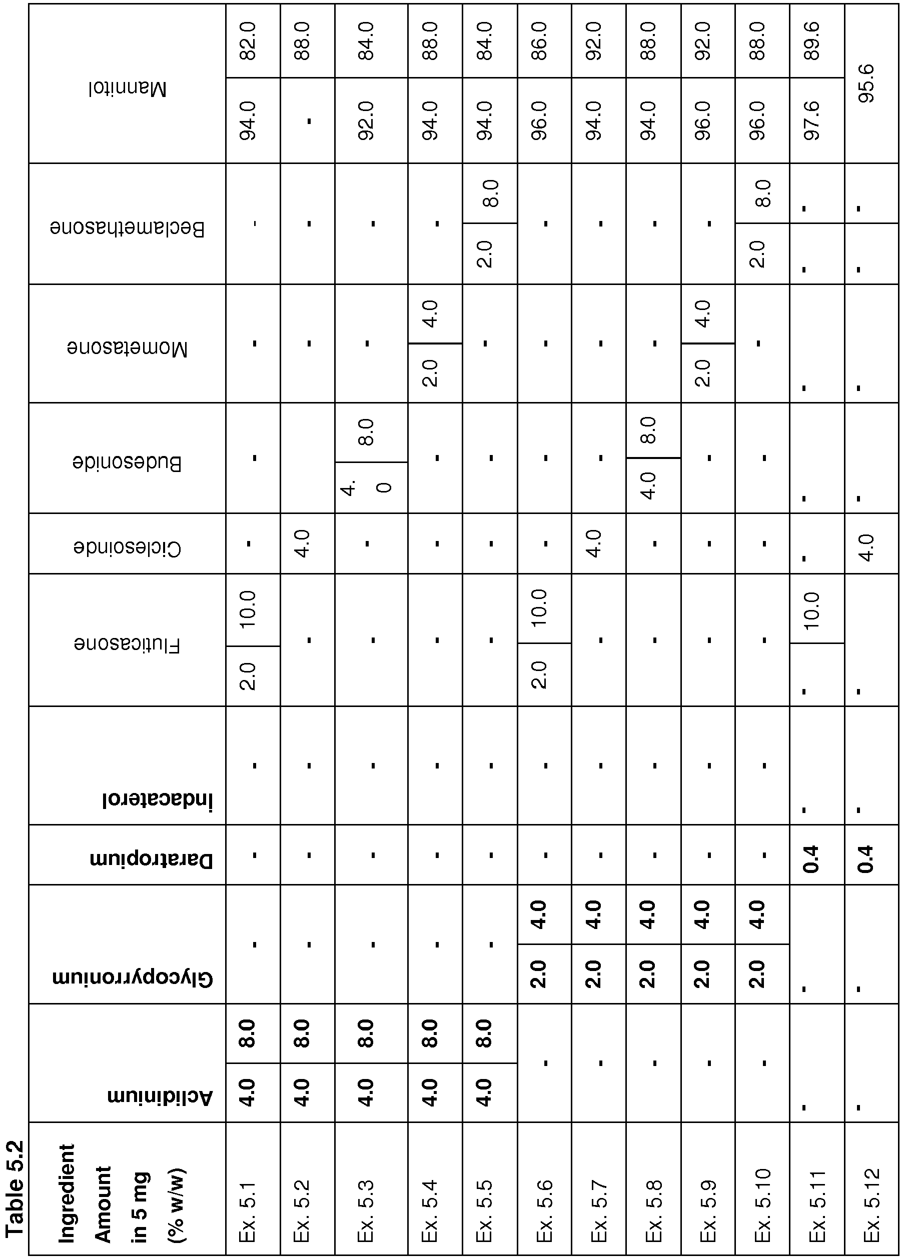

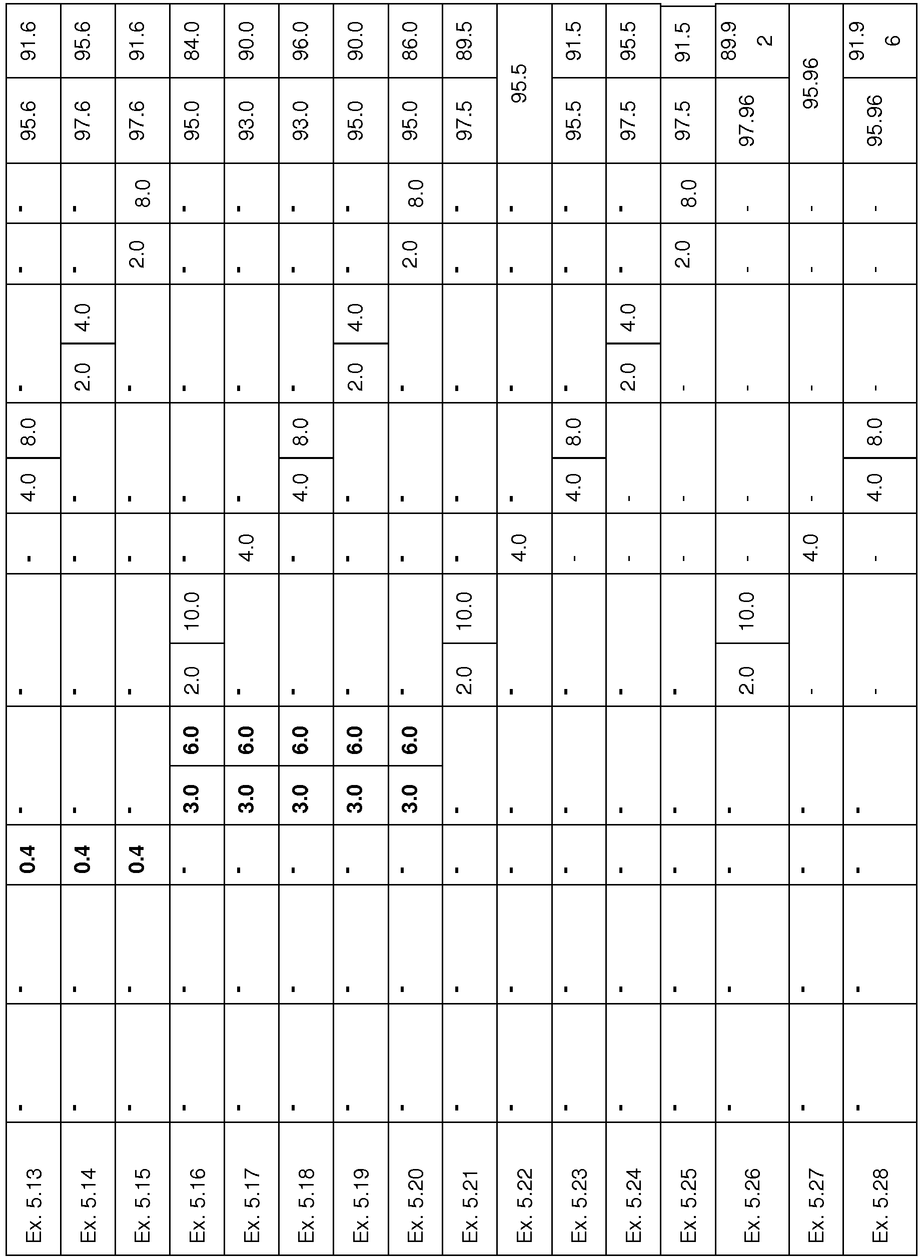

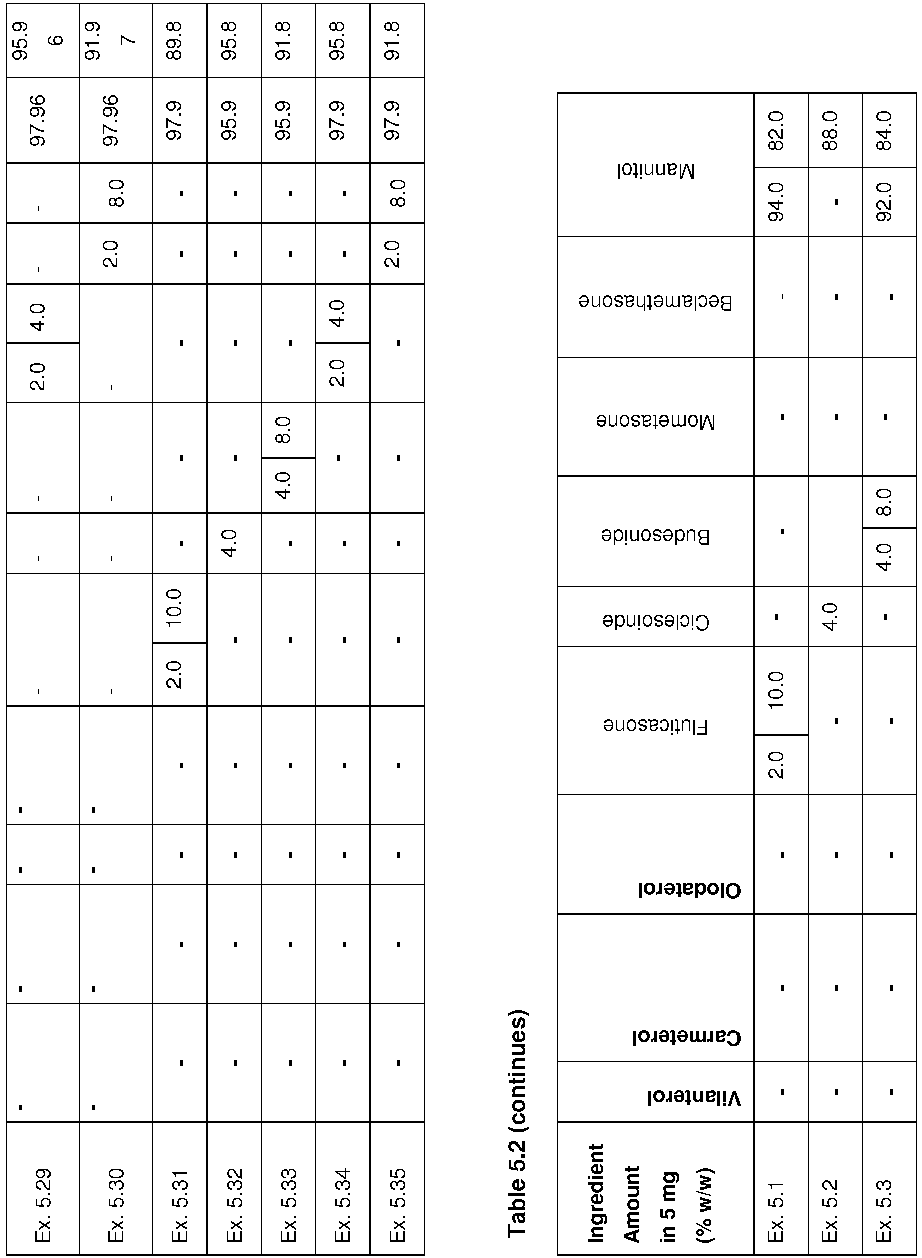

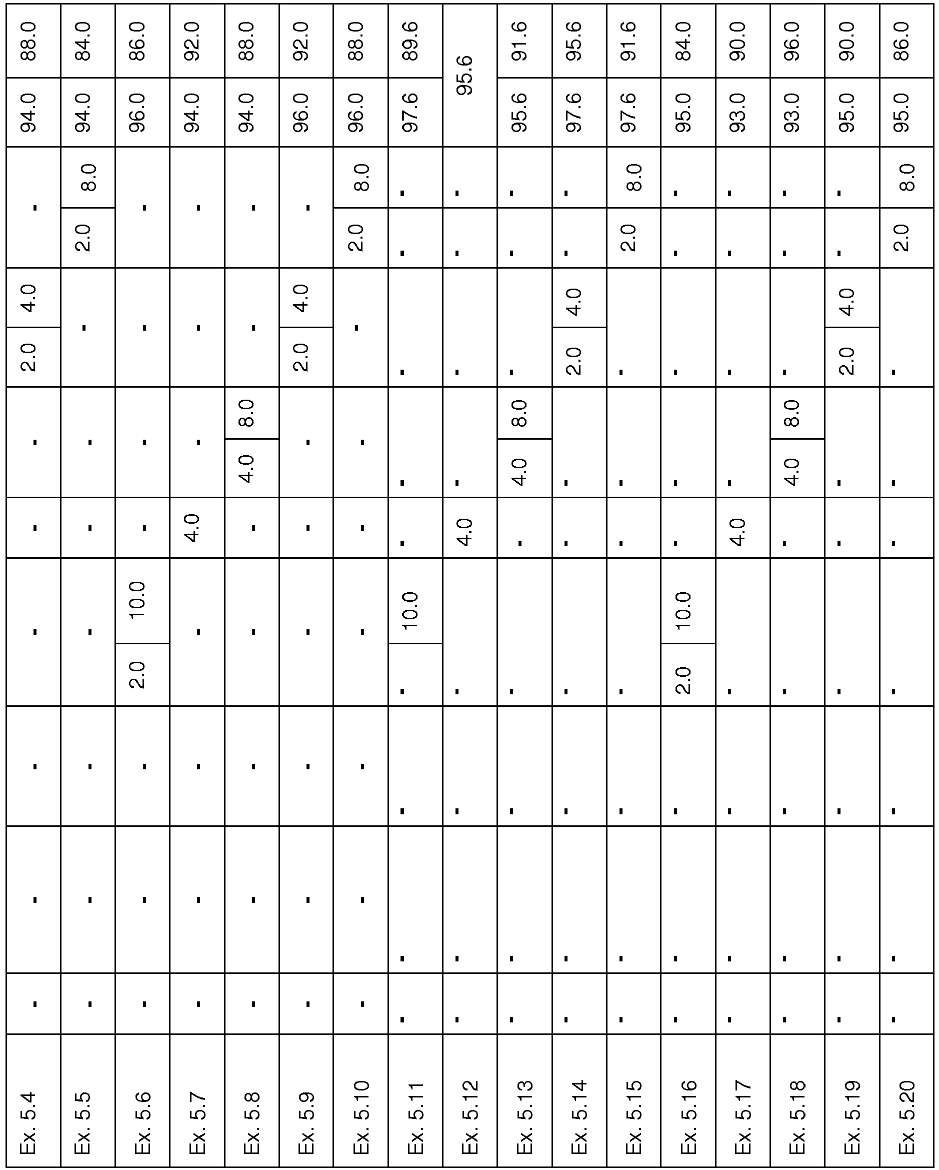

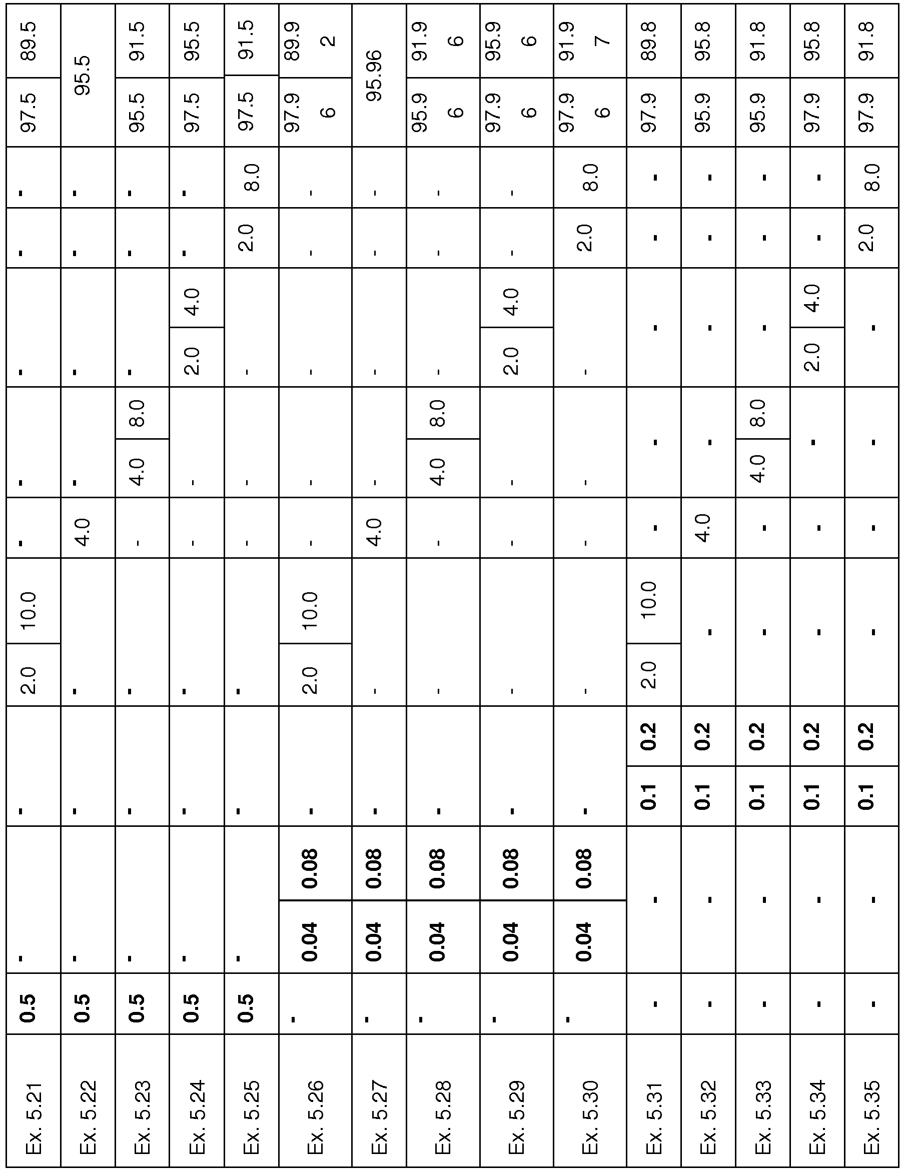

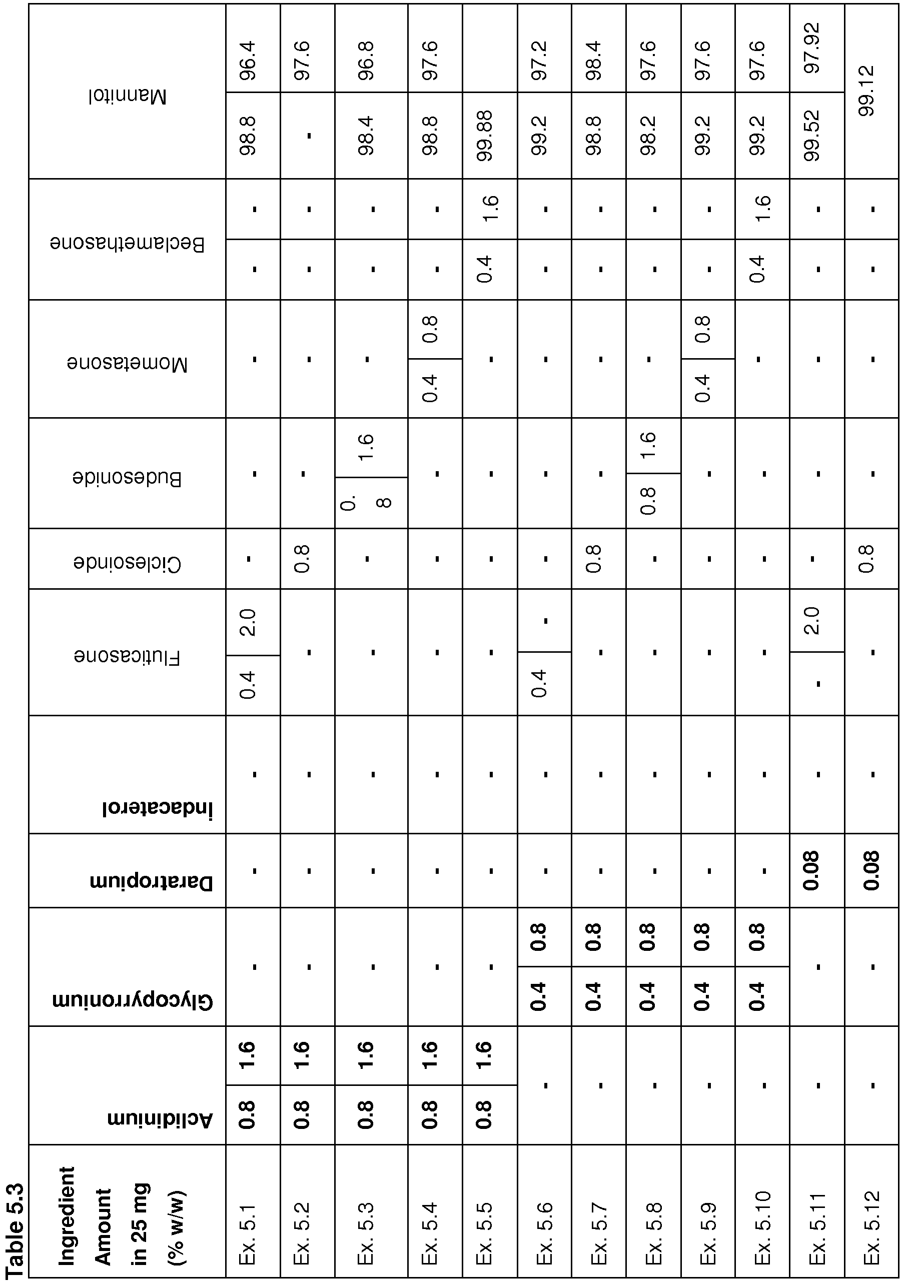

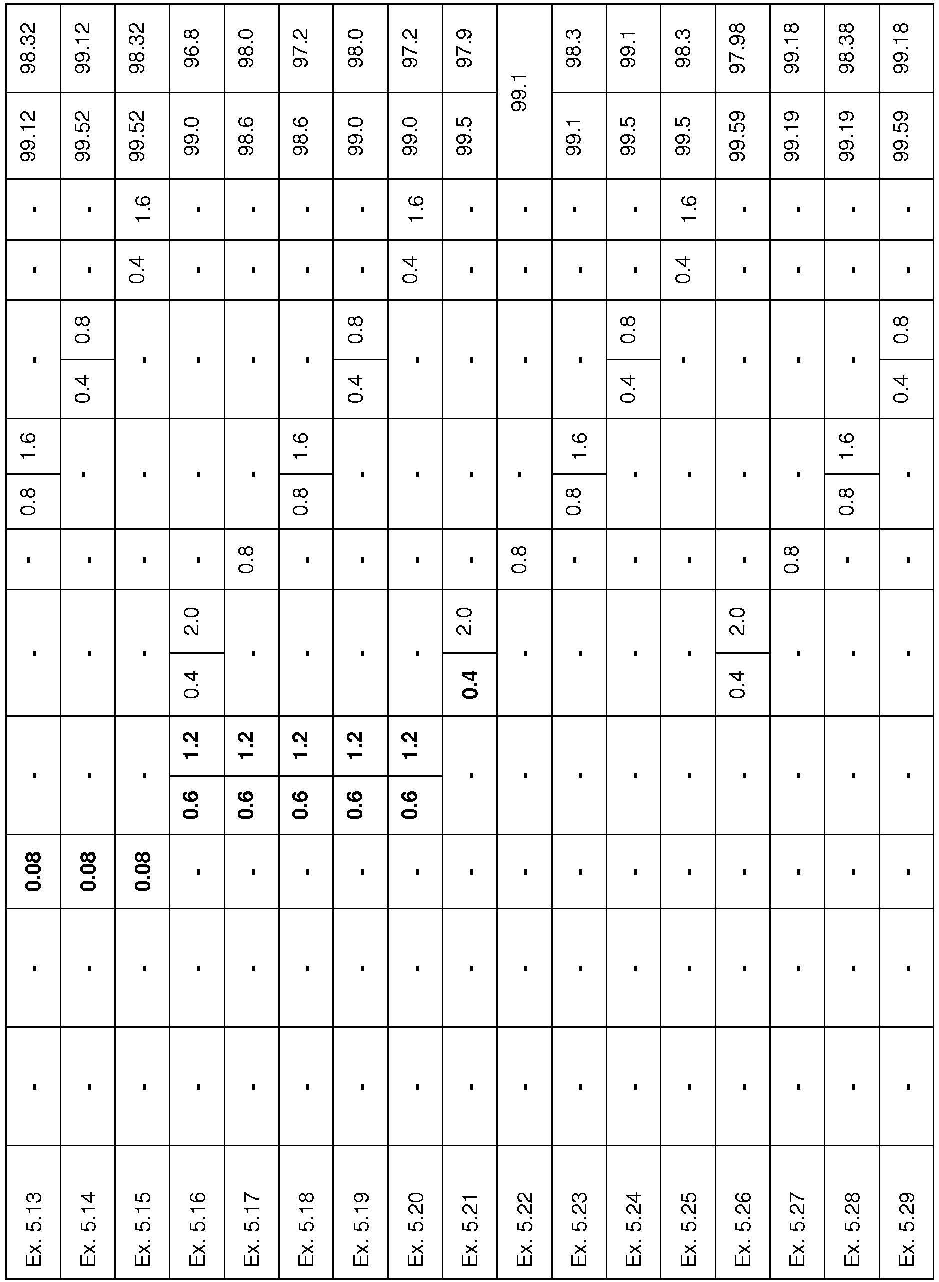

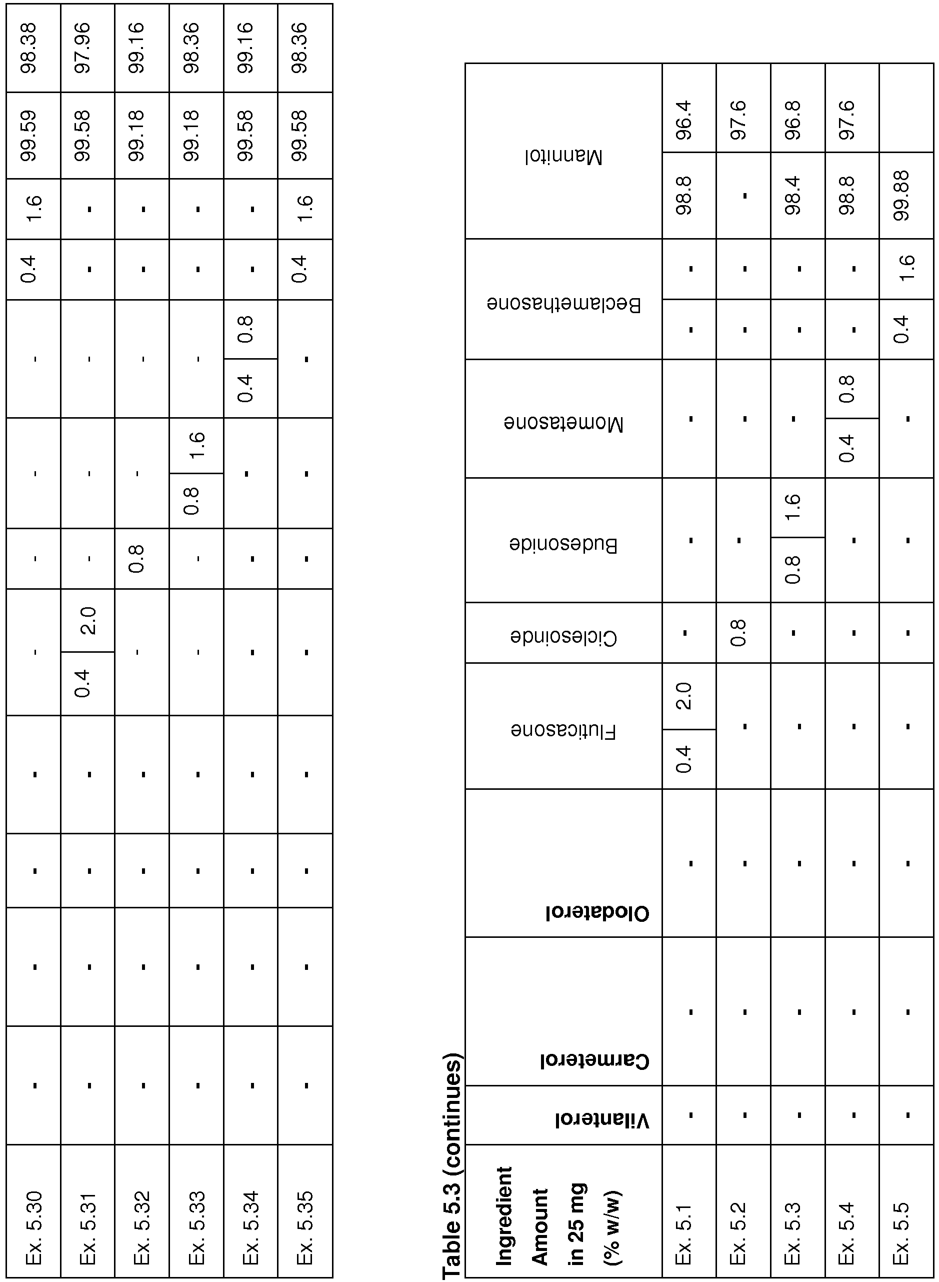

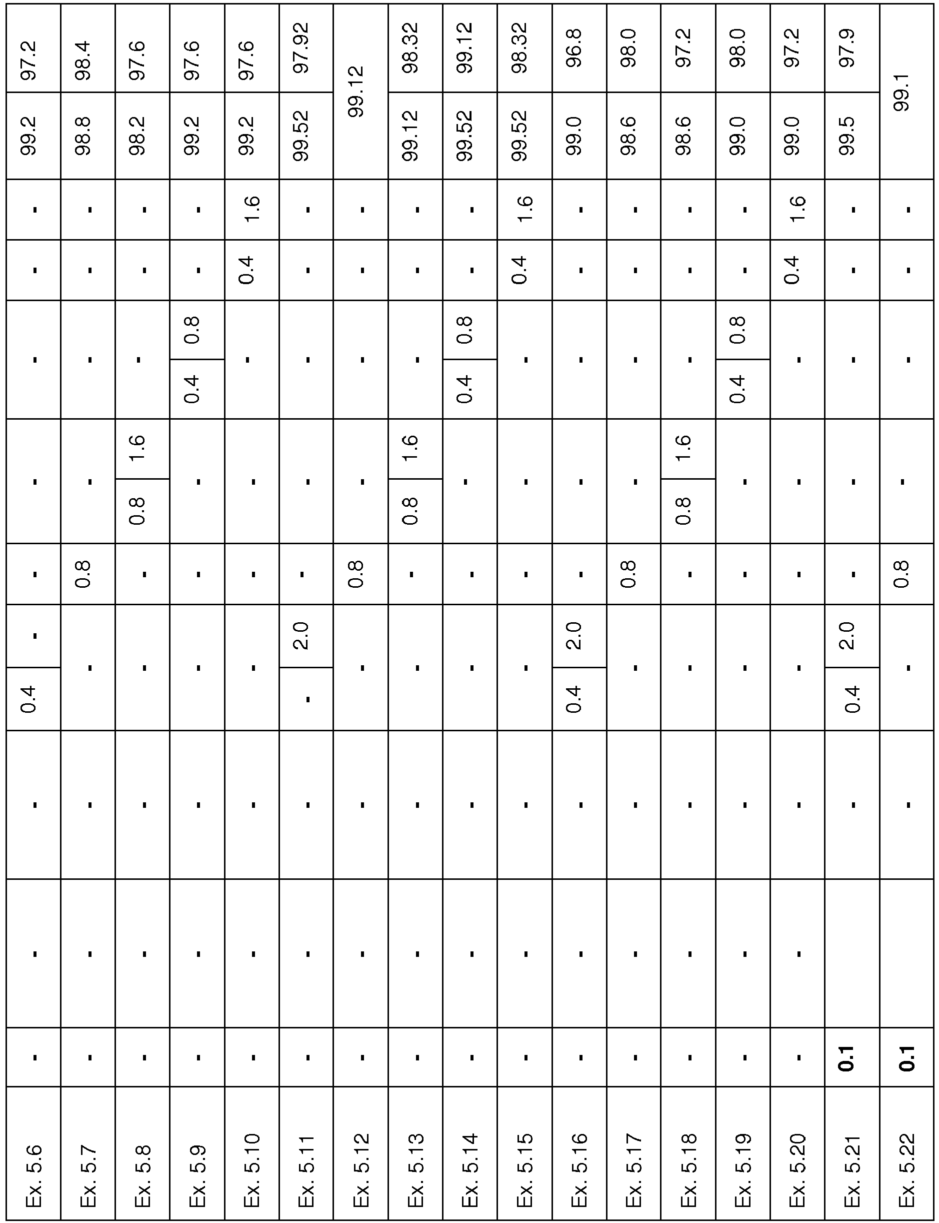

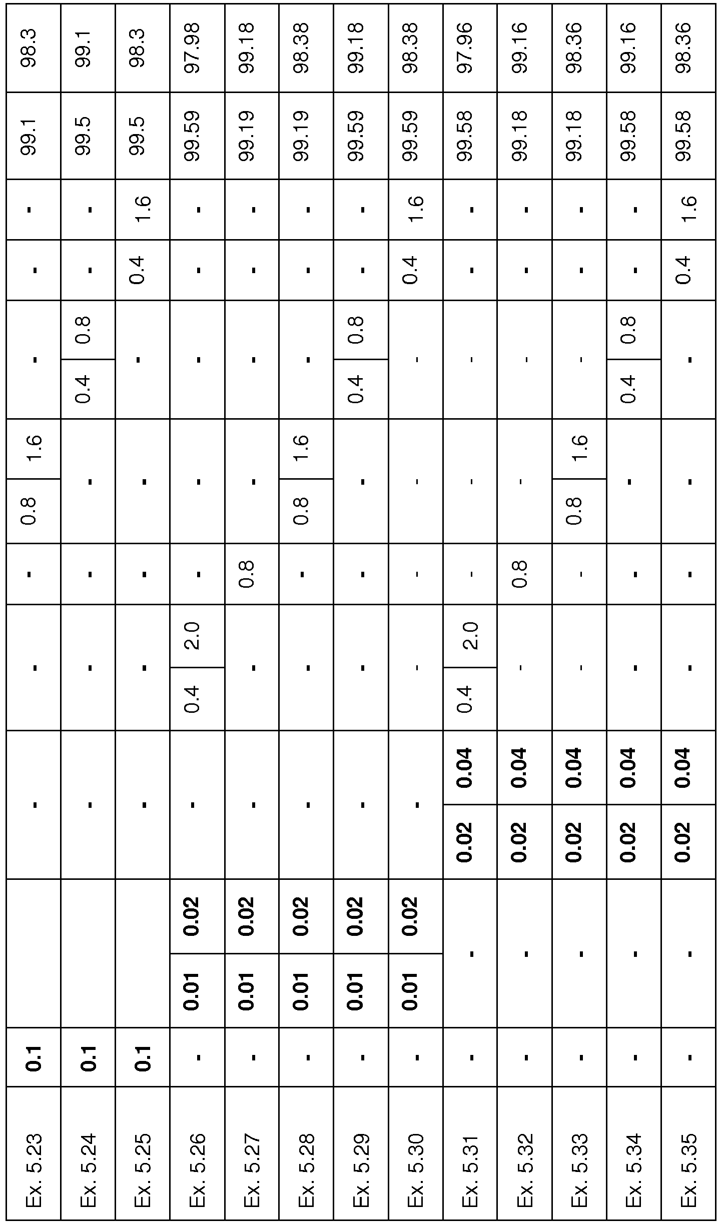

### Examples - 6:

Particle size of each actives and mannitols are the same as above examples 1 to 5.

Amounts of the each actives are same as in 5mg and 25mg of the formulations given in examples 1 to 5.

**Table 6.1**

| **ingredients** | **Amount % (w/w)** |
|---|---|
| drugs having amine | 0.01 - 10.0 |
| LAMAs | 0.01 - 10.0 |
| LABAs | 0.01 - 10.0 |
| fine mannitol | 2.0 - 20.0 |
| coarse mannitol | 20.0 - 98.0 |

**Table 6.2**

| **ingredients** | **Amount % (w/w)** |
|---|---|
| drugs having amine | 0.01 - 10.0 |
| LAMAs | 0.01 - 10.0 |
| SABAs | 0.01 - 10.0 |
| fine mannitol | 2.0 - 20.0 |
| coarse mannitol | 20.0 - 98.0 |

**Table 6.3**

| **ingredients** | **Amount % (w/w)** |
|---|---|
| drugs having amine | 0.01 - 10.0 |
| LAMAs | 0.01 - 10.0 |
| corticosteroids | 0.01 - 10.0 |
| fine mannitol | 2.0 - 20.0 |
| coarse mannitol | 20.0 - 98.0 |

**Table 6.4**

| **ingredients** | **Amount % (w/w)** |
|---|---|
| drugs having amine | 0.01 - 10.0 |
| LABAs | 0.01 - 10.0 |
| SABAs | 0.01 - 10.0 |
| fine mannitol | 2.0 - 20.0 |
| coarse mannitol | 20.0 - 98.0 |

**Table 6.5**

| **ingredients** | **Amount % (w/w)** |
|---|---|
| drugs having amine | 0.01 - 10.0 |
| LABAs | 0.01 - 10.0 |
| corticosteroids | 0.01 - 10.0 |
| fine mannitol | 2.0 - 20.0 |
| coarse mannitol | 20.0 - 98.0 |

**Table 6.6**

| **ingredients** | **Amount % (w/w)** |
|---|---|
| drugs having amine | 0.01 - 10.0 |
| SABAs | 0.01 - 10.0 |
| corticosteroids | 0.01 - 10.0 |
| fine mannitol | 2.0 - 20.0 |
| coarse mannitol | 20.0 - 98.0 |

## Claims

1. A pharmaceutical composition for inhalation administered separately, sequentially or together, comprising drugs having amine in the form of a dry powder which are selected from the group consisting of aclidinium, glycopyrronium, daratropium, indacaterol, vilanterol, carmoterol and olodaterol or their pharmaceutically acceptable salt or ester, or in enantiomerically pure form or as a racemic mixture in admixture with a pharmaceutically acceptable carrier, other than lactose, said pharmaceutically acceptable carrier comprising fine and coarse particles wherein pharmaceutically acceptable carrier is selected from the group consisting of mannitol, glucose, trehalose, cellobiose, sorbitol and maltitol or a combination of two or more of them, and the ratio between the fine particles to the coarse particles is between 0.01 - 0.25 by weight.

2. The pharmaceutical composition according to claim 1, wherein the fine particles of the said pharmaceutically acceptable carrier have a particle diameter of d₁₀ between 1.0 - 4.0 µm, d₅₀ between 4.0 - 7.0 µm and d₉₀ between 7.0 - 15.0 µm.

3. The pharmaceutical composition according to claim 1, wherein the coarse particles of the said pharmaceutically acceptable carrier have a particle diameter of d₁₀ between 10 - 50 µm, d₅₀ between 50 - 75 µm and d₉₀ between 75 - 250 µm.

4. The pharmaceutical composition according to claim 1 to 3, wherein the pharmaceutically acceptable carrier is mannitol.

5. The pharmaceutical composition according to claim 4, wherein the pharmaceutically acceptable carrier is spray dried mannitol.

6. The pharmaceutical composition according to claim 1, wherein the average particle diameter (d₅₀) of the drugs having amine is between 1.5 - 2.5 µm.

7. The pharmaceutical composition according to claim 1, further comprising one or more additional active agents selected from long acting muscarinic antagonists, long acting beta agonists, short acting beta-2 agonists, corticosteroids or a combination of two or more of them in admixture with a pharmaceutically acceptable carrier, other than lactose, as defined in claim 1.

8. The pharmaceutical composition according to claim 7, wherein the long acting muscarinic antagonists are selected from the group comprising tiotropium, glycopyrronium, ipratropium, aclidinium, oxitropium or a pharmaceutically acceptable salt or ester thereof, or in enantiomerically pure form or as a racemic mixture or a combination of two or more of them.

9. The pharmaceutical composition according to claim 7, wherein the long acting beta agonists are selected from the group comprising salmeterol, formoterol, arformoterol, indacaterol, olodaterol, vilanterol, carmoterol, bambuterol or a pharmaceutically acceptable salt or ester thereof, or in enantiomerically pure form or as a racemic mixture or a combination of two or more of them.

10. The pharmaceutical composition according to claim 7, wherein the short acting beta-2 agonists are selected from the group comprising salbutamol, levosalbutamol, terbutaline, pirbuterol, procaterol, fenoterol, bitolterol, ritodrine, metaproterenol or a pharmaceutically acceptable salt or ester thereof, or in enantiomerically pure form or as a racemic mixture or a combination of two or more of them.

11. The pharmaceutical composition according to claim 7, wherein the corticosteroids are selected from the group comprising fluticasone, ciclesonide, budesonide, mometasone, beclomethasone, triamcinolone, flunisolide, dexamethasone or a pharmaceutically acceptable salt or ester thereof, or in enantiomerically pure form or as a racemic mixture or a combination of two or more of them.

12. The pharmaceutical composition according to claim 8, comprising any of the following combinations, said combinations being suitable for administration separately, sequentially or together in effective amounts;
i. Aclidinium and tiotropium
ii. Aclidinium and glycopyrronium
iii. Aclidinum and ipratropium
iv. Aclidinum and oxitropium
v. Glycopyrronium and tiotropium
vi. Glycopyrronium and ipratropium
vii. Glycopyrronium and oxitropium
viii. Darotropium and tiotropium
ix. Darotropium and glycopyrronium
x. Darotropium and ipratropium
xi. Darotropium and aclidinium
xii. Darotropium and oxitropium
xiii. Indacaterol and tiotropium
xiv. Indacaterol and glycopyrronium
xv. Indacaterol and ipratropium
xvi. Indacaterol and aclidinium
xvii. Indacaterol and oxitropium
xviii. Vilanterol and tiotropium
xix. Vilanterol and glycopyrronium
xx. Vilanterol and ipratropium
xxi. Vilanterol and aclidinium
xxii. Vilanterol and oxitropium
xxiii. Carmoterol and tiotropium
xxiv. Carmoterol and glycopyrronium
xxv. Carmoterol and ipratropium
xxvi. Carmoterol and aclidinium
xxvii. Carmoterol and oxitropium
xxviii. Olodaterol and tiotropium
xxix. Olodaterol and ipratropium
xxx. Olodaterol and glycopyrronium
xxxi. Olodaterol and aclidinium
xxxii. Olodaterol and oxitropium
wherein said therapeutic agents can be present as a pharmaceutically acceptable salt or ester thereof, or in enantiomerically pure form or as a racemic mixture.

13. The pharmaceutical composition according to claim 9, comprising any of the following combinations, said combinations being suitable for administration separately, sequentially or together in effective amounts;
i. Aclidinium and salmeterol
ii. Aclidinum and formoterol
iii. Aclidinium and arformoterol
iv. Aclidinium and indacaterol
v. Aclidinium and olodaterol
vi. Aclidinium and vilanterol
vii. Aclidinium and carmoterol
viii. Aclidinium and bambuterol
ix. Glycopyrronium and salmeterol
x. Glycopyrronium and formoterol
xi. Glycopyrronium and arformoterol
xii. Glycopyrronium and indacaterol
xiii. Glycopyrronium and olodaterol
xiv. Glycopyrronium and vilanterol
xv. Glycopyrronium and carmoterol
xvi. Glycopyrronium and bambuterol
xvii. Darotropium and salmeterol
xviii. Darotropium and formoterol
xix. Darotropium and arformoterol
xx. Darotropium and indacaterol
xxi. Darotropium and olodaterol
xxii. Darotropium and vilanterol
xxiii. Darotropium and carmoterol
xxiv. Darotropium and bambuterol
xxv. Indacaterol and salmeterol
xxvi. Indacaterol and formoterol
xxvii. Indacaterol and arformoterol
xxviii. Indacaterol and olodaterol
xxix. Indacaterol and vilanterol
xxx. Indacaterol and carmoterol
xxxi. Indacaterol and bambuterol
xxxii. Vilanterol and salmeterol
xxxiii. Vilanterol and formoterol
xxxiv. Vilanterol and arformoterol
xxxv. Vilanterol and olodaterol
xxxvi. Vilanterol and carmoterol
xxxvii. Vilanterol and bambuterol
xxxviii. Carmoterol and salmeterol
xxxix. Carmoterol and formoterol
xl. Carmoterol and arformoterol
xli. Carmoterol and olodaterol
xlii. Carmoterol and bambuterol
xliii. Olodaterol and salmeterol
xliv. Olodaterol and formoterol
xlv. Olodaterol and arformoterol
xlvi. Olodaterol and bambuterol
wherein said therapeutic agents can be present as a pharmaceutically acceptable salt or ester thereof, or in enantiomerically pure form or as a racemic mixture.

14. The pharmaceutical composition according to claim 10, comprising any of the following combinations, said combinations being suitable for administration separately, sequentially or together in effective amounts;
i. Aclidinium and salbutamol
ii. Aclidinium and levosalbutamol
iii. Aclidinium and terbutaline
iv. Aclidinium and pirbuterol
v. Aclidinium and procaterol
vi. Aclidinium and fenoterol
vii. Aclidinium and bitolterol
viii. Aclidinium and ritodrine
ix. Aclidinium and metaproterenol
x. Glycopyrronium and salbutamol
xi. Glycopyrronium and levosalbutamol
xii. Glycopyrronium and terbutaline
xiii. Glycopyrronium and pirbuterol
xiv. Glycopyrronium and procaterol
xv. Glycopyrronium and fenoterol
xvi. Glycopyrronium and bitolterol
xvii. Glycopyrronium and ritodrine
xviii. Glycopyrronium and metaproterenol
xix. Darotropium and salbutamol
xx. Darotropium and levosalbutamol
xxi. Darotropium and terbutaline
xxii. Darotropium and pirbuterol
xxiii. Darotropium and procaterol
xxiv. Darotropium and fenoterol
xxv. Darotropium and bitolterol
xxvi. Darotropium and ritodrine
xxvii. Darotropium and metaproterenol
xxviii. Indacaterol and salbutamol
xxix. Indacaterol and levosalbutamol
xxx. Indacaterol and terbutaline
xxxi. Indacaterol and pirbuterol
xxxii. Indacaterol and procaterol
xxxiii. Indacaterol and fenoterol
xxxiv. Indacaterol and bitolterol
xxxv. Indacaterol and ritodrine
xxxvi. Indacaterol and metaproterenol
xxxvii. Vilanterol and salbutamol
xxxviii. Vilanterol and levosalbutamol
xxxix. Vilanterol and terbutaline
xl. Vilanterol and pirbuterol
xli. Vilanterol and procaterol
xlii. Vilanterol and fenoterol
xliii. Vilanterol and bitolterol
xliv. Vilanterol and ritodrine
xlv. Vilanterol and metaproterenol
xlvi. Carmoterol and salbutamol
xlvii. carmoterol and levosalbutamol
xlviii. carmoterol and terbutaline
xlix. carmoterol and pirbuterol
l. carmoterol and procaterol
li. carmoterol and fenoterol
lii. carmoterol and bitolterol
liii. carmoterol and ritodrine
liv. carmoterol and metaproterenol
lv. olodaterol and salbutamol
lvi. olodaterol and levosalbutamol
lvii. olodaterol and terbutaline
lviii. olodaterol and pirbuterol
lix. olodaterol and procaterol
lx. olodaterol and fenoterol
lxi. olodaterol and bitolterol
lxii. olodaterol and ritodrine
lxiii. olodaterol and metaproterenol
wherein said therapeutic agents can be present as a pharmaceutically acceptable salt or ester thereof, or in enantiomerically pure form or as a racemic mixture.

15. The pharmaceutical composition according to claim 11, comprising any of the following combinations, said combinations being suitable for administration separately, sequentially or together in effective amounts;
i. Aclidinium and fluticasone
ii. Aclidinium and ciclesonide
iii. Aclidinium and budesonide
iv. Aclidinium and mometasone
v. Aclidinium and beclomethasone
vi. Aclidinium and triamcinolone
vii. Aclidinium and flunisolide
viii. Aclidinium and dexamethasone
ix. Glycopyrronium and fluticasone
x. Glycopyrronium and ciclesonide
xi. Glycopyrronium and budesonide
xii. Glycopyrronium and mometasone
xiii. Glycopyrronium and beclomethasone
xiv. Glycopyrronium and triamcinolone
xv. Glycopyrronium and flunisolide
xvi. Glycopyrronium and dexamethasone
xvii. Darotropium and fluticasone
xviii. Darotropium and ciclesonide
xix. Darotropium and budesonide
xx. Darotropium and mometasone
xxi. Darotropium and beclomethasone
xxii. Darotropium and triamcinolone
xxiii. Darotropium and flunisolide
xxiv. Darotropium and dexamethasone
xxv. Indacaterol and fluticasone
xxvi. Indacaterol and ciclesonide
xxvii. Indacaterol and budesonide
xxviii. Indacaterol and mometasone
xxix. Indacaterol and beclomethasone
xxx. Indacaterol and triamcinolone
xxxi. Indacaterol and flunisolide
xxxii. Indacaterol and dexamethasone
xxxiii. Vilanterol and fluticasone
xxxiv. Vilanterol and ciclesonide
xxxv. Vilanterol and budesonide
xxxvi. Vilanterol and mometasone
xxxvii. Vilanterol and beclomethasone
xxxviii. Vilanterol and triamcinolone
xxxix. Vilanterol and flunisolide
xl. Vilanterol and dexamethasone
xli. Carmoterol and fluticasone
xlii. Carmoterol and ciclesonide
xliii. Carmoterol and budesonide
xliv. Carmoterol and mometasone
xlv. Carmoterol and beclomethasone
xlvi. Carmoterol and triamcinolone
xlvii. Carmoterol and flunisolide
xlviii. Carmoterol and dexamethasone
xlix. Olodaterol and fluticasone
l. Olodaterol and ciclesonide
li. Olodaterol and budesonide
lii. Olodaterol and mometasone
liii. Olodaterol and beclamethasone
liv. Olodaterol and triamcinolone
lv. Olodaterol and flunisolide
lvi. Olodaterol and dexamethasone
wherein said therapeutic agents can be present as a pharmaceutically acceptable salt or ester thereof, or in enantiomerically pure form or as a racemic mixture.

16. The pharmaceutical composition according to claims 12, comprising any of the following combinations, said combinations being suitable for administration separately, sequentially or together in effective amounts;
i. Aclidinum, tiotropium and salmeterol
ii. Aclidinum, tiotropium and formoterol
iii. Aclidinum, tiotropium and arformoterol
iv. Aclidinum, tiotropium and indacaterol
v. Aclidinum, tiotropium and olodaterol
vi. Aclidinum, tiotropium and vilanterol
vii. Aclidinum, tiotropium and carmoterol
viii. Aclidinum, tiotropium and bambuterol
ix. Aclidinum, glycopyrronium and salmeterol
x. Aclidinum, glycopyrronium and formoterol
xi. Aclidinum, glycopyrronium and arformoterol
xii. Aclidinum, glycopyrronium and indacaterol
xiii. Aclidinum, glycopyrronium and olodaterol
xiv. Aclidinum, glycopyrronium and vilanterol
xv. Aclidinum, glycopyrronium and carmoterol
xvi. Aclidinum, glycopyrronium and bambuterol
xvii. Aclidinum, oxitropium and salmeterol
xviii. Aclidinum, oxitropium and formoterol
xix. Aclidinum, oxitropium and arformoterol
xx. Aclidinum, oxitropium and indacaterol
xxi. Aclidinum, oxitropium and olodaterol
xxii. Aclidinum, oxitropium and vilanterol
xxiii. Aclidinum, oxitropium and carmoterol
xxiv. Aclidinum, oxitropium and bambuterol
xxv. Glycopyrronium, tiotropium and salmeterol
xxvi. Glycopyrronium, tiotropium and formoterol
xxvii. Glycopyrronium, tiotropium and arformoterol
xxviii. Glycopyrronium, tiotropium and indacaterol
xxix. Glycopyrronium, tiotropium and olodaterol
xxx. Glycopyrronium, tiotropium and vilanterol
xxxi. Glycopyrronium, tiotropium and carmoterol
xxxii. Glycopyrronium, tiotropium and bambuterol
xxxiii. Glycopyrronium, oxitropium and salmeterol
xxxiv. Glycopyrronium, oxitropium and formoterol
xxxv. Glycopyrronium, oxitropium and arformoterol
xxxvi. Glycopyrronium, oxitropium and indacaterol
xxxvii. Glycopyrronium, oxitropium and olodaterol
xxxviii. Glycopyrronium, oxitropium and vilanterol
xxxix. Glycopyrronium, oxitropium and carmoterol
xl. Glycopyrronium, oxitropium and bambuterol
xli. Daratropium, tiotropium and salmeterol
xlii. Daratropium, tiotropium and formoterol
xliii. Daratropium, tiotropium and arformoterol
xliv. Daratropium, tiotropium and indacaterol
xlv. Daratropium, tiotropium and olodaterol
xlvi. Daratropium, tiotropium and vilanterol
xlvii. Daratropium, tiotropium and carmoterol
xlviii. Daratropium, tiotropium and bambuterol
xlix. Daratropium, glycopyrronium and salmeterol
l. Daratropium, glycopyrronium and formoterol
li. Daratropium, glycopyrronium and arformoterol
lii. Daratropium, glycopyrronium and indacaterol
liii. Daratropium, glycopyrronium and olodaterol
liv. Daratropium, glycopyrronium and vilanterol
lv. Daratropium, glycopyrronium and carmoterol
lvi. Daratropium, glycopyrronium and bambuterol
lvii. Daratropium, aclidinium and salmeterol
lviii. Daratropium, aclidinium and formoterol
lix. Daratropium, aclidinium and arformoterol
lx. Daratropium, aclidinium and indacaterol
lxi. Daratropium, aclidinium and olodaterol
lxii. Daratropium, aclidinium and vilanterol
lxiii. Daratropium, aclidinium and carmoterol
lxiv. Daratropium, aclidinium and bambuterol
lxv. Daratropium, oxitropium and salmeterol
lxvi. Daratropium, oxitropium and formoterol
lxvii. Daratropium, oxitropium and arformoterol
lxviii. Daratropium, oxitropium and indacaterol
lxix. Daratropium, oxitropium and olodaterol
lxx. Daratropium, oxitropium and vilanterol
lxxi. Daratropium, oxitropium and carmoterol
lxxii. Daratropium, oxitropium and bambuterol
lxxiii. Indacaterol, tiotropium and salmeterol
lxxiv. Indacaterol, tiotropium and formoterol
lxxv. Indacaterol, tiotropium and arformoterol
lxxvi. Indacaterol, tiotropium and olodaterol
lxxvii. Indacaterol, tiotropium and vilanterol
lxxviii. Indacaterol, tiotropium and carmoterol
lxxix. Indacaterol, tiotropium and bambuterol
lxxx. Indacaterol, glycopyrronium and salmeterol
lxxxi. Indacaterol, glycopyrronium and formoterol
lxxxii. Indacaterol, glycopyrronium and arformoterol
lxxxiii. Indacaterol, glycopyrronium and olodaterol
lxxxiv. Indacaterol, glycopyrronium and vilanterol
lxxxv. Indacaterol, glycopyrronium and carmoterol
lxxxvi. Indacaterol, glycopyrronium and bambuterol
lxxxvii. Indacaterol, aclidinium and salmeterol
lxxxviii. Indacaterol, aclidinium and formoterol
lxxxix. Indacaterol, aclidinium and arformoterol
xc. Indacaterol, aclidinium and olodaterol
xci. Indacaterol, aclidinium and vilanterol
xcii. Indacaterol, aclidinium and carmoterol
xciii. Indacaterol, aclidinium and bambuterol
xciv. Indacaterol, oxitropium and salmeterol
xcv. Indacaterol, oxitropium and formoterol
xcvi. Indacaterol, oxitropium and arformoterol
xcvii. Indacaterol, oxitropium and olodaterol
xcviii. Indacaterol, oxitropium and vilanterol
xcix. Indacaterol, oxitropium and carmoterol
c. Indacaterol, oxitropium and bambuterol
ci. Vilanterol, tiotropium and salmeterol
cii. Vilanterol, tiotropium and formoterol
ciii. Vilanterol, tiotropium and arformoterol
civ. Vilanterol, tiotropium and indacaterol
cv. Vilanterol, tiotropium and olodaterol
cvi. Vilanterol, tiotropium and carmoterol
cvii. Vilanterol, tiotropium and bambuterol
cviii. Vilanterol, glycopyrronium and salmeterol
cix. Vilanterol, glycopyrronium and formoterol
cx. Vilanterol, glycopyrronium and arformoterol
cxi. Vilanterol, glycopyrronium and indacaterol
cxii. Vilanterol, glycopyrronium and olodaterol
cxiii. Vilanterol, glycopyrronium and carmoterol
cxiv. Vilanterol, glycopyrronium and bambuterol
cxv. Vilanterol, aclidinium and salmeterol
cxvi. Vilanterol, aclidinium and formoterol
cxvii. Vilanterol, aclidinium and arformoterol
cxviii. Vilanterol, aclidinium and indacaterol
cxix. Vilanterol, aclidinium and olodaterol
cxx. Vilanterol, aclidinium and carmoterol
cxxi. Vilanterol, aclidinium and bambuterol
cxxii. Vilanterol, oxitropium and salmeterol
cxxiii. Vilanterol, oxitropium and formoterol
cxxiv. Vilanterol, oxitropium and arformoterol
cxxv. Vilanterol, oxitropium and indacaterol
cxxvi. Vilanterol, oxitropium and olodaterol
cxxvii. Vilanterol, oxitropium and carmoterol
cxxviii. Vilanterol, oxitropium and bambuterol
cxxix. Carmoterol, tiotropium and salmeterol
cxxx. Carmoterol, tiotropium and formoterol
cxxxi. Carmoterol, tiotropium and arformoterol
cxxxii. Carmoterol, tiotropium and indacaterol
cxxxiii. Carmoterol, tiotropium and olodaterol
cxxxiv. Carmoterol, tiotropium and vilanterol
cxxxv. Carmoterol, tiotropium and bambuterol
cxxxvi. Carmoterol, glycopyrronium and salmeterol
cxxxvii.Carmoterol, glycopyrronium and formoterol
cxxxviii. Carmoterol, glycopyrronium and arformoterol
cxxxix. Carmoterol, glycopyrronium and indacaterol
cxl. Carmoterol, glycopyrronium and olodaterol
cxli. Carmoterol, glycopyrronium and vilanterol
cxlii. Carmoterol, glycopyrronium and bambuterol
cxliii. Carmoterol, aclidinium and salmeterol
cxliv. Carmoterol, aclidinium and formoterol
cxlv. Carmoterol, aclidinium and arformoterol
cxlvi. Carmoterol, aclidinium and indacaterol
cxlvii. Carmoterol, aclidinium and olodaterol
cxlviii. Carmoterol, aclidinium and vilanterol
cxlix. Carmoterol, aclidinium and bambuterol
cl. Carmoterol, oxitropium and salmeterol
cli. Carmoterol, oxitropium and formoterol
clii. Carmoterol, oxitropium and arformoterol
cliii. Carmoterol, oxitropium and indacaterol
cliv. Carmoterol, oxitropium and olodaterol
clv. Carmoterol, oxitropium and vilanterol
clvi. Carmoterol, oxitropium and bambuterol
clvii. Olodaterol, tiotropium and salmeterol
clviii. Olodaterol, tiotropium and formoterol
clix. Olodaterol, tiotropium and arformoterol
clx. Olodaterol, tiotropium and indacaterol
clxi. Olodaterol, tiotropium and vilanterol
clxii. Olodaterol, tiotropium and bambuterol
clxiii. Olodaterol, glycopyrronium and salmeterol
clxiv. Olodaterol, glycopyrronium and formoterol
clxv. Olodaterol, glycopyrronium and arformoterol
clxvi. Olodaterol, glycopyrronium and indacaterol
clxvii. Olodaterol, glycopyrronium and vilanterol
clxviii. Olodaterol, glycopyrronium and bambuterol
clxix. Olodaterol, aclidinium and salmeterol
clxx. Olodaterol, aclidinium and formoterol
clxxi. Olodaterol, aclidinium and arformoterol
clxxii. Olodaterol, aclidinium and indacaterol
clxxiii. Olodaterol, aclidinium and vilanterol
clxxiv. Olodaterol, aclidinium and bambuterol
clxxv. Olodaterol, oxitropium and salmeterol
clxxvi. Olodaterol, oxitropium and formoterol
clxxvii. Olodaterol, oxitropium and arformoterol
clxxviii.Olodaterol, oxitropium and indacaterol
clxxix. Olodaterol, oxitropium and vilanterol
clxxx. Olodaterol, oxitropium and bambuterol
wherein said therapeutic agents can be present as a pharmaceutically acceptable salt or ester thereof, or in enantiomerically pure form or as a racemic mixture.

17. The pharmaceutical composition according to claims 13, comprising any of the following combinations, said combinations being suitable for administration separately, sequentially or together in effective amounts;
i. Aclidinum, tiotropium and salbutamol
ii. Aclidinum, tiotropium and levosalbutamol
iii. Aclidinum, tiotropium and terbutaline
iv. Aclidinum, tiotropium and pirbutarol
v. Aclidinum, tiotropium and procaterol
vi. Aclidinum, tiotropium and fenoterol
vii. Aclidinum, tiotropium and ritodrine
viii. Aclidinum, tiotropium and bitolterol
ix. Aclidinum, tiotropium and metaproterenol
x. Aclidinum, glycopyrronium and salbutamol
xi. Aclidinum, glycopyrronium and levosalbutamol
xii. Aclidinum, glycopyrronium and terbutaline
xiii. Aclidinum, glycopyrronium and pirbuterol
xiv. Aclidinum, glycopyrronium and procaterol
xv. Aclidinum, glycopyrronium and fenoterol
xvi. Aclidinum, glycopyrronium and bitolterol
xvii. Aclidinum, glycopyrronium and ritodrine
xviii. Aclidinum, glycopyrronium and metaproterenol
xix. Aclidinum, ipratropium and salbutamol
xx. Aclidinum, ipratropium and levosalbutamol
xxi. Aclidinum, ipratropium and terbutaline
xxii. Aclidinum, ipratropium and pirbuterol
xxiii. Aclidinum, ipratropium and procaterol
xxiv. Aclidinum, ipratropium and fenoterol
xxv. Aclidinum, ipratropium and bitolterol
xxvi. Aclidinum, ipratropium and ritodrine
xxvii. Aclidinum, ipratropium and metaproterenol
xxviii. Aclidinum, oxitropium and salbutamol
xxix. Aclidinum, oxitropium and levosalbutamol
xxx. Aclidinum, oxitropium and terbutaline
xxxi. Aclidinum, oxitropium and pirbuterol
xxxii. Aclidinum, oxitropium and procaterol
xxxiii. Aclidinum, oxitropium and fenoterol
xxxiv. Aclidinum, oxitropium and bitolterol
xxxv. Aclidinum, oxitropium and ritodrine
xxxvi. Aclidinum, oxitropium and metaproterenol
xxxvii. Glycopyrronium, tiotropium and salbutamol
xxxviii. Glycopyrronium, tiotropium and levosalbutamol
xxxix. Glycopyrronium, tiotropium and terbutaline
xl. Glycopyrronium, tiotropium and pirbuterol
xli. Glycopyrronium, tiotropium and procaterol
xlii. Glycopyrronium, tiotropium and fenoterol
xliii. Glycopyrronium, tiotropium and bitolterol
xliv. Glycopyrronium, tiotropium and ritodrine
xlv. Glycopyrronium, tiotropium and metaproterenol
xlvi. Glycopyrronium, ipratropium and salbutamol
xlvii. Glycopyrronium, ipratropium and levosalbutamol
xlviii. Glycopyrronium, ipratropium and terbutaline
xlix. Glycopyrronium, ipratropium and pirbuterol
l. Glycopyrronium, ipratropium and procaterol
li. Glycopyrronium, ipratropium and fenoterol
lii. Glycopyrronium, ipratropium and bitolterol
liii. Glycopyrronium, ipratropium and ritodrine
liv. Glycopyrronium, ipratropium and metaproterenol
lv. Glycopyrronium, oxitropium and salbutamol
lvi. Glycopyrronium, oxitropium and levosalbutamol
lvii. Glycopyrronium, oxitropium and terbutaline
lviii. Glycopyrronium, oxitropium and pirbuterol
lix. Glycopyrronium, oxitropium and procaterol
lx. Glycopyrronium, oxitropium and fenoterol
lxi. Glycopyrronium, oxitropium and bitolterol
lxii. Glycopyrronium, oxitropium and ritodrine
lxiii. Glycopyrronium, oxitropium and metaproterenol
lxiv. Daratropium, tiotropium and salbutamol
lxv. Daratropium, tiotropium and levosalbutamol
lxvi. Daratropium, tiotropium and terbutaline
lxvii. Daratropium, tiotropium and pirbuterol
lxviii. Daratropium, tiotropium and procaterol
lxix. Daratropium, tiotropium and fenoterol
lxx. Daratropium, tiotropium and bitolterol
lxxi. Daratropium, tiotropium and ritodrine
lxxii. Daratropium, tiotropium and metaproterenol
lxxiii. Daratropium, aclidinium and salbutamol
lxxiv. Daratropium, aclidinium and levosalbutamol
lxxv. Daratropium, aclidinium and terbutaline
lxxvi. Daratropium, aclidinium and pirbuterol
lxxvii. Daratropium, aclidinium and procaterol
lxxviii. Daratropium, aclidinium and fenoterol
lxxix. Daratropium, aclidinium and bitolterol
lxxx. Daratropium, aclidinium and ritodrine
lxxxi. Daratropium, aclidinium and metaproterenol
lxxxii. Daratropium, glycopyrronium and salbutamol
lxxxiii. Daratropium, glycopyrronium and levosalbutamol
lxxxiv. Daratropium, glycopyrronium and terbutaline
lxxxv. Daratropium, glycopyrronium and pirbuterol
lxxxvi. Daratropium, glycopyrronium and procaterol
lxxxvii. Daratropium, glycopyrronium and fenoterol
lxxxviii. Daratropium, glycopyrronium and bitolterol
lxxxix. Daratropium, glycopyrronium and ritodrine
xc. Daratropium, glycopyrronium and metaproterenol
xci. Daratropium, ipratropium and salbutamol
xcii. Daratropium, ipratropium and levosalbutamol
xciii. Daratropium, ipratropium and terbutaline
xciv. Daratropium, ipratropium and pirbuterol
xcv. Daratropium, ipratropium and procaterol
xcvi. Daratropium, ipratropium and fenoterol
xcvii. Daratropium, ipratropium and bitolterol
xcviii. Daratropium, ipratropium and ritodrine
xcix. Daratropium, ipratropium and metaproterenol
c. Daratropium, oxitropium and salbutamol
ci. Daratropium, oxitropium and levosalbutamol
cii. Daratropium, oxitropium and terbutaline
ciii. Daratropium, oxitropium and pirbuterol
civ. Daratropium, oxitropium and procaterol
cv. Daratropium, oxitropium and fenoterol
cvi. Daratropium, oxitropium and bitolterol
cvii. Daratropium, oxitropium and ritodrine
cviii. Daratropium, oxitropium and metaproterenol
cix. Indacaterol, tirotropium and salbutamol
cx. Indacaterol, tirotropium and levosalbutamol
cxi. Indacaterol, tirotropium and terbutaline
cxii. Indacaterol, tirotropium and pirbuterol
cxiii. Indacaterol, tirotropium and procaterol
cxiv. Indacaterol, tirotropium and fenoterol
cxv. Indacaterol, tirotropium and bitolterol
cxvi. Indacaterol, tirotropium and ritodrine
cxvii. Indacaterol, tirotropium and metaproterenol
cxviii. Indacaterol, glycopyrronium and salbutamol
cxix. Indacaterol, glycopyrronium and levosalbutamol
cxx. Indacaterol, glycopyrronium and terbutaline
cxxi. Indacaterol, glycopyrronium and pirbuterol
cxxii. Indacaterol, glycopyrronium and procaterol
cxxiii. Indacaterol, glycopyrronium and fenoterol
cxxiv. Indacaterol, glycopyrronium and bitolterol
cxxv. Indacaterol, glycopyrronium and ritodrine
cxxvi. Indacaterol, glycopyrronium and metaproterenol
cxxvii. Indacaterol, aclidinium and salbutamol
cxxviii. Indacaterol, aclidinium and levosalbutamol
cxxix. Indacaterol, aclidinium and terbutaline
cxxx. Indacaterol, aclidinium and pirbuterol
cxxxi. Indacaterol, aclidinium and procaterol
cxxxii. Indacaterol, aclidinium and fenoterol
cxxxiii. Indacaterol, aclidinium and bitolterol
cxxxiv. Indacaterol, aclidinium and ritodrine
cxxxv. Indacaterol, aclidinium and metaproterenol
cxxxvi. Indacaterol, ipratropium and salbutamol
cxxxvii. Indacaterol, ipratropium and levosalbutamol
cxxxviii. Indacaterol, ipratropium and terbutaline
cxxxix. Indacaterol, ipratropium and pirbuterol
cxl. Indacaterol, ipratropium and procaterol
cxli. Indacaterol, ipratropium and fenoterol
cxlii. Indacaterol, ipratropium and bitolterol
cxliii. Indacaterol, ipratropium and ritodrine
cxliv. Indacaterol, ipratropium and metaproterenol
cxlv. Indacaterol, oxitropium and salbutamol
cxlvi. Indacaterol, oxitropium and levosalbutamol
cxlvii. Indacaterol, oxitropium and terbutaline
cxlviii. Indacaterol, oxitropium and pirbuterol
cxlix. Indacaterol, oxitropium and procaterol
cl. Indacaterol, oxitropium and fenoterol
cli. Indacaterol, oxitropium and bitolterol
clii. Indacaterol, oxitropium and ritodrine
cliii. Indacaterol, oxitropium and metaproterenol
cliv. Vilanterol, tiotropium and salbutamol
clv. Vilanterol, tiotropium and levosalbutamol
clvi. Vilanterol, tiotropium and terbutaline
clvii. Vilanterol, tiotropium and pirbuterol
clviii. Vilanterol, tiotropium and procaterol
clix. Vilanterol, tiotropium and fenoterol
clx. Vilanterol, tiotropium and bitolterol
clxi. Vilanterol, tiotropium and ritodrine
clxii. Vilanterol, tiotropium and metaproterenol
clxiii. Vilanterol, glycopyrronium and salbutamol
clxiv. Vilanterol, glycopyrronium and levosalbutamol
clxv. Vilanterol, glycopyrronium and terbutaline
clxvi. Vilanterol, glycopyrronium and pirbuterol
clxvii. Vilanterol, glycopyrronium and procaterol
clxviii. Vilanterol, glycopyrronium and fenoterol
clxix. Vilanterol, glycopyrronium and bitolterol
clxx. Vilanterol, glycopyrronium and ritodrine
clxxi. Vilanterol, glycopyrronium and metaproterenol
clxxii. Vilanterol, ipratropium and salbutamol
clxxiii. Vilanterol, ipratropium and levosalbutamol
clxxiv. Vilanterol, ipratropium and terbutaline
clxxv. Vilanterol, ipratropium and pirbuterol
clxxvi. Vilanterol, ipratropium and procaterol
clxxvii. Vilanterol, ipratropium and fenoterol
clxxviii. Vilanterol, ipratropium and bitolterol
clxxix. Vilanterol, ipratropium and ritodrine
clxxx. Vilanterol, ipratropium and metaproterenol
clxxxi. Vilanterol, aclidinium and salbutamol
clxxxii. Vilanterol, aclidinium and levosalbutamol
clxxxiii. Vilanterol, aclidinium and terbutaline
clxxxiv.Vilanterol, aclidinium and pirbuterol
clxxxv. Vilanterol, aclidinium and procaterol
clxxxvi.Vilanterol, aclidinium and fenoterol
clxxxvii. Vilanterol, aclidinium and bitolterol
clxxxviii. Vilanterol, aclidinium and ritodrine
clxxxix.Vilanterol, aclidinium and metaproterenol
cxc. Vilanterol, oxitropium and salbutamol
cxci. Vilanterol, oxitropium and levosalbutamol
cxcii. Vilanterol, oxitropium and terbutaline
cxciii. Vilanterol, oxitropium and pirbuterol
cxciv. Vilanterol, oxitropium and procaterol
cxcv. Vilanterol, oxitropium and fenoterol
cxcvi. Vilanterol, oxitropium and bitolterol
cxcvii. Vilanterol, oxitropium and ritodrine
cxcviii. Vilanterol, oxitropium and metaproterenol
cxcix. Carmoterol, tiotropium and salbutamol
cc. Carmoterol, tiotropium and levosalbutamol
cci. Carmoterol, tiotropium and terbutaline
ccii. Carmoterol, tiotropium and pirbuterol
cciii. Carmoterol, tiotropium and procaterol
cciv. Carmoterol, tiotropium and fenoterol
ccv. Carmoterol, tiotropium and bitolterol
ccvi. Carmoterol, tiotropium and ritodrine
ccvii. Carmoterol, tiotropium and metaproterenol
ccviii. Carmoterol, ipratropium and levosalbutamol
ccix. Carmoterol, ipratropium and salbutamol
ccx. Carmoterol, ipratropium and terbutaline
ccxi. Carmoterol, ipratropium and pirbuterol
ccxii. Carmoterol, ipratropium and procaterol
ccxiii. Carmoterol, ipratropium and fenoterol
ccxiv. Carmoterol, ipratropium and bitolterol
ccxv. Carmoterol, ipratropium and ritodrine
ccxvi. Carmoterol, ipratropium and metaproterenol
ccxvii. Carmoterol, aclidinum and levosalbutamol
ccxviii. Carmoterol, aclidinum and salbutamol
ccxix. Carmoterol, aclidinum and terbutaline
ccxx. Carmoterol, aclidinum and pirbuterol
ccxxi. Carmoterol, aclidinum and procaterol
ccxxii. Carmoterol, aclidinum and fenoterol
ccxxiii. Carmoterol, aclidinum and bitolterol
ccxxiv. Carmoterol, aclidinum and ritodrine
ccxxv. Carmoterol, aclidinum and metaproterenol
ccxxvi. Carmoterol, oxitropium and salbutamol
ccxxvii.Carmoterol, oxitropium and levosalbutamol
ccxxviii. Carmoterol, oxitropium and terbutaline
ccxxix. Carmoterol, oxitropium and pirbuterol
ccxxx. Carmoterol, oxitropium and procaterol
ccxxxi. Carmoterol, oxitropium and fenoterol
ccxxxii.Carmoterol, oxitropium and bitolterol
ccxxxiii. Carmoterol, oxitropium and ritodrine
ccxxxiv. Carmoterol, oxitropium and metaproterenol
ccxxxv. Olodaterol, tiotropium and salbutamol
ccxxxvi. Olodaterol, tiotropium and levosalbutamol
ccxxxvii. Olodaterol, tiotropium and terbutaline
ccxxxviii. Olodaterol, tiotropium and pirbuterol
ccxxxix. Olodaterol, tiotropium and procaterol
ccxl. Olodaterol, tiotropium and fenoterol
ccxli. Olodaterol, tiotropium and bitolterol
ccxlii. Olodaterol, tiotropium and ritodrine
ccxliii. Olodaterol, tiotropium and metaproterenol
ccxliv. Olodaterol, ipratropium and salbutamol
ccxlv. Olodaterol, ipratropium and levosalbutamol
ccxlvi. Olodaterol, ipratropium and terbutaline
ccxlvii. Olodaterol, ipratropium and pirbuterol
ccxlviii.Olodaterol, ipratropium and procaterol
ccxlix. Olodaterol, ipratropium and fenoterol
ccl. Olodaterol, ipratropium and bitolterol
ccli. Olodaterol, ipratropium and ritodrine
cclii. Olodaterol, ipratropium and metaproterenol
ccliii. Olodaterol, aclidinum and salbutamol
ccliv. Olodaterol, aclidinum and levosalbutamol
cclv. Olodaterol, aclidinum and terbutaline
cclvi. Olodaterol, aclidinum and pirbuterol
cclvii. Olodaterol, aclidinum and procaterol
cclviii. Olodaterol, aclidinum and fenoterol
cclix. Olodaterol, aclidinum and bitolterol
cclx. Olodaterol, aclidinum and ritodrine
cclxi. Olodaterol, aclidinum and metaproterenol
cclxii. Olodaterol, glycopyrronium and salbutamol
cclxiii. Olodaterol, glycopyrronium and levosalbutamol
cclxiv. Olodaterol, glycopyrronium and terbutaline
cclxv. Olodaterol, glycopyrronium and pirbuterol
cclxvi. Olodaterol, glycopyrronium and procaterol
cclxvii. Olodaterol, glycopyrronium and fenoterol
cclxviii.Olodaterol, glycopyrronium and bitolterol
cclxix. Olodaterol, glycopyrronium and ritodrine
cclxx. Olodaterol, glycopyrronium and metaproterenol
cclxxi. Olodaterol, oxitropium and salbutamol
cclxxii. Olodaterol, oxitropium and levosalbutamol
cclxxiii. Olodaterol, oxitropium and terbutaline
cclxxiv.Olodaterol, oxitropium and pirbuterol
cclxxv. Olodaterol, oxitropium and procaterol
cclxxvi.Olodaterol, oxitropium and fenoterol
cclxxvii. Olodaterol, oxitropium and bitolterol
cclxxviii. Olodaterol, oxitropium and ritodrine
cclxxix.Olodaterol, oxitropium and metaproterenol
wherein said therapeutic agents can be present as a pharmaceutically acceptable salt or ester thereof, or in enantiomerically pure form or as a racemic mixture.

18. The pharmaceutical composition according to claims 14, comprising any of the following combinations, said combinations being suitable for administration separately, sequentially or together in effective amounts;
i. Aclidinum, tiotropium and fluticasone
ii. Aclidinum, tiotropium and ciclesonide
iii. Aclidinum, tiotropium and budesonide
iv. Aclidinum, tiotropium and mometasone
v. Aclidinum, tiotropium and beclomethasone
vi. Aclidinum, tiotropium and triamcinolone
vii. Aclidinum, tiotropium and flunisolide
viii. Aclidinum, tiotropium and dexomethasone
ix. Daratropium, tiotropium and fluticasone
x. Daratropium, tiotropium and ciclesonide
xi. Daratropium, tiotropium and budesonide
xii. Daratropium, tiotropium and mometasone
xiii. Daratropium, tiotropium and beclamethasone
xiv. Daratropium, tiotropium and triamcinolone
xv. Daratropium, tiotropium and flunisolide
xvi. Daratropium, tiotropium and dexomethasone
xvii. Indacaterol, tiotropium and fluticasone
xviii. Indacaterol, tiotropium and budesonide
xix. Indacaterol, tiotropium and ciclesonide
xx. Indacaterol, tiotropium and mometasone
xxi. Indacaterol, tiotropium and beclamethasone
xxii. Indacaterol, tiotropium and triamcinolone
xxiii. Indacaterol, tiotropium and flunisolide
xxiv. Indacaterol, tiotropium and dexomethasone
xxv. Vilanterol, tiotropium and ciclesonide
xxvi. vilanterol, tiotropium and fluticasone
xxvii. vilanterol, tiotropium and budesonide
xxviii. vilanterol, tiotropium and mometasone
xxix. vilanterol, tiotropium and beclamethasone
xxx. vilanterol, tiotropium and triamcinolone
xxxi. vilanterol, tiotropium and flunisolide
xxxii. vilanterol, tiotropium and dexomethasone
xxxiii. carmoterol, tiotropium and budesonide
xxxiv. carmoterol, tiotropium and ciclesonide
xxxv. carmoterol, tiotropium and fluticasone
xxxvi. carmoterol, tiotropium and mometasone
xxxvii. carmoterol, tiotropium and beclamethasone
xxxviii. carmoterol, tiotropium and triamcinolone
xxxix. carmoterol, tiotropium and flunisolide
xl. carmoterol, tiotropium and dexomethasone
xli. Olodaterol, tiotropium and ciclesonide
xlii. Olodaterol, tiotropium and fluticasone
xliii. Olodaterol, tiotropium and budesonide
xliv. Olodaterol, tiotropium and mometasone
xlv. Olodaterol, tiotropium and beclamethasone
xlvi. Olodaterol, tiotropium and triamcinolone
xlvii. Olodaterol, tiotropium and flunisolide
xlviii. Olodaterol, tiotropium and dexomethasone
wherein said therapeutic agents can be present as a pharmaceutically acceptable salt or ester thereof, or in enantiomerically pure form or as a racemic mixture.

19. The pharmaceutical composition according to claims 15, comprising any of the following combinations, said combinations being suitable for administration separately, sequentially or together in effective amounts;
i. Aclidinium, salmeterol and salbutamol
ii. Aclidinium, salmeterol and levosalbutamol
iii. Aclidinium, formoterol and salbutamol
iv. Aclidinium, formoterol and levosalbutamol
v. Aclidinium, arformoterol and salbutamol
vi. Aclidinium, arformoterol and levosalbutamol
vii. Aclidinium, indacaterol and salbutamol
viii. Aclidinium, indacaterol and levosalbutamol
ix. Aclidinium, olodaterol and salbutamol
x. Aclidinium, olodaterol and levosalbutamol
xi. Aclidinium, vilanterol and salbutamol
xii. Aclidinium, vilanterol and levosalbutamol
xiii. Aclidinium, carmoterol and salbutamol
xiv. Aclidinium, carmoterol and levosalbutamol
xv. Aclidinium, bambuterol and salbutamol
xvi. Aclidinium, bambuterol and levosalbutamol
xvii. Glycopyrronium, indacaterol and salbutamol
xviii. Glycopyrronium, indacaterol and levosalbutamol
xix. Glycopyrronium, salmeterol and salbutamol
xx. Glycopyrronium, salmeterol and levosalbutamol
xxi. Glycopyrronium, formoterol and salbutamol
xxii. Glycopyrronium, formoterol and levosalbutamol
xxiii. Glycopyrronium, arformoterol and salbutamol
xxiv. Glycopyrronium, arformoterol and levosalbutamol
xxv. Glycopyrronium, carmoterol and salbutamol
xxvi. Glycopyrronium, carmoterol and levosalbutamol
xxvii. Glycopyrronium, olodaterol and salbutamol
xxviii. Glycopyrronium, olodaterol and levosalbutamol
xxix. Glycopyrronium, vilanterol and salbutamol
xxx. Glycopyrronium, vilanterol and levosalbutamol
xxxi. Glycopyrronium, bambuterol and salbutamol
xxxii. Glycopyrronium, bambuterol and levosalbutamol
xxxiii. Daratropium, indacaterol and salbutamol
xxxiv. Daratropium, indacaterol and levosalbutamol
xxxv. Daratropium, salmeterol and salbutamol
xxxvi. Daratropium, salmeterol and levosalbutamol
xxxvii. Daratropium, formoterol and salbutamol
xxxviii. Daratropium, formoterol and levosalbutamol
xxxix. Daratropium, carmoterol and salbutamol
xl. Daratropium, carmoterol and levosalbutamol
xli. Daratropium, olodaterol and salbutamol
xlii. Daratropium, olodaterol and levosalbutamol
xliii. Daratropium, vilanterol and salbutamol
xliv. Daratropium, vilanterol and levosalbutamol
xlv. Daratropium, bambuterol and salbutamol
xlvi. Daratropium, bambuterol and levosalbutamol
xlvii. Daratropium, arformoterol and salbutamol
xlviii. Daratropium, arformoterol and levosalbutamol
wherein said therapeutic agents can be present as a pharmaceutically acceptable salt or ester thereof, or in enantiomerically pure form or as a racemic mixture.

20. The pharmaceutical composition according to claims 16, comprising any of the following combinations, said combinations being suitable for administration separately, sequentially or together in effective amounts;
i. Aclidinium, salmeterol and mometasone
ii. Aclidinium, salmeterol and fluticasone
iii. Aclidinium, salmeterol and budesonide
iv. Aclidinium, formoterol and mometasone
v. Aclidinium, formoterol and fluticasone
vi. Aclidinium, formoterol and budesonide
vii. Aclidinium, arformoterol and mometasone
viii. Aclidinium, arformoterol and fluticasone
ix. Aclidinium, arformoterol and budesonide
x. Aclidinium, indacaterol and mometasone
xi. Aclidinium, indacaterol and fluticasone
xii. Aclidinium, indacaterol and budesonide
xiii. Aclidinium, olodaterol and mometasone
xiv. Aclidinium, olodaterol and fluticasone
xv. Aclidinium, olodaterol and budesonide
xvi. Aclidinium, vilanterol and mometasone
xvii. Aclidinium, vilanterol and fluticasone
xviii. Aclidinium, vilanterol and budesonide
xix. Aclidinium, carmoterol and mometasone
xx. Aclidinium, carmoterol and fluticasone
xxi. Aclidinium, carmoterol and budesonide
xxii. Aclidinium, bambuterol and mometasone
xxiii. Aclidinium, bambuterol and fluticasone
xxiv. Aclidinium, bambuterol and budesonide
xxv. Glycopyrronium, indacaterol and mometasone
xxvi. Glycopyrronium, indacaterol and fluticasone
xxvii. Glycopyrronium, indacaterol and budesonide
xxviii. Glycopyrronium, salmeterol and mometasone
xxix. Glycopyrronium, salmeterol and fluticasone
xxx. Glycopyrronium, salmeterol and budesonide
xxxi. Glycopyrronium, formoterol and mometasone
xxxii. Glycopyrronium, formoterol and fluticasone
xxxiii. Glycopyrronium, formoterol and budesonide
xxxiv. Glycopyrronium, arformoterol and mometasone
xxxv. Glycopyrronium, arformoterol and fluticasone
xxxvi. Glycopyrronium, arformoterol and budesonide
xxxvii. Glycopyrronium, carmoterol and mometasone
xxxviii. Glycopyrronium, carmoterol and fluticasone
xxxix. Glycopyrronium, carmoterol and budesonide
xl. Glycopyrronium, olodaterol and mometasone
xli. Glycopyrronium, olodaterol and fluticasone
xlii. Glycopyrronium, olodaterol and budesonide
xliii. Glycopyrronium, vilanterol and mometasone
xliv. Glycopyrronium, vilanterol and fluticasone
xlv. Glycopyrronium, vilanterol and budesonide
xlvi. Glycopyrronium, bambuterol and mometasone
xlvii. Glycopyrronium, bambuterol and fluticasone
xlviii. Glycopyrronium, bambuterol and budesonide
xlix. Daratropium, indacaterol and mometasone
l. Daratropium, indacaterol and fluticasone
li. Daratropium, indacaterol and budesonide
lii. Daratropium, salmeterol and mometasone
liii. Daratropium, salmeterol and fluticasone
liv. Daratropium, salmeterol and budesonide
lv. Daratropium, formoterol and mometasone
lvi. Daratropium, formoterol and fluticasone
lvii. Daratropium, formoterol and budesonide
lviii. Daratropium, carmoterol and mometasone
lix. Daratropium, carmoterol and fluticasone
lx. Daratropium, carmoterol and budesonide
lxi. Daratropium, olodaterol and mometasone
lxii. Daratropium, olodaterol and fluticasone
lxiii. Daratropium, olodaterol and budesonide
lxiv. Daratropium, vilanterol and mometasone
lxv. Daratropium, vilanterol and fluticasone
lxvi. Daratropium, vilanterol and budesonide
lxvii. Daratropium, bambuterol and mometasone
lxviii. Daratropium, bambuterol and fluticasone
lxix. Daratropium, bambuterol and budesonide
lxx. Daratropium, arformoterol and mometasone
lxxi. Daratropium, arformoterol and fluticasone
lxxii. Daratropium, arformoterol and budesonide
lxxiii. Indacaterol, salmeterol and mometasone
lxxiv. Indacaterol, salmeterol and fluticasone
lxxv. Indacaterol, salmeterol and budesonide
lxxvi. Indacaterol, formoterol and mometasone
lxxvii. Indacaterol, formoterol and fluticasone
lxxviii. Indacaterol, formoterol and budesonide
lxxix. Indacaterol, arformoterol and mometasone
lxxx. Indacaterol, arformoterol and fluticasone
lxxxi. Indacaterol, arformoterol and budesonide
lxxxii. Indacaterol, olodaterol and mometasone
lxxxiii. Indacaterol, olodaterol and fluticasone
lxxxiv. Indacaterol, olodaterol and budesonide
lxxxv. Indacaterol, vilanterol and mometasone
lxxxvi. Indacaterol, vilanterol and fluticasone
lxxxvii. Indacaterol, vilanterol and budesonide
lxxxviii. Indacaterol, carmeterol and mometasone
lxxxix. Indacaterol, carmeterol and fluticasone
xc. Indacaterol, carmeterol and budesonide
xci. Indacaterol, bambuterol and mometasone
xcii. Indacaterol, bambuterol and fluticasone
xciii. Indacaterol, bambuterol and budesonide
xciv. Vilanterol, salmeterol and mometasone
xcv. Vilanterol, salmeterol and fluticasone
xcvi. Vilanterol, salmeterol and budesonide
xcvii. Vilanterol, formoterol and mometasone
xcviii. Vilanterol, formoterol and fluticasone
xcix. Vilanterol, formoterol and budesonide
c. Vilanterol, arformoterol and mometasone
ci. Vilanterol, arformoterol and fluticasone
cii. Vilanterol, arformoterol and budesonide
ciii. Vilanterol, olodaterol and mometasone
civ. Vilanterol, olodaterol and fluticasone
cv. Vilanterol, olodaterol and budesonide
cvi. Vilanterol, carmeterol and mometasone
cvii. Vilanterol, carmeterol and fluticasone
cviii. Vilanterol, carmeterol and budesonide
cix. Vilanterol, bambuterol and mometasone
cx. Vilanterol, bambuterol and fluticasone
cxi. Vilanterol, bambuterol and budesonide
cxii. Vilanterol, indacaterol and mometasone
cxiii. Vilanterol, indacaterol and fluticasone
cxiv. Vilanterol, indacaterol and budesonide
cxv. Carmeterol, salmeterol and mometasone
cxvi. Carmeterol, salmeterol and fluticasone
cxvii. Carmeterol, salmeterol and budesonide
cxviii. Carmeterol, formoterol and mometasone
cxix. Carmeterol, formoterol and fluticasone
cxx. Carmeterol, formoterol and budesonide
cxxi. Carmeterol, arformoterol and mometasone
cxxii. Carmeterol, arformoterol and fluticasone
cxxiii. Carmeterol, arformoterol and budesonide
cxxiv. Carmeterol, indaceterol and mometasone
cxxv. Carmeterol, indaceterol and fluticasone
cxxvi. Carmeterol, indaceterol and budesonide
cxxvii. Carmeterol, olodaterol and mometasone
cxxviii. Carmeterol, olodaterol and fluticasone
cxxix. Carmeterol, olodaterol and budesonide
cxxx. Carmeterol, vilanterol and mometasone
cxxxi. Carmeterol, vilanterol and fluticasone
cxxxii. Carmeterol, vilanterol and budesonide
cxxxiii. Carmeterol, bambuterol and mometasone
cxxxiv. Carmeterol, bambuterol and fluticasone
cxxxv. Carmeterol, bambuterol and budesonide
cxxxvi. Olodaterol, salmeterol and mometasone
cxxxvii. Olodaterol, salmeterol and fluticasone
cxxxviii. Olodaterol, salmeterol and budesonide
cxxxix. Olodaterol, formoterol and mometasone
cxl. Olodaterol, formoterol and fluticasone
cxli. Olodaterol, formoterol and budesonide
cxlii. Olodaterol, arformoterol and mometasone
cxliii. Olodaterol, arformoterol and fluticasone
cxliv. Olodaterol, arformoterol and budesonide
cxlv. Olodaterol, indacaterol and mometasone
cxlvi. Olodaterol, indacaterol and fluticasone
cxlvii. Olodaterol, indacaterol and budesonide
cxlviii. Olodaterol, vilanterol and mometasone
cxlix. Olodaterol, vilanterol and fluticasone
cl. Olodaterol, vilanterol and budesonide
cli. Olodaterol, carmeterol and mometasone
clii. Olodaterol, carmeterol and fluticasone
cliii. Olodaterol, carmeterol and budesonide
cliv. Olodaterol, bambuterol and mometasone
clv. Olodaterol, bambuterol and fluticasone
clvi. Olodaterol, bambuterol and budesonide
wherein said therapeutic agents can be present as a pharmaceutically acceptable salt or ester thereof, or in enantiomerically pure form or as a racemic mixture.

21. The pharmaceutical composition according to claims 17, comprising any of the following combinations, said combinations being suitable for administration separately, sequentially or together in effective amounts;
i. Aclidinium, salbutamol and fluticasone
ii. Aclidinium, levosalbutamol and fluticasone
iii. Aclidinium, salbutamol and ciclesonide
iv. Aclidinium, levosalbutamol and ciclesonide
v. Aclidinium, salbutamol and budesonide
vi. Aclidinium, levosalbutamol and budesonide
vii. Aclidinium, salbutamol and mometasone
viii. Aclidinium, levosalbutamol and mometasone
ix. Aclidinium, salbutamol and beclometahsone
x. Aclidinium, levosalbutamol and beclometahsone
xi. Aclidinium, salbutamol and triamcinolone
xii. Aclidinium, levosalbutamol and triamcinolone
xiii. Aclidinium, salbutamol and flunisolide
xiv. Aclidinium, levosalbutamol and flunisolide
xv. Aclidinium, salbutamol and dexamethasone
xvi. Aclidinium, levosalbutamol and dexamethasone
xvii. Glycopyrronium, salbutamol and fluticasone
xviii. Glycopyrronium, levosalbutamol and fluticasone
xix. Glycopyrronium, salbutamol and ciclesonide
xx. Glycopyrronium, levosalbutamol and ciclesonide
xxi. Glycopyrronium, salbutamol and budesonide
xxii. Glycopyrronium, levosalbutamol and budesonide
xxiii. Glycopyrronium, salbutamol and mometasone
xxiv. Glycopyrronium, levosalbutamol and mometasone
xxv. Glycopyrronium, salbutamol and beclometahsone
xxvi. Glycopyrronium, levosalbutamol and beclometahsone
xxvii. Glycopyrronium, salbutamol and triamcinolone
xxviii. Glycopyrronium, levosalbutamol and triamcinolone
xxix. Glycopyrronium, salbutamol and flunisolide
xxx. Glycopyrronium, levosalbutamol and flunisolide
xxxi. Glycopyrronium, salbutamol and dexamethasone
xxxii. Glycopyrronium, levosalbutamol and dexamethasone
xxxiii. Daratropium, salbutamol and fluticasone
xxxiv. Daratropium, levosalbutamol and fluticasone
xxxv. Daratropium, salbutamol and ciclesonide
xxxvi. Daratropium, levosalbutamol and ciclesonide
xxxvii. Daratropium, salbutamol and budesonide
xxxviii. Daratropium, levosalbutamol and budesonide
xxxix. Daratropium, salbutamol and mometasone
xl. Daratropium, levosalbutamol and mometasone
xli. Daratropium, salbutamol and beclometahsone
xlii. Daratropium, levosalbutamol and beclometahsone
xliii. Daratropium, salbutamol and triamcinolone
xliv. Daratropium, levosalbutamol and triamcinolone
xlv. Daratropium, salbutamol and flunisolide
xlvi. Daratropium, levosalbutamol and flunisolide
xlvii. Daratropium, salbutamol and dexamethasone
xlviii. Daratropium, levosalbutamol and dexamethasone
xlix. Indacaterol, salbutamol and fluticasone
l. Indacaterol, levosalbutamol and fluticasone
li. Indacaterol, salbutamol and ciclesonide
lii. Indacaterol, levosalbutamol and ciclesonide
liii. Indacaterol, salbutamol and budesonide
liv. Indacaterol, levosalbutamol and budesonide
lv. Indacaterol, salbutamol and mometasone
lvi. Indacaterol, levosalbutamol and mometasone
lvii. Indacaterol, salbutamol and beclometahsone
lviii. Indacaterol, levosalbutamol and beclometahsone
lix. Indacaterol, salbutamol and triamcinolone
lx. Indacaterol, levosalbutamol and triamcinolone
lxi. Indacaterol, salbutamol and flunisolide
lxii. Indacaterol, levosalbutamol and flunisolide
lxiii. Indacaterol, salbutamol and dexamethasone
lxiv. Indacaterol, levosalbutamol and dexamethasone
lxv. Vilanterol, salbutamol and fluticasone
lxvi. Vilanterol, levosalbutamol and fluticasone
lxvii. Vilanterol, salbutamol and budesonide
lxviii. Vilanterol, levosalbutamol and budesonide
lxix. Vilanterol, salbutamol and ciclesonide
lxx. Vilanterol, levosalbutamol and ciclesonide
lxxi. Vilanterol, salbutamol and mometasone
lxxii. Vilanterol, levosalbutamol and mometasone
lxxiii. Vilanterol, salbutamol and beclomethasone
lxxiv. Vilanterol, levosalbutamol and beclomethasone
lxxv. Vilanterol, salbutamol and triamcinolone
lxxvi. Vilanterol, levosalbutamol and triamcinolone
lxxvii. Vilanterol, salbutamol and flunisolide
lxxviii. Vilanterol, levosalbutamol and flunisolide
lxxix. Vilanterol, salbutamol and dexamethasone
lxxx. Vilanterol, levosalbutamol and dexamethasone
lxxxi. Carmeterol, salbutamol and fluticasone
lxxxii. Carmeterol, levosalbutamol and fluticasone
lxxxiii. Carmeterol, salbutamol and ciclesonide
lxxxiv. Carmeterol, levosalbutamol and ciclesonide
lxxxv. Carmeterol, salbutamol and budesonide
lxxxvi. Carmeterol, levosalbutamol and budesonide
lxxxvii. Carmeterol, salbutamol and mometasone
lxxxviii. Carmeterol, levosalbutamol and mometasone
lxxxix. Carmeterol, salbutamol and beclomethasone
xc. Carmeterol, levosalbutamol and beclomethasone
xci. Carmeterol, salbutamol and triamcinolone
xcii. Carmeterol, levosalbutamol and triamcinolone
xciii. Carmeterol, salbutamol and flunisolide
xciv. Carmeterol, levosalbutamol and flunisolide
xcv. Carmeterol, salbutamol and dexamethasone
xcvi. Carmeterol, levosalbutamol and dexamethasone
xcvii. Olodaterol, salbutamol and fluticasone
xcviii. Olodaterol, levosalbutamol and fluticasone
xcix. Olodaterol, salbutamol and ciclesonide
c. Olodaterol, levosalbutamol and ciclesonide
ci. Olodaterol, salbutamol and budesonide
cii. Olodaterol, levosalbutamol and budesonide
ciii. Olodaterol, salbutamol and mometasone
civ. Olodaterol, levosalbutamol and mometasone
cv. Olodaterol, salbutamol and beclomethasone
cvi. Olodaterol, levosalbutamol and beclomethasone
cvii. Olodaterol, salbutamol and triamcinolone
cviii. Olodaterol, levosalbutamol and triamcinolone
cix. Olodaterol, salbutamol and flunisolide
cx. Olodaterol, levosalbutamol and flunisolide
cxi. Olodaterol, salbutamol and dexamethasone
cxii. Olodaterol, levosalbutamol and dexamethasone
wherein said therapeutic agents can be present as a pharmaceutically acceptable salt or ester thereof, or in enantiomerically pure form or as a racemic mixture.

22. The pharmaceutical composition according to claims 12 to 17, comprising any of the following combinations, said combinations being suitable for administration separately, sequentially or together in effective amounts;
i. Formoterol, budesonide and tiotropium
ii. Salmeterol, fluticasone and tiotropium
iii. Carmoterol, tiotropium and fluticasone
iv. Salbutamol, formoterol and budesonide
v. Salbutamol, salmeterol and fluticasone
vi. Salbutamol, arformoterol and fluticasone
wherein said therapeutic agents can be present as a pharmaceutically acceptable salt or ester thereof, or in enantiomerically pure form or as a racemic mixture.

23. The pharmaceutical composition according to any of the preceding claims, wherein the therapeutically effective amount of said pharmaceutical composition is administered once a day or said pharmaceutical composition is administered twice a day..

24. The pharmaceutical composition according to any of the preceding claims, for use in the treatment of respiratory condition selected from asthma and chronic obstructive pulmonary disease and other obstructive airways diseases.

25. The pharmaceutical composition according to any of the preceding claims, wherein said pharmaceutical composition being suitable for administration separately, sequentially or together in effective amounts, together with a moisture tight and high barrier sealed blister.

26. The pharmaceutical composition according to any of the preceding claims, wherein said pharmaceutical composition being suitable for administration separately, sequentially or together in effective amounts, together with a capsule.

27. The pharmaceutical composition according to claim 25 or 26, wherein said pharmaceutical composition being suitable for administration separately, sequentially or together in effective amounts, together with a dry powder inhalation device.

28. The pharmaceutical composition according to claim 25, wherein said pharmaceutical composition being suitable for administration separately, sequentially or together in effective amounts, together with an inhalation device, **characterized by** said device comprises at least one lock mechanism, enabling the device to remain locked in both positions in which the device is ready for inhalation and the lid is in the closed position, and further enabling the device to setup again automatically, when the lid is closed.

29. A pharmaceutical kit comprising the drugs having amine as defined in claim 1 and one or more additional active agents as defined in claim 7 in the form of a dry powder in admixture with a pharmaceutically acceptable carrier, other than lactose as defined in claim 1, in separate unit dosage forms, said forms being suitable for administration separately, sequentially or together in effective amounts, together with one or more inhalation devices for administration of drugs having amine and one or more additional active agents.

## Patentansprüche

1. Pharmazeutische Zusammensetzung zur Inhalation, die separat, sequentiell oder zusammen verabreicht wird, umfassend Arzneien mit Amin in der Form eines trockenen Pulvers, die ausgewählt sind aus der Gruppe, bestehend aus Aclidinium, Glycopyrronium, Daratropium, Indacaterol, Vilanterol, Carmoterol und Olodaterol oder ihr pharmazeutisch akzeptables Salz oder Ester, oder in enantiomer-reiner Form oder als eine razemische Mischung in Beimischung mit einem pharmazeutisch akzeptablen Träger, der nicht Laktose ist, wobei der pharmazeutisch akzeptable Träger feine und grobe Partikel umfasst, wobei der pharmazeutisch akzeptable Träger ausgewählt ist aus der Gruppe, bestehend aus Mannitol, Glucose, Trehalose, Cellobiose, Sorbitol und Maltitol oder einer Kombination von zwei oder mehreren davon, und wobei das Verhältnis zwischen den feinen Partikeln zu den groben Partikeln zwischen 0,01 bis 0,25, bezogen auf das Gewicht, beträgt.

2. Pharmazeutische Zusammensetzung nach Anspruch 1, wobei die feinen Partikel des pharmazeutisch akzeptablen Trägers einen Partikeldurchmesser von d₁₀ zwischen 1,0-4,0 µm, d₅₀ zwischen 4,0 - 7,0 µm und d₉₀ zwischen 7,0 - 15,0 µm haben.

3. Pharmazeutische Zusammensetzung nach Anspruch 1, wobei die groben Partikel des pharmazeutisch akzeptablen Trägers einen Partikeldurchmesser d₁₀ zwischen 10 - 50 µm, d₅₀ zwischen 50 - 75 µm und d₉₀ zwischen 75 - 250 µm haben.

4. Pharmazeutische Zusammensetzung nach Anspruch 1 bis 3, wobei der pharmazeutisch akzeptable Träger Mannitol ist.

5. Pharmazeutische Zusammensetzung nach Anspruch 4, wobei der pharmazeutisch akzeptable Träger sprühgetrocknetes Mannitol ist.

6. Pharmazeutische Zusammensetzung nach Anspruch 1, wobei der durchschnittliche Partikeldurchmesser (d₅₀) der Arzneien mit Amin zwischen 1,5 - 2,5 µm ist.

7. Pharmazeutische Zusammensetzung nach Anspruch 1, weiterhin umfassend ein oder mehrere zusätzliche aktive Mittel, ausgewählt aus lang wirkenden Muskarinantagonisten, langwirkenden Beta-Agonisten, kurzwirkenden Beta-2-Agonisten, Corticosteroiden oder eine Kombination von zwei oder mehreren davon in Beimischung mit einem pharmazeutisch akzeptablen Träger, der nicht Laktose ist, wie in Anspruch 1 definiert.

8. Pharmazeutische Zusammensetzung nach Anspruch 7, wobei die lang wirkenden Muskarinantgonisten ausgewählt sind aus der Gruppe, umfassend Tiotropium, Glycopyrronium, Ipratropium, Aclidinium, Oxitropium oder ein pharmazeutisch akzeptables Salz oder Ester davon, oder in enantiomer-reiner Form oder als eine razemische Mischung oder eine Kombination von zwei oder mehreren davon.

9. Pharmazeutische Zusammensetzung nach Anspruch 7, wobei die lang wirkenden Beta-Agonisten ausgewählt sind aus der Gruppe, umfassend Salmeterol, Formoterol, Arformoterol, Indacaterol, Olodaterol, Vilanterol, Carmoterol, Bambuterol oder ein pharmazeutisch akzeptables Salz oder Ester davon, oder in enantiomer-reiner Form oder als eine razemische Mischung oder eine Kombination von zwei oder mehreren davon.

10. Pharmazeutische Zusammensetzung nach Anspruch 7, wobei die kurz wirkenden Beta-2-Agonisten ausgewählt sind aus der Gruppe, umfassend Salbutamol, Levosalbutamol, Terbutalin, Pirbuterol, Procaterol, Fenoterol, Bitolterol, Ritodrin, Metaproterenol oder ein pharmazeutisch akzeptables Salz oder Ester davon, oder in enantiomer-reiner Form oder als eine razemische Mischung oder eine Kombination von zwei oder mehreren davon.

11. Pharmazeutische Zusammensetzung nach Anspruch 7, wobei die Corticosteroide ausgewählt sind aus der Gruppe, umfassend Fluticason, Ciclesonid, Budesonid, Mometason, Beclomethason, Triamcinolon, Flunisolid, Dexamethason oder ein pharmazeutisch akzeptables Salz oder Ester davon, oder in enantiomer-reiner Form oder als eine razemische Mischung oder eine Kombination von zwei oder mehreren davon.

12. Pharmazeutische Zusammensetzung nach Anspruch 8, umfassend eine der folgenden Kombinationen, wobei die Kombinationen zur Verabreichung separat, sequentiell oder zusammen in wirksamen Mengen geeignet sind;
i. Aclidinium und Tiotropium
ii. Aclidinium und Glycopyrronium
iii. Aclidinum und Ipratropium
iv. Aclidinum und Oxitropium
v. Glycopyrronium und Tiotropium
vi. Glycopyrronium und Ipratropium
vii.Glycopyrronium und Oxitropium
viii. Darotropium und Tiotropium
ix. Darotropium und Glycopyrronium
x. Darotropium und Ipratropium
xi. Darotropium und Aclidinium
xii.Darotropium und Oxitropium
xiii. Indacaterol und Tiotropium
xiv. Indacaterol und Glycopyrronium
xv. Indacaterol und Ipratropium
xvi. Indacaterol und Aclidinium
xvii. Indacaterol und Oxitropium
xviii. Vilanterol und Tiotropium
xix. Vilanterol und Glycopyrronium
xx. Vilanterol und Ipratropium
xxi. Vilanterol und Aclidinium
xxii. Vilanterol und Oxitropium
xxiii. Carmoterol und Tiotropium
xxiv. Carmoterol und Glycopyrronium
xxv. Carmoterol und Ipratropium
xxvi. Carmoterol und Aclidinium
xxvii. Carmoterol und Oxitropium
xxviii. Olodaterol und Tiotropium
xxix. Olodaterol und Ipratropium
xxx. Olodaterol und Glycopyrronium
xxxi. Olodaterol und Aclidinium
xxxii. Olodaterol und Oxitropium,
wobei die therapeutischen Mittel als ein pharmazeutisch akzeptables Salz oder Ester davon vorhanden sein können, oder in enantiomer-reiner Form oder als eine razemische Mischung.

13. Pharmazeutische Zusammensetzung nach Anspruch 9, umfassend eine der folgenden Kombinationen, wobei die Kombinationen zur Verabreichung separat, sequentiell oder zusammen in wirksamen Mengen geeignet sind;
i. Aclidinium und Salmeterol
ii. Aclidinum und Formoterol
iii. Aclidinium und Arformoterol
iv. Aclidinium und Indacaterol
v. Aclidinium und Olodaterol
vi. Aclidinium und Vilanterol
vii.Aclidinium und Carmoterol
viii. Aclidinium und Bambuterol
ix. Glycopyrronium und Salmeterol
x. Glycopyrronium und Formoterol
xi. Glycopyrronium und Arformoterol
xii.Glycopyrronium und Indacaterol
xiii. Glycopyrronium und Olodaterol
xiv. Glycopyrronium und Vilanterol
xv. Glycopyrronium und Carmoterol
xvi. Glycopyrronium und Bambuterol
xvii. Darotropium und Salmeterol
xviii. Darotropium und Formoterol
xix. Darotropium und Arformoterol
xx. Darotropium und Indacaterol
xxi. Darotropium und Olodaterol
xxii. Darotropium und Vilanterol
xxiii. Darotropium und Carmoterol
xxiv. Darotropium und Bambuterol
xxv. Indacaterol und Salmeterol
xxvi. Indacaterol und Formoterol
xxvii. Indacaterol und Arformoterol
xxviii. Indacaterol und Olodaterol
xxix. Indacaterol und Vilanterol
xxx. Indacaterol und Carmoterol
xxxi. Indacaterol und Bambuterol
xxxii. Vilanterol und Salmeterol
xxxiii. Vilanterol und Formoterol
xxxiv. Vilanterol und Arformoterol
xxxv. Vilanterol und Olodaterol
xxxvi. Vilanterol und Carmoterol
xxxvii. Vilanterol und Bambuterol
xxxviii. Carmoterol und Salmeterol
xxxix. Carmoterol und Formoterol
xl. Carmoterol und Arformoterol
xli.Carmoterol und Olodaterol
xlii. Carmoterol und Bambuterol
xliii. Olodaterol und Salmeterol
xliv. Olodaterol und Formoterol
xlv. Olodaterol und Arformoterol
xlvi. Olodaterol und Bambuterol
wobei die therapeutischen Mittel als ein pharmazeutisch akzeptables Salz oder Ester davon oder in enantiomer-reiner Form oder als eine razemische Mischung vorhanden sein können.

14. Pharmazeutische Zusammensetzung nach Anspruch 10, umfassend eine der folgenden Kombinationen, wobei die Kombinationen zur Verabreichung separat, sequentiell oder zusammen in wirksamen Mengen geeignet sind;
i. Aclidinium und Salbutamol
ii. Aclidinium und Levosalbutamol
iii. Aclidinium und Terbutalin
iv. Aclidinium und Pirbuterol
v. Aclidinium und Procaterol
vi. Aclidinium und Fenoterol
vii.Aclidinium und Bitolterol
viii. Aclidinium und Ritodrin
ix. Aclidinium und Metaproterenol
x. Glycopyrronium und Salbutamol
xi. Glycopyrronium und Levosalbutamol
xii.Glycopyrronium und Terbutalin
xiii. Glycopyrronium und Pirbuterol
xiv. Glycopyrronium und Procaterol
xv. Glycopyrronium und Fenoterol
xvi. Glycopyrronium und Bitolterol
xvii. Glycopyrronium und Ritodrin
xviii. Glycopyrronium und Metaproterenol
xix. Darotropium und Salbutamol
xx. Darotropium und Levosalbutamol
xxi. Darotropium und Terbutalin
xxii. Darotropium und Pirbuterol
xxiii. Darotropium und Procaterol
xxiv. Darotropium und Fenoterol
xxv. Darotropium und Bitolterol
xxvi. Darotropium und Ritodrin
xxvii. Darotropium und Metaproterenol
xxviii. Indacaterol und Salbutamol
xxix. Indacaterol und Levosalbutamol
xxx. Indacaterol und Terbutalin
xxxi. Indacaterol und Pirbuterol
xxxii. Indacaterol und Procaterol
xxxiii. Indacaterol und Fenoterol
xxxiv. Indacaterol und Bitolterol
xxxv. Indacaterol und Ritodrin
xxxvi. Indacaterol und Metaproterenol
xxxvii. Vilanterol und Salbutamol
xxxviii. Vilanterol und Levosalbutamol
xxxix. Vilanterol und Terbutalin
xl. Vilanterol und Pirbuterol
xli.Vilanterol und Procaterol
xlii. Vilanterol und Fenoterol
xliii. Vilanterol und Bitolterol
xliv. Vilanterol und Ritodrin
xlv. Vilanterol und Metaproterenol
xlvi. Carmoterol und Salbutamol
xlvii. Carmoterol und Levosalbutamol
xlviii. Carmoterol und Terbutalin
xlix. Carmoterol und Pirbuterol
l. Carmoterol und Procaterol
li. Carmoterol und Fenoterol
lii. Carmoterol und Bitolterol
liii. Carmoterol und Ritodrin
liv. Carmoterol und Metaproterenol
lv. Olodaterol und Salbutamol
lvi. Olodaterol und Levosalbutamol
lvii. Olodaterol und Terbutalin
lviii. Olodaterol und Pirbuterol
lix.Olodaterol und Procaterol
lx. Olodaterol und Fenoterol
lxi. Olodaterol und Bitolterol
lxii. Olodaterol und Ritodrin
lxiii. Olodaterol und Metaproterenol
wobei die therapeutischen Mittel als ein pharmazeutisch akzeptables Salz oder Ester davon oder in enantiomer-reiner Form oder als eine razemische Mischung vorhanden sein können.

15. Pharmazeutische Zusammensetzung nach Anspruch 11, umfassend eine der folgenden Kombinationen, wobei die Kombinationen zur Verabreichung separat, sequentiell oder zusammen in wirksamen Mengen geeignet sind;
i. Aclidinium und Fluticason
ii. Aclidinium und Ciclesonid
iii. Aclidinium und Budesonid
iv. Aclidinium und Mometason
v. Aclidinium und Beclomethason
vi. Aclidinium und Triamcinolon
vii.Aclidinium und Flunisolid
viii. Aclidinium und Dexamethason
ix. Glycopyrronium und Fluticason
x. Glycopyrronium und Ciclesonid
xi. Glycopyrronium und Budesonid
xii.Glycopyrronium und Mometason
xiii. Glycopyrronium und Beclomethason
xiv. Glycopyrronium und Triamcinolon
xv. Glycopyrronium und Flunisolid
xvi. Glycopyrronium und Dexamethason
xvii. Darotropium und Fluticason
xviii. Darotropium und Ciclesonid
xix. Darotropium und Budesonid
xx. Darotropium und Mometason
xxi. Darotropium und Beclomethason
xxii. Darotropium und Triamcinolon
xxiii. Darotropium und Flunisolid
xxiv. Darotropium und Dexamethason
xxv. Indacaterol und Fluticason
xxvi. Indacaterol und Ciclesonid
xxvii. Indacaterol und Budesonid
xxviii. Indacaterol und Mometason
xxix. Indacaterol und Beclomethason
xxx. Indacaterol und Triamcinolon
xxxi. Indacaterol und Flunisolid
xxxii. Indacaterol und Dexamethason
xxxiii. Vilanterol und Fluticason
xxxiv. Vilanterol und Ciclesonid
xxxv. Vilanterol und Budesonid
xxxvi. Vilanterol und Mometason
xxxvii. Vilanterol und Beclomethason
xxxviii. Vilanterol und Triamcinolon
xxxix. Vilanterol und Flunisolid
xl. Vilanterol und Dexamethason
xli.Carmoterol und Fluticason
xlii. Carmoterol und Ciclesonid
xliii. Carmoterol und Budesonid
xliv. Carmoterol und Mometason
xlv. Carmoterol und Beclomethason
xlvi. Carmoterol und Triamcinolon
xlvii. Carmoterol und Flunisolid
xlviii. Carmoterol und Dexamethason
xlix. Olodaterol und Fluticason
l. Olodaterol und Ciclesonid
li. Olodaterol und Budesonid
lii. Olodaterol und Mometason
liii. Olodaterol und Beclamethason
liv.Olodaterol und Triamcinolon
lv. Olodaterol und Flunisolid
lvi .Olodaterol und Dexamethason
wobei die therapeutischen Mittel als ein pharmazeutisch akzeptables Salz oder Ester davon oder in enantiomer-reiner Form oder als eine razemische Mischung vorhanden sein können.

16. Pharmazeutische Zusammensetzung nach Anspruch 12, umfassend eine der folgenden Kombinationen, wobei die Kombinationen zur Verabreichung separat, sequentiell oder zusammen in wirksamen Mengen geeignet sind;
i. Aclidinum, Tiotropium und Salmeterol
ii. Aclidinum, Tiotropium und Formoterol
iii. Aclidinum, Tiotropium und Arformoterol
iv. Aclidinum, Tiotropium und Indacaterol
v. Aclidinum, Tiotropium und Olodaterol
vi. Aclidinum, Tiotropium und Vilanterol
vii.Aclidinum, Tiotropium und Carmoterol
viii. Aclidinum, Tiotropium und Bambuterol
ix. Aclidinum, Glycopyrronium und Salmeterol
x. Aclidinum, Glycopyrronium und Formoterol
xi. Aclidinum, Glycopyrronium und Arformoterol
xii. Aclidinum, Glycopyrronium und Indacaterol
xiii. Aclidinum, Glycopyrronium und Olodaterol
xiv. Aclidinum, Glycopyrronium und Vilanterol
xv. Aclidinum, Glycopyrronium und Carmoterol
xvi. Aclidinum, Glycopyrronium und Bambuterol
xvii. Aclidinum, Oxitropium und Salmeterol
xviii. Aclidinum, Oxitropium und Formoterol
xix. Aclidinum, Oxitropium und Arformoterol
xx. Aclidinum, Oxitropium und Indacaterol
xxi. Aclidinum, Oxitropium und Olodaterol
xxii. Aclidinum, Oxitropium und Vilanterol
xxiii. Aclidinum, Oxitropium und Carmoterol
xxiv. Aclidinum, Oxitropium und Bambuterol
xxv. Glycopyrronium, Tiotropium und Salmeterol
xxvi. Glycopyrronium, Tiotropium und Formoterol
xxvii. Glycopyrronium, Tiotropium und Arformoterol
xxviii. Glycopyrronium, Tiotropium und Indacaterol
xxix. Glycopyrronium, Tiotropium und Olodaterol
xxx. Glycopyrronium, Tiotropium und Vilanterol
xxxi. Glycopyrronium, Tiotropium und Carmoterol
xxxii. Glycopyrronium, Tiotropium und Bambuterol
xxxiii. Glycopyrronium, Oxitropium und Salmeterol
xxxiv. Glycopyrronium, Oxitropium und Formoterol
xxxv. Glycopyrronium, Oxitropium und Arformoterol
xxxvi. Glycopyrronium, Oxitropium und Indacaterol
xxxvii. Glycopyrronium, Oxitropium und Olodaterol
xxxviii. Glycopyrronium, Oxitropium und Vilanterol
xxxix. Glycopyrronium, Oxitropium und Carmoterol
xl. Glycopyrronium, Oxitropium und Bambuterol
xli.Daratropium, Tiotropium und Salmeterol
xlii. Daratropium, Tiotropium und Formoterol
xliii. Daratropium, Tiotropium und Arformoterol
xliv. Daratropium, Tiotropium und Indacaterol
xlv. Daratropium, Tiotropium und Olodaterol
xlvi. Daratropium, Tiotropium und Vilanterol
xlvii. Daratropium, Tiotropium und Carmoterol
xlviii. Daratropium, Tiotropium und Bambuterol
xlix. Daratropium, Glycopyrronium und Salmeterol
l. Daratropium, Glycopyrronium und Formoterol
li. Daratropium, Glycopyrronium und Arformoterol
lii. Daratropium, Glycopyrronium und Indacaterol
liii.Daratropium, Glycopyrronium und Olodaterol
liv. Daratropium, Glycopyrronium und Vilanterol
lv. Daratropium, Glycopyrronium und Carmoterol
lvi. Daratropium, Glycopyrronium und Bambuterol
lvii. Daratropium, Aclidinium und Salmeterol
lviii. Daratropium, Aclidinium und Formoterol
lix. Daratropium, Aclidinium und Arformoterol
lx. Daratropium, Aclidinium und Indacaterol
lxi. Daratropium, Aclidinium und Olodaterol
lxii. Daratropium, Aclidinium und Vilanterol
lxiii. Daratropium, Aclidinium und Carmoterol
lxiv. Daratropium, Aclidinium und Bambuterol
lxv. Daratropium, Oxitropium und Salmeterol
lxvi. Daratropium, Oxitropium und Formoterol
lxvii. Daratropium, Oxitropium und Arformoterol
lxviii. Daratropium, Oxitropium und Indacaterol
lxix. Daratropium, Oxitropium und Olodaterol
lxx. Daratropium, Oxitropium und Vilanterol
lxxi. Daratropium, Oxitropium und Carmoterol
lxxii. Daratropium, Oxitropium und Bambuterol
lxxiii. Indacaterol, Tiotropium und Salmeterol
lxxiv. Indacaterol, Tiotropium und Formoterol
lxxv. Indacaterol, Tiotropium und Arformoterol
lxxvi. Indacaterol, Tiotropium und Olodaterol
lxxvii. Indacaterol, Tiotropium und Vilanterol
lxxviii. Indacaterol, Tiotropium und Carmoterol
lxxix. Indacaterol, Tiotropium und Bambuterol
lxxx. Indacaterol, Glycopyrronium und Salmeterol
lxxxi. Indacaterol, Glycopyrronium und Formoterol
lxxxii. Indacaterol, Glycopyrronium und Arformoterol
lxxxiii. Indacaterol, Glycopyrronium und Olodaterol
lxxxiv. Indacaterol, Glycopyrronium und Vilanterol
lxxxv. Indacaterol, Glycopyrronium und Carmoterol
lxxxvi. Indacaterol, Glycopyrronium und Bambuterol
lxxxvii. Indacaterol, Aclidinium und Salmeterol
lxxxviii. Indacaterol, Aclidinium und Formoterol
lxxxix. Indacaterol, Aclidinium und Arformoterol
xc.Indacaterol, Aclidinium und Olodaterol
xci. Indacaterol, Aclidinium und Vilanterol
xcii. Indacaterol, Aclidinium und Carmoterol
xciii. Indacaterol, Aclidinium und Bambuterol
xciv. Indacaterol, Oxitropium und Salmeterol
xcv. Indacaterol, Oxitropium und Formoterol
xcvi. Indacaterol, Oxitropium und Arformoterol
xcvii. Indacaterol, Oxitropium und Olodaterol
xcviii. Indacaterol, Oxitropium und Vilanterol
xcix. Indacaterol, Oxitropium und Carmoterol
c. Indacaterol, Oxitropium und Bambuterol
ci. Vilanterol, Tiotropium und Salmeterol
cii.Vilanterol, Tiotropium und Formoterol
ciii. Vilanterol, Tiotropium und Arformoterol
civ. Vilanterol, Tiotropium und Indacaterol
cv.Vilanterol, Tiotropium und Olodaterol
cvi. Vilanterol, Tiotropium und Carmoterol
cvii. Vilanterol, Tiotropium und Bambuterol
cviii. Vilanterol, Glycopyrronium und Salmeterol
cix. Vilanterol, Glycopyrronium und Formoterol
cx.Vilanterol, Glycopyrronium und Arformoterol
cxi. Vilanterol, Glycopyrronium und Indacaterol
cxii. Vilanterol, Glycopyrronium und Olodaterol
cxiii. Vilanterol, Glycopyrronium und Carmoterol
cxiv. Vilanterol, Glycopyrronium und Bambuterol
cxv. Vilanterol, Aclidinium und Salmeterol
cxvi. Vilanterol, Aclidinium und Formoterol
cxvii. Vilanterol, Aclidinium und Arformoterol
cxviii. Vilanterol, Aclidinium und Indacaterol
cxix. Vilanterol, Aclidinium und Olodaterol
cxx. Vilanterol, Aclidinium und Carmoterol
cxxi. Vilanterol, Aclidinium und Bambuterol
cxxii. Vilanterol, Oxitropium und Salmeterol
cxxiii. Vilanterol, Oxitropium und Formoterol
cxxiv. Vilanterol, Oxitropium und Arformoterol
cxxv. Vilanterol, Oxitropium und Indacaterol
cxxvi. Vilanterol, Oxitropium und Olodaterol
cxxvii. Vilanterol, Oxitropium und Carmoterol
cxxviii. Vilanterol, Oxitropium und Bambuterol
cxxix. Carmoterol, Tiotropium und Salmeterol
cxxx. Carmoterol, Tiotropium und Formoterol
cxxxi. Carmoterol, Tiotropium und Arformoterol
cxxxii. Carmoterol, Tiotropium und Indacaterol
cxxxiii. Carmoterol, Tiotropium und Olodaterol
cxxxiv. Carmoterol, Tiotropium und Vilanterol
cxxxv. Carmoterol, Tiotropium und Bambuterol
cxxxvi. Carmoterol, Glycopyrronium und Salmeterol
cxxxvii. Carmoterol, Glycopyrronium und Formoterol
cxxxviii. Carmoterol, Glycopyrronium und Arformoterol
cxxxix. Carmoterol, Glycopyrronium und Indacaterol
cxl. Carmoterol, Glycopyrronium und Olodaterol
cxli. Carmoterol, Glycopyrronium und Vilanterol
cxlii. Carmoterol, Glycopyrronium und Bambuterol
cxliii. Carmoterol, Aclidinium und Salmeterol
cxliv. Carmoterol, Aclidinium und Formoterol
cxlv. Carmoterol, Aclidinium und Arformoterol
cxlvi. Carmoterol, Aclidinium und Indacaterol
cxlvii. Carmoterol, Aclidinium und Olodaterol
cxlviii. Carmoterol, Aclidinium und Vilanterol
cxlix. Carmoterol, Aclidinium und Bambuterol
cl. Carmoterol, Oxitropium und Salmeterol
cli.Carmoterol, Oxitropium und Formoterol
clii. Carmoterol, Oxitropium und Arformoterol
cliii. Carmoterol, Oxitropium und Indacaterol
cliv. Carmoterol, Oxitropium und Olodaterol
clv. Carmoterol, Oxitropium und Vilanterol
clvi. Carmoterol, Oxitropium und Bambuterol
clvii. Olodaterol, Tiotropium und Salmeterol
clviii. Olodaterol, Tiotropium und Formoterol
clix. Olodaterol, Tiotropium und Arformoterol
clx. Olodaterol, Tiotropium und Indacaterol
clxi. Olodaterol, Tiotropium und Vilanterol
clxii. Olodaterol, Tiotropium und Bambuterol
clxiii. Olodaterol, Glycopyrronium und Salmeterol
clxiv. Olodaterol, Glycopyrronium und Formoterol
clxv. Olodaterol, Glycopyrronium und Arformoterol
clxvi. Olodaterol, Glycopyrronium und Indacaterol
clxvii. Olodaterol, Glycopyrronium und Vilanterol
clxviii. Olodaterol, Glycopyrronium und Bambuterol
clxix. Olodaterol, Aclidinium und Salmeterol
clxx. Olodaterol, Aclidinium und Formoterol
clxxi. Olodaterol, Aclidinium und Arformoterol
clxxii. Olodaterol, Aclidinium und Indacaterol
clxxiii. Olodaterol, Aclidinium und Vilanterol
clxxiv. Olodaterol, Aclidinium und Bambuterol
clxxv. Olodaterol, Oxitropium und Salmeterol
clxxvi. Olodaterol, Oxitropium und Formoterol
clxxvii. Olodaterol, Oxitropium und Arformoterol
clxxviii. Olodaterol, Oxitropium und Indacaterol
clxxix. Olodaterol, Oxitropium und Vilanterol
clxxx. Olodaterol, Oxitropium und Bambuterol
wobei die therapeutischen Mittel als ein pharmazeutisch akzeptables Salz oder Ester davon oder in enantiomer-reiner Form oder als eine razemische Mischung vorhanden sein können.

17. Pharmazeutische Zusammensetzung nach Anspruch 13, umfassend eine der folgenden Kombinationen, wobei die Kombinationen zur Verabreichung separat, sequentiell oder zusammen in wirksamen Mengen geeignet sind;
i. Aclidinum, Tiotropium und Salbutamol
ii. Aclidinum, Tiotropium und Levosalbutamol
iii. Aclidinum, Tiotropium und Terbutalin
iv. Aclidinum, Tiotropium und Pirbutarol
v. Aclidinum, Tiotropium und Procaterol
vi. Aclidinum, Tiotropium und Fenoterol
vii.Aclidinum, Tiotropium und Ritodrin
viii. Aclidinum, Tiotropium und Bitolterol
ix. Aclidinum, Tiotropium und Metaproterenol
x. Aclidinum, Glycopyrronium und Salbutamol
xi. Aclidinum, Glycopyrronium und Levosalbutamol
xii. Aclidinum, Glycopyrronium und Terbutalin
xiii. Aclidinum, Glycopyrronium und Pirbuterol
xiv. Aclidinum, Glycopyrronium und Procaterol
xv. Aclidinum, Glycopyrronium und Fenoterol
xvi. Aclidinum, Glycopyrronium und Bitolterol
xvii. Aclidinum, Glycopyrronium und Ritodrin
xviii. Aclidinum, Glycopyrronium und Metaproterenol
xix. Aclidinum, Ipratropium und Salbutamol
xx. Aclidinum, Ipratropium und Levosalbutamol
xxi. Aclidinum, Ipratropium und Terbutalin
xxii. Aclidinum, Ipratropium und Pirbuterol
xxiii. Aclidinum, Ipratropium und Procaterol
xxiv. Aclidinum, Ipratropium und Fenoterol
xxv. Aclidinum, Ipratropium und Bitolterol
xxvi. Aclidinum, Ipratropium und Ritodrin
xxvii. Aclidinum, Ipratropium und Metaproterenol
xxviii. Aclidinum, Oxitropium und Salbutamol
xxix. Aclidinum, Oxitropium und Levosalbutamol
xxx. Aclidinum, Oxitropium und Terbutalin
xxxi. Aclidinum, Oxitropium und Pirbuterol
xxxii. Aclidinum, Oxitropium und Procaterol
xxxiii. Aclidinum, Oxitropium und Fenoterol
xxxiv. Aclidinum, Oxitropium und Bitolterol
xxxv. Aclidinum, Oxitropium und Ritodrin
xxxvi. Aclidinum, Oxitropium und Metaproterenol
xxxvii. Glycopyrronium, Tiotropium und Salbutamol
xxxviii. Glycopyrronium, Tiotropium und Levosalbutamol
xxxix. Glycopyrronium, Tiotropium und Terbutalin
xl. Glycopyrronium, Tiotropium und Pirbuterol
xli.Glycopyrronium, Tiotropium und Procaterol
xlii. Glycopyrronium, Tiotropium und Fenoterol
xliii. Glycopyrronium, Tiotropium und Bitolterol
xliv. Glycopyrronium, Tiotropium und Ritodrin
xlv. Glycopyrronium, Tiotropium und Metaproterenol
xlvi. Glycopyrronium, Ipratropium und Salbutamol
xlvii. Glycopyrronium, Ipratropium und Levosalbutamol
xlviii. Glycopyrronium, Ipratropium und Terbutalin
xlix. Glycopyrronium, Ipratropium und Pirbuterol
l. Glycopyrronium, Ipratropium und Procaterol
li. Glycopyrronium, Ipratropium und Fenoterol
lii. Glycopyrronium, Ipratropium und Bitolterol
liii.Glycopyrronium, Ipratropium und Ritodrin
liv.Glycopyrronium, Ipratropium und Metaproterenol
lv. Glycopyrronium, Oxitropium und Salbutamol
lvi. Glycopyrronium, Oxitropium und Levosalbutamol
lvii. Glycopyrronium, Oxitropium und Terbutalin
lviii. Glycopyrronium, Oxitropium und Pirbuterol
lix. Glycopyrronium, Oxitropium und Procaterol
lx. Glycopyrronium, Oxitropium und Fenoterol
lxi. Glycopyrronium, Oxitropium und Bitolterol
lxii. Glycopyrronium, Oxitropium und Ritodrin
lxiii. Glycopyrronium, Oxitropium und Metaproterenol
lxiv. Daratropium, Tiotropium und Salbutamol
lxv. Daratropium, Tiotropium und Levosalbutamol
lxvi. Daratropium, Tiotropium und Terbutalin
lxvii. Daratropium, Tiotropium und Pirbuterol
lxviii. Daratropium, Tiotropium und Procaterol
lxix. Daratropium, Tiotropium und Fenoterol
lxx. Daratropium, Tiotropium und Bitolterol
lxxi. Daratropium, Tiotropium und Ritodrin
lxxii. Daratropium, Tiotropium und Metaproterenol
lxxiii. Daratropium, Aclidinium und Salbutamol
lxxiv. Daratropium, Aclidinium und Levosalbutamol
lxxv. Daratropium, Aclidinium und Terbutalin
lxxvi. Daratropium, Aclidinium und Pirbuterol
lxxvii. Daratropium, Aclidinium und Procaterol
lxxviii. Daratropium, Aclidinium und Fenoterol
lxxix. Daratropium, Aclidinium und Bitolterol
lxxx. Daratropium, Aclidinium und Ritodrin
lxxxi. Daratropium, Aclidinium und Metaproterenol
lxxxii. Daratropium, Glycopyrronium und Salbutamol
lxxxiii. Daratropium, Glycopyrronium und Levosalbutamol
lxxxiv. Daratropium, Glycopyrronium und Terbutalin
lxxxv. Daratropium, Glycopyrronium und Pirbuterol
lxxxvi. Daratropium, Glycopyrronium und Procaterol
lxxxvii. Daratropium, Glycopyrronium und Fenoterol
lxxxviii. Daratropium, Glycopyrronium und Bitolterol
lxxxix. Daratropium, Glycopyrronium und Ritodrin
xc.Daratropium, Glycopyrronium und Metaproterenol
xci. Daratropium, Ipratropium und Salbutamol
xcii. Daratropium, Ipratropium und Levosalbutamol
xciii. Daratropium, Ipratropium und Terbutalin
xciv. Daratropium, Ipratropium und Pirbuterol
xcv. Daratropium, Ipratropium und Procaterol
xcvi. Daratropium, Ipratropium und Fenoterol
xcvii. Daratropium, Ipratropium und Bitolterol
xcviii. Daratropium, Ipratropium und Ritodrin
xcix. Daratropium, Ipratropium und Metaproterenol
c. Daratropium, Oxitropium und Salbutamol
ci. Daratropium, Oxitropium und Levosalbutamol
cii. Daratropium, Oxitropium und Terbutalin
ciii. Daratropium, Oxitropium und Pirbuterol
civ. Daratropium, Oxitropium und Procaterol
cv.Daratropium, Oxitropium und Fenoterol
cvi. Daratropium, Oxitropium und Bitolterol
cvii. Daratropium, Oxitropium und Ritodrin
cviii. Daratropium, Oxitropium und Metaproterenol
cix. Indacaterol, Tirotropium und Salbutamol
cx.Indacaterol, Tirotropium und Levosalbutamol
cxi. Indacaterol, Tirotropium und Terbutalin
cxii. Indacaterol, Tirotropium und Pirbuterol
cxiii. Indacaterol, Tirotropium und Procaterol
cxiv. Indacaterol, Tirotropium und Fenoterol
cxv. Indacaterol, Tirotropium und Bitolterol
cxvi. Indacaterol, Tirotropium und Ritodrin
cxvii. Indacaterol, Tirotropium und Metaproterenol
cxviii. Indacaterol, Glycopyrronium und Salbutamol
cxix. Indacaterol, Glycopyrronium und Levosalbutamol
cxx. Indacaterol, Glycopyrronium und Terbutalin
cxxi. Indacaterol, Glycopyrronium und Pirbuterol
cxxii. Indacaterol, Glycopyrronium und Procaterol
cxxiii. Indacaterol, Glycopyrronium und Fenoterol
cxxiv. Indacaterol, Glycopyrronium und Bitolterol
cxxv. Indacaterol, Glycopyrronium und Ritodrin
cxxvi. Indacaterol, Glycopyrronium und Metaproterenol
cxxvii. Indacaterol, Aclidinium und Salbutamol
cxxviii. Indacaterol, Aclidinium und Levosalbutamol
cxxix. Indacaterol, Aclidinium und Terbutalin
cxxx. Indacaterol, Aclidinium und Pirbuterol
cxxxi. Indacaterol, Aclidinium und Procaterol
cxxxii. Indacaterol, Aclidinium und Fenoterol
cxxxiii. Indacaterol, Aclidinium und Bitolterol
cxxxiv. Indacaterol, Aclidinium und Ritodrin
cxxxv. Indacaterol, Aclidinium und Metaproterenol
cxxxvi. Indacaterol, Ipratropium und Salbutamol
cxxxvii. Indacaterol, Ipratropium und Levosalbutamol
cxxxviii. Indacaterol, Ipratropium und Terbutalin
cxxxix. Indacaterol, Ipratropium und Pirbuterol
cxl. Indacaterol, Ipratropium und Procaterol
cxli. Indacaterol, Ipratropium und Fenoterol
cxlii. Indacaterol, Ipratropium und Bitolterol
cxliii. Indacaterol, Ipratropium und Ritodrin
cxliv. Indacaterol, Ipratropium und Metaproterenol
cxlv. Indacaterol, Oxitropium und Salbutamol
cxlvi. Indacaterol, Oxitropium und Levosalbutamol
cxlvii. Indacaterol, Oxitropium und Terbutalin
cxlviii. Indacaterol, Oxitropium und Pirbuterol
cxlix. Indacaterol, Oxitropium und Procaterol
cl. Indacaterol, Oxitropium und Fenoterol
cli.Indacaterol, Oxitropium und Bitolterol
clii. Indacaterol, Oxitropium und Ritodrin
cliii. Indacaterol, Oxitropium und Metaproterenol
cliv. Vilanterol, Tiotropium und Salbutamol
clv. Vilanterol, Tiotropium und Levosalbutamol
clvi. Vilanterol, Tiotropium und Terbutalin
clvii. Vilanterol, Tiotropium und Pirbuterol
clviii. Vilanterol, Tiotropium und Procaterol
clix. Vilanterol, Tiotropium und Fenoterol
clx. Vilanterol, Tiotropium und Bitolterol
clxi. Vilanterol, Tiotropium und Ritodrin
clxii. Vilanterol, Tiotropium und Metaproterenol
clxiii. Vilanterol, Glycopyrronium und Salbutamol
clxiv. Vilanterol, Glycopyrronium und Levosalbutamol
clxv. Vilanterol, Glycopyrronium und Terbutalin
clxvi. Vilanterol, Glycopyrronium und Pirbuterol
clxvii. Vilanterol, Glycopyrronium und Procaterol
clxviii. Vilanterol, Glycopyrronium und Fenoterol
clxix. Vilanterol, Glycopyrronium und Bitolterol
clxx. Vilanterol, Glycopyrronium und Ritodrin
clxxi. Vilanterol, Glycopyrronium und Metaproterenol
clxxii. Vilanterol, Ipratropium und Salbutamol
clxxiii. Vilanterol, Ipratropium und Levosalbutamol
clxxiv. Vilanterol, Ipratropium und Terbutalin
clxxv. Vilanterol, Ipratropium und Pirbuterol
clxxvi. Vilanterol, Ipratropium und Procaterol
clxxvii. Vilanterol, Ipratropium und Fenoterol
clxxviii. Vilanterol, Ipratropium und Bitolterol
clxxix. Vilanterol, Ipratropium und Ritodrin
clxxx. Vilanterol, Ipratropium und Metaproterenol
clxxxi. Vilanterol, Aclidinium und Salbutamol
clxxxii. Vilanterol, Aclidinium und Levosalbutamol
clxxxiii. Vilanterol, Aclidinium und Terbutalin
clxxxiv. Vilanterol, Aclidinium und Pirbuterol
clxxxv. Vilanterol, Aclidinium und Procaterol
clxxxvi. Vilanterol, Aclidinium und Fenoterol
clxxxvii. Vilanterol, Aclidinium und Bitolterol
clxxxviii. Vilanterol, Aclidinium und Ritodrin
clxxxix. Vilanterol, Aclidinium und Metaproterenol
cxc. Vilanterol, Oxitropium und Salbutamol
cxci. Vilanterol, Oxitropium und Levosalbutamol
cxcii. Vilanterol, Oxitropium und Terbutalin
cxciii. Vilanterol, Oxitropium und Pirbuterol
cxciv. Vilanterol, Oxitropium und Procaterol
cxcv. Vilanterol, Oxitropium und Fenoterol
cxcvi. Vilanterol, Oxitropium und Bitolterol
cxcvii. Vilanterol, Oxitropium und Ritodrin
cxcviii. Vilanterol, Oxitropium und Metaproterenol
cxcix. Carmoterol, Tiotropium und Salbutamol
cc.Carmoterol, Tiotropium und Levosalbutamol
cci. Carmoterol, Tiotropium und Terbutalin
ccii. Carmoterol, Tiotropium und Pirbuterol
cciii. Carmoterol, Tiotropium und Procaterol
cciv. Carmoterol, Tiotropium und Fenoterol
ccv. Carmoterol, Tiotropium und Bitolterol
ccvi. Carmoterol, Tiotropium und Ritodrin
ccvii. Carmoterol, Tiotropium und Metaproterenol
ccviii. Carmoterol, Ipratropium und Levosalbutamol
ccix. Carmoterol, Ipratropium und Salbutamol
ccx. Carmoterol, Ipratropium und Terbutalin
ccxi. Carmoterol, Ipratropium und Pirbuterol
ccxii. Carmoterol, Ipratropium und Procaterol
ccxiii. Carmoterol, Ipratropium und Fenoterol
ccxiv. Carmoterol, Ipratropium und Bitolterol
ccxv. Carmoterol, Ipratropium und Ritodrin
ccxvi. Carmoterol, Ipratropium und Metaproterenol
ccxvii. Carmoterol, Aclidinum und Levosalbutamol
ccxviii. Carmoterol, Aclidinum und Salbutamol
ccxix. Carmoterol, Aclidinum und Terbutalin
ccxx. Carmoterol, Aclidinum und Pirbuterol
ccxxi. Carmoterol, Aclidinum und Procaterol
ccxxii. Carmoterol, Aclidinum und Fenoterol
ccxxiii. Carmoterol, Aclidinum und Bitolterol
ccxxiv. Carmoterol, Aclidinum und Ritodrin
ccxxv. Carmoterol, Aclidinum und Metaproterenol
ccxxvi. Carmoterol, Oxitropium und Salbutamol
ccxxvii. Carmoterol, Oxitropium und Levosalbutamol
ccxxviii. Carmoterol, Oxitropium und Terbutalin
ccxxix. Carmoterol, Oxitropium und Pirbuterol
ccxxx. Carmoterol, Oxitropium und Procaterol
ccxxxi. Carmoterol, Oxitropium und Fenoterol
ccxxxii. Carmoterol, Oxitropium und Bitolterol
ccxxxiii. Carmoterol, Oxitropium und Ritodrin
ccxxxiv. Carmoterol, Oxitropium und Metaproterenol
ccxxxv. Olodaterol, Tiotropium und Salbutamol
ccxxxvi. Olodaterol, Tiotropium und Levosalbutamol
ccxxxvii. Olodaterol, Tiotropium und Terbutalin
ccxxxviii. Olodaterol, Tiotropium und Pirbuterol
ccxxxix. Olodaterol, Tiotropium und Procaterol
ccxl. Olodaterol, Tiotropium und Fenoterol
ccxli. Olodaterol, Tiotropium und Bitolterol
ccxlii. Olodaterol, Tiotropium und Ritodrin
ccxliii. Olodaterol, Tiotropium und Metaproterenol
ccxliv. Olodaterol, Ipratropium und Salbutamol
ccxlv. Olodaterol, Ipratropium und Levosalbutamol
ccxlvi. Olodaterol, Ipratropium und Terbutalin
ccxlvii. Olodaterol, Ipratropium und Pirbuterol
ccxlviii. Olodaterol, Ipratropium und Procaterol
ccxlix. Olodaterol, Ipratropium und Fenoterol
ccl. Olodaterol, Ipratropium und Bitolterol
ccli. Olodaterol, Ipratropium und Ritodrin
cclii. Olodaterol, Ipratropium und Metaproterenol
ccliii. Olodaterol, Aclidinum und Salbutamol
ccliv. Olodaterol, Aclidinum und Levosalbutamol
cclv. Olodaterol, Aclidinum und Terbutalin
cclvi. Olodaterol, Aclidinum und Pirbuterol
cclvii. Olodaterol, Aclidinum und Procaterol
cclviii. Olodaterol, Aclidinum und Fenoterol
cclix. Olodaterol, Aclidinum und Bitolterol
cclx. Olodaterol, Aclidinum und Ritodrin
cclxi. Olodaterol, Aclidinum und Metaproterenol
cclxii. Olodaterol, Glycopyrronium und Salbutamol
cclxiii. Olodaterol, Glycopyrronium und Levosalbutamol
cclxiv. Olodaterol, Glycopyrronium und Terbutalin
cclxv. Olodaterol, Glycopyrronium und Pirbuterol
cclxvi. Olodaterol, Glycopyrronium und Procaterol
cclxvii. Olodaterol, Glycopyrronium und Fenoterol
cclxviii. Olodaterol, Glycopyrronium und Bitolterol
cclxix. Olodaterol, Glycopyrronium und Ritodrin
cclxx. Olodaterol, Glycopyrronium und Metaproterenol
cclxxi. Olodaterol, Oxitropium und Salbutamol
cclxxii. Olodaterol, Oxitropium und Levosalbutamol
cclxxiii. Olodaterol, Oxitropium und Terbutalin
cclxxiv. Olodaterol, Oxitropium und Pirbuterol
cclxxv. Olodaterol, Oxitropium und Procaterol
cclxxvi. Olodaterol, Oxitropium und Fenoterol
cclxxvii. Olodaterol, Oxitropium und Bitolterol
cclxxviii. Olodaterol, Oxitropium und Ritodrin
cclxxix. Olodaterol, Oxitropium und Metaproterenol
wobei die therapeutischen Mittel als ein pharmazeutisch akzeptables Salz oder Ester davon oder in enantiomer-reiner Form oder als eine razemische Mischung vorhanden sein können.

18. Pharmazeutische Zusammensetzung nach Anspruch 14, umfassend eine der folgenden Kombinationen, wobei die Kombinationen zur Verabreichung separat, sequentiell oder zusammen in wirksamen Mengen geeignet sind;
i. Aclidinum, Tiotropium und Fluticason
ii. Aclidinum, Tiotropium und Ciclesonid
iii. Aclidinum, Tiotropium und Budesonid
iv. Aclidinum, Tiotropium und Mometason
v. Aclidinum, Tiotropium und Beclomethason
vi. Aclidinum, Tiotropium und Triamcinolon
vii.Aclidinum, Tiotropium und Flunisolid
viii. Aclidinum, Tiotropium und Dexomethason
ix. Daratropium, Tiotropium und Fluticason
x. Daratropium, Tiotropium und Ciclesonid
xi. Daratropium, Tiotropium und Budesonid
xii.Daratropium, Tiotropium und Mometason
xiii. Daratropium, Tiotropium und Beclamethason
xiv. Daratropium, Tiotropium und Triamcinolon
xv. Daratropium, Tiotropium und Flunisolid
xvi. Daratropium, Tiotropium und Dexomethason
xvii. Indacaterol, Tiotropium und Fluticason
xviii. Indacaterol, Tiotropium und Budesonid
xix. Indacaterol, Tiotropium und Ciclesonid
xx. Indacaterol, Tiotropium und Mometason
xxi. Indacaterol, Tiotropium und Beclamethason
xxii. Indacaterol, Tiotropium und Triamcinolon
xxiii. Indacaterol, Tiotropium und Flunisolid
xxiv. Indacaterol, Tiotropium und Dexomethason
xxv. Vilanterol, Tiotropium und Ciclesonid
xxvi. Vilanterol, Tiotropium und Fluticason
xxvii. Vilanterol, Tiotropium und Budesonid
xxviii. Vilanterol, Tiotropium und Mometason
xxix. Vilanterol, Tiotropium und Beclamethason
xxx. Vilanterol, Tiotropium und Triamcinolon
xxxi. Vilanterol, Tiotropium und Flunisolid
xxxii. Vilanterol, Tiotropium und Dexomethason
xxxiii. Carmoterol, Tiotropium und Budesonid
xxxiv. Carmoterol, Tiotropium und Ciclesonid
xxxv. Carmoterol, Tiotropium und Fluticason
xxxvi. Carmoterol, Tiotropium und Mometason
xxxvii. Carmoterol, Tiotropium und Beclamethason
xxxviii. Carmoterol, Tiotropium und Triamcinolon
xxxix. Carmoterol, Tiotropium und Flunisolid
xl. Carmoterol, Tiotropium und Dexomethason
xli.Olodaterol, Tiotropium und Ciclesonid
xlii. Olodaterol, Tiotropium und Fluticason
xliii. Olodaterol, Tiotropium und Budesonid
xliv. Olodaterol, Tiotropium und Mometason
xlv. Olodaterol, Tiotropium und Beclamethason
xlvi. Olodaterol, Tiotropium und Triamcinolon
xlvii. Olodaterol, Tiotropium und Flunisolid
xlviii. Olodaterol, Tiotropium und Dexomethason
wobei die therapeutischen Mittel als ein pharmazeutisch akzeptables Salz oder Ester davon oder in enantiomer-reiner Form oder als eine razemische Mischung vorhanden sein können.

19. Pharmazeutische Zusammensetzung nach Anspruch 15, umfassend eine der folgenden Kombinationen, wobei die Kombinationen zur Verabreichung separat, sequentiell oder zusammen in wirksamen Mengen geeignet sind;
i. Aclidinium, Salmeterol und Salbutamol
ii. Aclidinium, Salmeterol und Levosalbutamol
iii. Aclidinium, Formoterol und Salbutamol
iv. Aclidinium, Formoterol und Levosalbutamol
v. Aclidinium, Arformoterol und Salbutamol
vi. Aclidinium, Arformoterol und Levosalbutamol
vii.Aclidinium, Indacaterol und Salbutamol
viii. Aclidinium, Indacaterol und Levosalbutamol
ix. Aclidinium, Olodaterol und Salbutamol
x. Aclidinium, Olodaterol und Levosalbutamol
xi. Aclidinium, Vilanterol und Salbutamol
xii.Aclidinium, Vilanterol und Levosalbutamol
xiii. Aclidinium, Carmoterol und Salbutamol
xiv. Aclidinium, Carmoterol und Levosalbutamol
xv. Aclidinium, Bambuterol und Salbutamol
xvi. Aclidinium, Bambuterol und Levosalbutamol
xvii. Glycopyrronium, Indacaterol und Salbutamol
xviii. Glycopyrronium, Indacaterol und Levosalbutamol
xix. Glycopyrronium, Salmeterol und Salbutamol
xx. Glycopyrronium, Salmeterol und Levosalbutamol
xxi. Glycopyrronium, Formoterol und Salbutamol
xxii. Glycopyrronium, Formoterol und Levosalbutamol
xxiii. Glycopyrronium, Arformoterol und Salbutamol
xxiv. Glycopyrronium, Arformoterol und Levosalbutamol
xxv. Glycopyrronium, Carmoterol und Salbutamol
xxvi. Glycopyrronium, Carmoterol und Levosalbutamol
xxvii. Glycopyrronium, Olodaterol und Salbutamol
xxviii. Glycopyrronium, Olodaterol und Levosalbutamol
xxix. Glycopyrronium, Vilanterol und Salbutamol
xxx. Glycopyrronium, Vilanterol und Levosalbutamol
xxxi. Glycopyrronium, Bambuterol und Salbutamol
xxxii. Glycopyrronium, Bambuterol und Levosalbutamol xxxiii. Daratropium, Indacaterol und Salbutamol
xxxiv. Daratropium, Indacaterol und Levosalbutamol
xxxv. Daratropium, Salmeterol und Salbutamol
xxxvi. Daratropium, Salmeterol und Levosalbutamol
xxxvii. Daratropium, Formoterol und Salbutamol
xxxviii. Daratropium, Formoterol und Levosalbutamol
xxxix. Daratropium, Carmoterol und Salbutamol
xl. Daratropium, Carmoterol und Levosalbutamol
xli.Daratropium, Olodaterol und Salbutamol
xlii. Daratropium, Olodaterol und Levosalbutamol
xliii. Daratropium, Vilanterol und Salbutamol
xliv. Daratropium, Vilanterol und Levosalbutamol
xlv. Daratropium, Bambuterol und Salbutamol
xlvi. Daratropium, Bambuterol und Levosalbutamol
xlvii. Daratropium, Arformoterol und Salbutamol
xlviii. Daratropium, Arformoterol und Levosalbutamol
wobei die therapeutischen Mittel als ein pharmazeutisch akzeptables Salz oder Ester davon oder in enantiomer-reiner Form oder als eine razemische Mischung vorhanden sein können.

20. Pharmazeutische Zusammensetzung nach Anspruch 16, umfassend eine der folgenden Kombinationen, wobei die Kombinationen zur Verabreichung separat, sequentiell oder zusammen in wirksamen Mengen geeignet sind;
i. Aclidinium, Salmeterol und Mometason
ii. Aclidinium, Salmeterol und Fluticason
iii. Aclidinium, Salmeterol und Budesonid
iv. Aclidinium, Formoterol und Mometason
v. Aclidinium, Formoterol und Fluticason
vi. Aclidinium, Formoterol und Budesonid
vii.Aclidinium, Arformoterol und Mometason
viii. Aclidinium, Arformoterol und Fluticason
ix. Aclidinium, Arformoterol und Budesonid
x. Aclidinium, Indacaterol und Mometason
xi. Aclidinium, Indacaterol und Fluticason
xii.Aclidinium, Indacaterol und Budesonid
xiii. Aclidinium, Olodaterol und Mometason
xiv. Aclidinium, Olodaterol und Fluticason
xv. Aclidinium, Olodaterol und Budesonid
xvi. Aclidinium, Vilanterol und Mometason
xvii. Aclidinium, Vilanterol und Fluticason
xviii. Aclidinium, Vilanterol und Budesonid
xix. Aclidinium, Carmoterol und Mometason
xx. Aclidinium, Carmoterol und Fluticason
xxi. Aclidinium, Carmoterol und Budesonid
xxii. Aclidinium, Bambuterol und Mometason
xxiii. Aclidinium, Bambuterol und Fluticason
xxiv. Aclidinium, Bambuterol und Budesonid
xxv. Glycopyrronium, Indacaterol und Mometason
xxvi. Glycopyrronium, Indacaterol und Fluticason
xxvii. Glycopyrronium, Indacaterol und Budesonid
xxviii. Glycopyrronium, Salmeterol und Mometason
xxix. Glycopyrronium, Salmeterol und Fluticason
xxx. Glycopyrronium, Salmeterol und Budesonid
xxxi. Glycopyrronium, Formoterol und Mometason
xxxii. Glycopyrronium, Formoterol und Fluticason
xxxiii. Glycopyrronium, Formoterol und Budesonid
xxxiv. Glycopyrronium, Arformoterol und Mometason
xxxv. Glycopyrronium, Arformoterol und Fluticason
xxxvi. Glycopyrronium, Arformoterol und Budesonid
xxxvii. Glycopyrronium, Carmoterol und Mometason
xxxviii. Glycopyrronium, Carmoterol und Fluticason
xxxix. Glycopyrronium, Carmoterol und Budesonid
xl. Glycopyrronium, Olodaterol und Mometason
xli.Glycopyrronium, Olodaterol und Fluticason
xlii. Glycopyrronium, Olodaterol und Budesonid
xliii. Glycopyrronium, Vilanterol und Mometason
xliv. Glycopyrronium, Vilanterol und Fluticason
xlv. Glycopyrronium, Vilanterol und Budesonid
xlvi. Glycopyrronium, Bambuterol und Mometason
xlvii. Glycopyrronium, Bambuterol und Fluticason
xlviii. Glycopyrronium, Bambuterol und Budesonid
xlix. Daratropium, Indacaterol und Mometason
l. Daratropium, Indacaterol und Fluticason
li. Daratropium, Indacaterol und Budesonid
lii. Daratropium, Salmeterol und Mometason
liii. Daratropium, Salmeterol und Fluticason
liv.Daratropium, Salmeterol und Budesonid
lv. Daratropium, Formoterol und Mometason
lvi. Daratropium, Formoterol und Fluticason
lvii. Daratropium, Formoterol und Budesonid
lviii. Daratropium, Carmoterol und Mometason
lix. Daratropium, Carmoterol und Fluticason
lx. Daratropium, Carmoterol und Budesonid
lxi. Daratropium, Olodaterol und Mometason
lxii. Daratropium, Olodaterol und Fluticason
lxiii. Daratropium, Olodaterol und Budesonid
lxiv. Daratropium, Vilanterol und Mometason
lxv. Daratropium, Vilanterol und Fluticason
lxvi. Daratropium, Vilanterol und Budesonid
lxvii. Daratropium, Bambuterol und Mometason
lxviii. Daratropium, Bambuterol und Fluticason
lxix. Daratropium, Bambuterol und Budesonid
lxx. Daratropium, Arformoterol und Mometason
lxxi. Daratropium, Arformoterol und Fluticason
lxxii. Daratropium, Arformoterol und Budesonid
lxxiii. Indacaterol, Salmeterol und Mometason
lxxiv. Indacaterol, Salmeterol und Fluticason
lxxv. Indacaterol, Salmeterol und Budesonid
lxxvi. Indacaterol, Formoterol und Mometason
lxxvii. Indacaterol, Formoterol und Fluticason
lxxviii. Indacaterol, Formoterol und Budesonid
lxxix. Indacaterol, Arformoterol und Mometason
lxxx. Indacaterol, Arformoterol und Fluticason
lxxxi. Indacaterol, Arformoterol und Budesonid
lxxxii. Indacaterol, Olodaterol und Mometason
lxxxiii. Indacaterol, Olodaterol und Fluticason
lxxxiv. Indacaterol, Olodaterol und Budesonid
lxxxv. Indacaterol, Vilanterol und Mometason
lxxxvi. Indacaterol, Vilanterol und Fluticason
lxxxvii. Indacaterol, Vilanterol und Budesonid
lxxxviii. Indacaterol, Carmeterol und Mometason
lxxxix. Indacaterol, Carmeterol und Fluticason
xc.Indacaterol, Carmeterol und Budesonid
xci. Indacaterol, Bambuterol und Mometason
xcii. Indacaterol, Bambuterol und Fluticason
xciii. Indacaterol, Bambuterol und Budesonid
xciv. Vilanterol, Salmeterol und Mometason
xcv. Vilanterol, Salmeterol und Fluticason
xcvi. Vilanterol, Salmeterol und Budesonid
xcvii. Vilanterol, Formoterol und Mometason
xcviii. Vilanterol, Formoterol und Fluticason
xcix. Vilanterol, Formoterol und Budesonid
c. Vilanterol, Arformoterol und Mometason
ci. Vilanterol, Arformoterol und Fluticason
cii.Vilanterol, Arformoterol und Budesonid
ciii. Vilanterol, Olodaterol und Mometason
civ. Vilanterol, Olodaterol und Fluticason
cv.Vilanterol, Olodaterol und Budesonid
cvi. Vilanterol, Carmeterol und Mometason
cvii. Vilanterol, Carmeterol und Fluticason
cviii. Vilanterol, Carmeterol und Budesonid
cix. Vilanterol, Bambuterol und Mometason
cx.Vilanterol, Bambuterol und Fluticason
cxi. Vilanterol, Bambuterol und Budesonid
cxii. Vilanterol, Indacaterol und Mometason
cxiii. Vilanterol, Indacaterol und Fluticason
cxiv. Vilanterol, Indacaterol und Budesonid
cxv. Carmeterol, Salmeterol und Mometason
cxvi. Carmeterol, Salmeterol und Fluticason
cxvii. Carmeterol, Salmeterol und Budesonid
cxviii. Carmeterol, Formoterol und Mometason
cxix. Carmeterol, Formoterol und Fluticason
cxx. Carmeterol, Formoterol und Budesonid
cxxi. Carmeterol, Arformoterol und Mometason
cxxii. Carmeterol, Arformoterol und Fluticason
cxxiii. Carmeterol, Arformoterol und Budesonid
cxxiv. Carmeterol, Indaceterol und Mometason
cxxv. Carmeterol, Indaceterol und Fluticason
cxxvi. Carmeterol, Indaceterol und Budesonid
cxxvii. Carmeterol, Olodaterol und Mometason
cxxviii. Carmeterol, Olodaterol und Fluticason
cxxix. Carmeterol, Olodaterol und Budesonid
cxxx. Carmeterol, Vilanterol und Mometason
cxxxi. Carmeterol, Vilanterol und Fluticason
cxxxii. Carmeterol, Vilanterol und Budesonid
cxxxiii. Carmeterol, Bambuterol und Mometason
cxxxiv. Carmeterol, Bambuterol und Fluticason
cxxxv. Carmeterol, Bambuterol und Budesonid
cxxxvi. Olodaterol, Salmeterol und Mometason
cxxxvii. Olodaterol, Salmeterol und Fluticason
cxxxviii. Olodaterol, Salmeterol und Budesonid
cxxxix. Olodaterol, Formoterol und Mometason
cxl. Olodaterol, Formoterol und Fluticason
cxli. Olodaterol, Formoterol und Budesonid
cxlii. Olodaterol, Arformoterol und Mometason
cxliii. Olodaterol, Arformoterol und Fluticason
cxliv. Olodaterol, Arformoterol und Budesonid
cxlv. Olodaterol, Indacaterol und Mometason
cxlvi. Olodaterol, Indacaterol und Fluticason
cxlvii. Olodaterol, Indacaterol und Budesonid
cxlviii. Olodaterol, Vilanterol und Mometason
cxlix. Olodaterol, Vilanterol und Fluticason
cl. Olodaterol, Vilanterol und Budesonid
cli.Olodaterol, Carmeterol und Mometason
clii. Olodaterol, Carmeterol und Fluticason
cliii. Olodaterol, Carmeterol und Budesonid
cliv. Olodaterol, Bambuterol und Mometason
clv. Olodaterol, Bambuterol und Fluticason
clvi. Olodaterol, Bambuterol und Budesonid
wobei die therapeutischen Mittel als ein pharmazeutisch akzeptables Salz oder Ester davon oder in enantiomer-reiner Form oder als eine razemische Mischung vorhanden sein können.

21. Pharmazeutische Zusammensetzung nach Anspruch 17, umfassend eine der folgenden Kombinationen, wobei die Kombinationen zur Verabreichung separat, sequentiell oder zusammen in wirksamen Mengen geeignet sind;
i. Aclidinium, Salbutamol und Fluticason
ii. Aclidinium, Levosalbutamol und Fluticason
iii. Aclidinium, Salbutamol und Ciclesonid
iv. Aclidinium, Levosalbutamol und Ciclesonid
v. Aclidinium, Salbutamol und Budesonid
vi. Aclidinium, Levosalbutamol und Budesonid
vii.Aclidinium, Salbutamol und Mometason
viii. Aclidinium, Levosalbutamol und Mometason
ix. Aclidinium, Salbutamol und Beclomethason
x. Aclidinium, Levosalbutamol und Beclomethason
xi. Aclidinium, Salbutamol und Triamcinolon
xii.Aclidinium, Levosalbutamol und Triamcinolon
xiii. Aclidinium, Salbutamol und Flunisolid
xiv. Aclidinium, Levosalbutamol und Flunisolid
xv. Aclidinium, Salbutamol und Dexamethason
xvi. Aclidinium, Levosalbutamol und Dexamethason
xvii. Glycopyrronium, Salbutamol und Fluticason
xviii. Glycopyrronium, Levosalbutamol und Fluticason
xix. Glycopyrronium, Salbutamol und Ciclesonid
xx. Glycopyrronium, Levosalbutamol und Ciclesonid
xxi. Glycopyrronium, Salbutamol und Budesonid
xxii. Glycopyrronium, Levosalbutamol und Budesonid
xxiii. Glycopyrronium, Salbutamol und Mometason
xxiv. Glycopyrronium, Levosalbutamol und Mometason
xxv. Glycopyrronium, Salbutamol und Beclomethason
xxvi. Glycopyrronium, Levosalbutamol und Beclomethason
xxvii. Glycopyrronium, Salbutamol und Triamcinolon
xxviii. Glycopyrronium, Levosalbutamol und Triamcinolon
xxix. Glycopyrronium, Salbutamol und Flunisolid
xxx. Glycopyrronium, Levosalbutamol und Flunisolid
xxxi. Glycopyrronium, Salbutamol und Dexamethason
xxxii. Glycopyrronium, Levosalbutamol und Dexamethason
xxxiii. Daratropium, Salbutamol und Fluticason
xxxiv. Daratropium, Levosalbutamol und Fluticason
xxxv. Daratropium, Salbutamol und Ciclesonid
xxxvi. Daratropium, Levosalbutamol und Ciclesonid
xxxvii. Daratropium, Salbutamol und Budesonid
xxxviii. Daratropium, Levosalbutamol und Budesonid
xxxix. Daratropium, Salbutamol und Mometason
xl. Daratropium, Levosalbutamol und Mometason
xli.Daratropium, Salbutamol und Beclomethason
xlii. Daratropium, Levosalbutamol und Beclomethason
xliii. Daratropium, Salbutamol und Triamcinolon
xliv. Daratropium, Levosalbutamol und Triamcinolon
xlv. Daratropium, Salbutamol und Flunisolid
xlvi. Daratropium, Levosalbutamol und Flunisolid
xlvii. Daratropium, Salbutamol und Dexamethason
xlviii. Daratropium, Levosalbutamol und Dexamethason
xlix. Indacaterol, Salbutamol und Fluticason
l. Indacaterol, Levosalbutamol und Fluticason
li. Indacaterol, Salbutamol und Ciclesonid
lii. Indacaterol, Levosalbutamol und Ciclesonid
liii.Indacaterol, Salbutamol und Budesonid
liv.Indacaterol, Levosalbutamol und Budesonid
lv. Indacaterol, Salbutamol und Mometason
lvi.Indacaterol, Levosalbutamol und Mometason
lvii. Indacaterol, Salbutamol und Beclomethason
lviii. Indacaterol, Levosalbutamol und Beclomethason
lix.Indacaterol, Salbutamol und Triamcinolon
lx. Indacaterol, Levosalbutamol und Triamcinolon
lxi.Indacaterol, Salbutamol und Flunisolid
lxii. Indacaterol, Levosalbutamol und Flunisolid
lxiii. Indacaterol, Salbutamol und Dexamethason
lxiv. Indacaterol, Levosalbutamol und Dexamethason
lxv. Vilanterol, Salbutamol und Fluticason
lxvi. Vilanterol, Levosalbutamol und Fluticason
lxvii. Vilanterol, Salbutamol und Budesonid
lxviii. Vilanterol, Levosalbutamol und Budesonid
lxix. Vilanterol, Salbutamol und Ciclesonid
lxx. Vilanterol, Levosalbutamol und Ciclesonid
lxxi. Vilanterol, Salbutamol und Mometason
lxxii. Vilanterol, Levosalbutamol und Mometason
lxxiii. Vilanterol, Salbutamol und Beclomethason
lxxiv. Vilanterol, Levosalbutamol und Beclomethason
lxxv. Vilanterol, Salbutamol und Triamcinolon
lxxvi. Vilanterol, Levosalbutamol und Triamcinolon
lxxvii. Vilanterol, Salbutamol und Flunisolid
lxxviii. Vilanterol, Levosalbutamol und Flunisolid
lxxix. Vilanterol, Salbutamol und Dexamethason
lxxx. Vilanterol, Levosalbutamol und Dexamethason
lxxxi. Carmeterol, Salbutamol und Fluticason
lxxxii. Carmeterol, Levosalbutamol und Fluticason
lxxxiii. Carmeterol, Salbutamol und Ciclesonid
lxxxiv. Carmeterol, Levosalbutamol und Ciclesonid
lxxxv. Carmeterol, Salbutamol und Budesonid
lxxxvi. Carmeterol, Levosalbutamol und Budesonid
lxxxvii. Carmeterol, Salbutamol und Mometason
lxxxviii. Carmeterol, Levosalbutamol und Mometason
lxxxix. Carmeterol, Salbutamol und Beclomethason
xc.Carmeterol, Levosalbutamol und Beclomethason
xci. Carmeterol, Salbutamol und Triamcinolon
xcii. Carmeterol, Levosalbutamol und Triamcinolon
xciii. Carmeterol, Salbutamol und Flunisolid
xciv. Carmeterol, Levosalbutamol und Flunisolid
xcv. Carmeterol, Salbutamol und Dexamethason
xcvi. Carmeterol, Levosalbutamol und Dexamethason
xcvii. Olodaterol, Salbutamol und Fluticason
xcviii. Olodaterol, Levosalbutamol und Fluticason
xcix. Olodaterol, Salbutamol und Ciclesonid
c. Olodaterol, Levosalbutamol und Ciclesonid
ci. Olodaterol, Salbutamol und Budesonid
cii.Olodaterol, Levosalbutamol und Budesonid
ciii. Olodaterol, Salbutamol und Mometason
civ. Olodaterol, Levosalbutamol und Mometason
cv.Olodaterol, Salbutamol und Beclomethason
cvi. Olodaterol, Levosalbutamol und Beclomethason
cvii. Olodaterol, Salbutamol und Triamcinolon
cviii. Olodaterol, Levosalbutamol und Triamcinolon
cix. Olodaterol, Salbutamol und Flunisolid
cx.Olodaterol, Levosalbutamol und Flunisolid
cxi. Olodaterol, Salbutamol und Dexamethason
cxii. Olodaterol, Levosalbutamol und Dexamethason
wobei die therapeutischen Mittel als ein pharmazeutisch akzeptables Salz oder Ester davon oder in enantiomer-reiner Form oder als eine razemische Mischung vorhanden sein können.

22. Pharmazeutische Zusammensetzung nach den Ansprüchen 12 bis 17, umfassend eine der folgenden Kombinationen, wobei die Kombinationen zur Verabreichung separat, sequentiell oder zusammen in wirksamen Mengen geeignet sind;
i. Formoterol, Budesonid und Tiotropium
ii. Salmeterol, Fluticason und Tiotropium
iii. Carmoterol, Tiotropium und Fluticason
iv. Salbutamol, Formoterol und Budesonid
v. Salbutamol, Salmeterol und Fluticason
vi. Salbutamol, Arformoterol und Fluticason
wobei die therapeutischen Mittel als ein pharmazeutisch akzeptables Salz oder Ester davon oder in enantiomer-reiner Form oder als eine razemische Mischung vorhanden sein können.

23. Pharmazeutische Zusammensetzung nach einem der vorangehenden Ansprüche, wobei die therapeutisch wirksame Menge der pharmazeutischen Zusammensetzung einmal pro Tag verabreicht wird, oder die pharmazeutische Zusammensetzung zweimal pro Tag verabreicht wird.

24. Pharmazeutische Zusammensetzung nach einem der vorangehenden Ansprüche, zur Verwendung bei der Behandlung einer Atemwegserkrankung, ausgewählt aus Asthma und chronisch obstruktiver Lungenerkrankung und anderen obstruktiven Atemwegserkrankungen.

25. Pharmazeutische Zusammensetzung nach einem der vorangehenden Ansprüche, wobei die pharmazeutische Zusammensetzung zur Verabreichung separat, sequentiell oder zusammen in wirksamen Mengen geeignet ist, zusammen mit einem feuchtigkeitsdichten und Hochbarriere-versiegelten Blister.

26. Pharmazeutische Zusammensetzung nach einem der vorangehenden Ansprüche, wobei die pharmazeutische Zusammensetzung zur Verabreichung separat, sequentiell oder zusammen in wirksamen Mengen geeignet ist, zusammen mit einer Kapsel.

27. Pharmazeutische Zusammensetzung nach Anspruch 25 oder 26, wobei die pharmazeutische Zusammensetzung zur Verabreichung separat, sequentiell oder zusammen in wirksamen Mengen geeignet ist, zusammen mit einer Trockenpulverinhalationsvorrichtung.

28. Pharmazeutische Zusammensetzung nach Anspruch 25, wobei die pharmazeutische Zusammensetzung zur Verabreichung separat, sequentiell oder zusammen in wirksamen Mengen geeignet ist, zusammen mit einer Inhalationsvorrichtung, **dadurch gekennzeichnet, dass** die Vorrichtung wenigstens einen Schließmechanismus umfasst, der es der Vorrichtung ermöglicht, in beiden Positionen, in denen die Vorrichtung zur Inhalation bereit ist und in der der Deckel in der geschlossenen Position ist, verschlossen zu bleiben, und der es der Vorrichtung weiterhin ermöglicht, sich automatisch aufzusetzen, wenn der Deckel geschlossen ist.

29. Pharmazeutisches Kit, umfassend die Arzneien mit Amin, wie in Anspruch 1 definiert, und ein oder mehrere zusätzliche aktive Mittel, wie in Anspruch 7 definiert, in der Form eines trockenen Pulvers in Beimischung mit einem pharmazeutisch akzeptablen Träger, der nicht Laktose ist, wie in Anspruch 1 definiert, in separaten Einheitsdosierungsformen, wobei die Formen zur Verabreichung separat, sequentiell oder zusammen in wirksamen Mengen geeignet sind, zusammen mit einer oder mehreren Inhalationsvorrichtungen zur Verabreichung von Arzneien mit Amin und einem oder mehreren zusätzlichen aktiven Mitteln.

## Revendications

1. Composition pharmaceutique pour inhalation administrée séparément, séquentiellement ou conjointement, comprenant des médicaments ayant une amine sous la forme d'une poudre sèche qui sont choisis dans le groupe constitué d'aclidinium, glycopyrronium, daratropium, indacatérol, vilantérol, carmotérol et olodatérol ou leurs sels ou esters pharmaceutiquement acceptables, ou sous une forme énantiomériquement pure ou en mélange racémique en mélange avec un véhicule pharmaceutiquement acceptable autre que le lactose, ledit véhicule pharmaceutiquement acceptable comprend des particules fines et grossières, dans laquelle ledit véhicule pharmaceutiquement acceptable est choisi dans le groupe constitué de mannitol, glucose, tréhalose, cellobiose, sorbitol et maltitol ou une combinaison de deux ou plus de ceux-ci, et le rapport entre les particules fines et les particules grossières se situe entre 0,01 et 0,25 en poids.

2. Composition pharmaceutique selon la revendication 1, dans laquelle les particules fines dudit véhicule pharmaceutiquement acceptable ont un diamètre de particules de d₁₀ entre 1,0 et 4,0 µm, d₅ₒ entre 4,0 et 7,0 µm et d₉₀ entre 7,0 et 15,0 µm.

3. Composition pharmaceutique selon la revendication 1, dans laquelle les particules grossières dudit véhicule pharmaceutiquement acceptable ont un diamètre de particules de d₁₀ entre 10 et 50 µm, d₅₀ entre 50 et 75 µm et d₉₀ entre 75 et 250 µm.

4. Composition pharmaceutique selon une revendication 1 à 3, dans laquelle le véhicule pharmaceutiquement acceptable est le mannitol.

5. Composition pharmaceutique selon la revendication 4, dans laquelle le véhicule pharmaceutiquement acceptable est le mannitol séché pulvérisé.

6. Composition pharmaceutique selon la revendication 1, dans laquelle le diamètre moyen de particule (d₅₀) des médicaments ayant une amine se situe entre 1,5 et 2,5 µm.

7. Composition pharmaceutique selon la revendication 1, comprenant en outre un ou plusieurs agents actifs supplémentaires choisis parmi les antagonistes muscariniques à longue durée d'action, les agonistes bêta à longue durée d'action, les agonistes bêta 2 à courte durée d'action, les corticoïdes ou une combinaison de deux ou plus de ceux-ci en mélange avec un véhicule pharmaceutiquement acceptable autre que le lactose tel que défini dans la revendication 1.

8. Composition pharmaceutique selon la revendication 7, dans laquelle les antagonistes muscariniques à longue durée d'action sont choisis dans le groupe comprenant le tiotropium, le glycopyrronium, l'ipratropium, l'aclidinium, l'oxytropium ou un sel ou ester pharmaceutiquement acceptable de ceux-ci, ou sous forme énantomériquement pure ou en mélange racémique ou une combinaison de deux ou plus de ceux-ci.

9. Composition pharmaceutique selon la revendication 7, dans laquelle les agonistes bêta à longue durée d'action sont choisis dans le groupe comprenant le salmétérol, le formotérol, l'arformotérol, l'indacatérol, l'olodatérol, le vilantérol, le carmotérol, le bambutérol ou un sel ou ester pharmaceutiquement acceptable de ceux-ci, ou sous forme énantomériquement pure ou en mélange racémique ou une combinaison de deux ou plus de ceux-ci.

10. Composition pharmaceutique selon la revendication 7, dans laquelle les agonistes bêta 2 à courte durée d'action sont choisis dans le groupe comprenant le salbutamol, le lévosalbutamol, la terbutaline, le pirbutérol, le procatérol, le fénotérol, le bitoltérol, la ritodrine, le métaprotérénol ou un sel ou ester pharmaceutiquement acceptable de ceux-ci, ou sous forme énantomériquement pure ou en mélange racémique ou une combinaison de deux ou plus de ceux-ci.

11. Composition pharmaceutique selon la revendication 7, dans laquelle les corticoïdes sont choisis dans le groupe comprenant la fluticasone, le ciclésonide, le budésonide, la mométasone, la béclométhasone, la triamcinolone, le flunisolide, la dexaméthasone ou un sel ou ester pharmaceutiquement acceptable de ceux-ci, ou sous forme énantomériquement pure ou en mélange racémique ou une combinaison de deux ou plus de ceux-ci.

12. Composition pharmaceutique selon la revendication 8, comprenant l'une quelconque des combinaisons suivantes, lesdites combinaisons étant appropriées à une administration séparée, séquentielle ou conjointe en quantités efficaces :
i. aclinidium et tiotropium
ii. aclinidium et glycopyrronium
iii. aclinidium et ipratropium
iv. aclinidium et oxytropium
v. glycopyrronium et tiotropium
vi. glycopyrronium et ipratropium
vii. glycopyrronium et oxytropium
viii. darotropium et tiotropium
ix. darotropium et glycopyrronium
x. darotropium et ipratropium
xi. darotropium et aclidinium
xii. darotropium et oxytropium
xiii. indacatérol et tiotropium
xiv. indacatérol et glycopyrronium
xv. indacatérol et ipratropium
xvi. indacatérol et aclidinium
xvii. indacatérol et oxytropium
xviii. vilantérol et triotropium
xix. vilantérol et glycopyrronium
xx. vilantérol et ipratropium
xxi. vilantérol et aclidinium
xxii. vilantérol et oxytropium
xxiii. carmotérol et triotropium
xxiv. carmotérol et glycopyrronium
xxv. carmotérol et ipratropium
xxvi. carmotérol et aclidinium
xxvii. carmotérol et oxytropium
xxviii. carmotérol et triotropium
xxix. Olodatérol et ipratropium
xxx. Olodatérol et glycopyrronium
xxxi. Olodatérol et aclidinium
xxxii. Olodatérol et oxytropium
dans laquelle lesdits agents thérapeutiques peuvent être présents en tant que sels ou esters pharmaceutiquement acceptables de ceux-ci ou sous une forme énantiomériquement pure ou en mélange racémique.

13. Composition pharmaceutique selon la revendication 9, comprenant l'une quelconque des combinaisons suivantes, lesdites combinaisons étant appropriées à une administration séparée, séquentielle ou conjointe en quantités efficaces :
i. aclinidium et salmétérol
ii. aclinidium et formotérol
iii. aclinidium et arformotérol
iv. aclinidium et indacatérol
v. aclinidium et olodatérol
vi. aclinidium et vilantérol
vii. aclinidium et carmotérol
viii. aclinidium et bambutérol
ix. glycopyrronium et samétérol
x. glycopyrronium et formotérol
xi. glycopyrronium et arformotérol
xii. glycopyrronium et indacatérol
xiii. glycopyrronium et olodatérol
xiv. glycopyrronium et vilantérol
xv. glycopyrronium et carmotérol
xvi. glycopyrronium et bambutérol
xvii. darotropium et salmétérol
xviii. darotropium et formotérol
xix. darotropiumet et arformotérol
xx. darotropium et indacatérol
xxi. darotropium et olodatérol
xxii. darotropium et vilantérol
xxiii. darotropium et carmotérol
xxiv. darotropium et bambutérol
xxv. indacatérol et salmétérol
xxvi. indacatérol et formotérol
xxvii. indacatérol et arformotérol
xxviii. indacatérol et olodatérol
xxix. indacatérol et vilantérol
xxx. indacatérol et carmotérol
xxxi. indacatérol et bambutérol
xxxii. vilantérol et salmétérol
xxxiii. vilantérol et formotérol
xxxiv. vilantérol et arformotérol
xxxv. vilantérol et olodatérol
xxxvi. indacatérol et vilantérol
xxxv. vilantérol et carmotérol
xxxvi. vilantérol et bambutérol
xxxviii. carmotérol et salmétérol
xxxiv. carmotérol et formotérol
xl. carmotérol et arformotérol
xli. carmotérol et olodatérol
xlii. carmotérol et bambutérol
xliii. olodatérol et salmétérol
xliv. olodatérol et formotérol
xlv. olodatérol et arformotérol
xlvi. olodatérol et bambutérol
dans laquelle lesdits agents thérapeutiques peuvent être présents en tant que sels ou esters pharmaceutiquement acceptables de ceux-ci ou sous une forme énantiomériquement pure ou en mélange racémique.

14. Composition pharmaceutique selon la revendication 10, comprenant l'une quelconque des combinaisons suivantes, lesdites combinaisons étant appropriées à une administration séparée, séquentielle ou conjointe en quantités efficaces :
i. aclidinium et salbutamol
ii. aclidinium et lévosalbutamol
iii. aclidinium et terbutaline
iv. aclidinium et pirbutérol
v. aclidinium et procatérol
vi. aclidinium et fénotérol
vii. aclidinium et bitoltérol
viii. aclidinium et ritodrine
ix. aclidinium et métaprotérénol
x. glycopyrronium et salbutamol
xi. glycopyrronium et lévosalbutamol
xii. glycopyrronium et terbutaline
xiii. glycopyrronium et pirbutérol
xiv. glycopyrronium et procatérol
xv. glycopyrronium et fénotérol
xvi. glycopyrronium et bitoltérol
xvii. glycopyrronium et ritodrine
xviii. glycopyrronium et métaprotérénol
xix. darotropium et salbutamol
xx. darotropium et lévosalbutamol
xxi. darotropium et terbutaline
xxii. darotropium et pirbutérol
xxiii. darotropium et procatérol
xxiv. darotropium et fénotérol
xxv. darotropium et bitoltérol
xxvi. darotropium et ritodrine
xxvii. darotropium et métaprotérénol
xxviii. indacatérol et salbutamol
xxix. indacatérol et lévosalbutamol
xxx. indacatérol et terbutaline
xxxi. indacatérol et pirbutérol
xxxii. indacatérol et procatérol
xxxiii. indacatérol et fénotérol
xxxiv. indacatérol et bitoltérol
xxxv. indacatérol et ritodrine
xxxvi. indacatérol et métaprotérénol
xxxvii. vilantérol et salbutamol
xxxviii. vilantérol et lévosalbutamol
xxxix. vilantérol et terbutaline
xl. vilantérol et pirbutérol
xli. vilantérol et procatérol
xlii. vilantérol et fénotérol
xliii. vilantérol et bitoltérol
xliv. vilantérol et ritodrine
xlv. vilantérol et métaprotérénol
xlvi. carmotérol et salbutamol
xlii. carmotérol et lévosalbutamol
xlviii. carmotérol et terbutaline
xlix. carmotérol et pirbutérol
L. carmotérol et procatérol
Li. carmotérol et fénotérol
Lii. carmotérol et bitoltérol
Liii. carmotérol et ritodrine
Liv. carmotérol et métaprotérénol
Lv. olodatérol et salbutamol
Lvi. olodatérol et lévosalbutamol
Lvii. olodatérol et terbutaline
Lviii. olodatérol et pirbutérol
Lix. olodatérol et procatérol
Lx. olodatérol et fénotérol
Lxi. olodatérol et bitoltérol
Lxii. olodatérol et ritodrine
Lxiii. olodatérol et métaprotérénol
dans laquelle lesdits agents thérapeutiques peuvent être présents en tant que sels ou esters pharmaceutiquement acceptables de ceux-ci ou sous une forme énantiomériquement pure ou en mélange racémique.

15. Composition pharmaceutique selon la revendication 11, comprenant l'une quelconque des combinaisons suivantes, lesdites combinaisons étant appropriées à une administration séparée, séquentielle ou conjointe en quantités efficaces :
i. aclidinium et fluticasone
ii. aclidinium et ciclésonide
iii. aclidinium et budésonide
iv. aclidinium et mométasone
v. aclidinium et béclométhasone
vi. aclidinium et triamcinolone
vii. aclidinium et flunisolide
viii. aclidinium et dexaméthasone
ix. glycopyrronium et fluticasone
x. glycopyrronium et ciclésonide
xi. glycopyrronium et budésonide
xii. glycopyrronium et mométasone
xiii. glycopyrronium et béclométhasone
xiv. glycopyrronium et triamcinolone
xv. glycopyrronium et flunisolide
xvi. glycopyrronium et dexaméthasone
xvii. darotropium et fluticasone
xviii. darotropium et ciclésonide
xix. darotropium et budésonide
xx. darotropium et mométasone
xxi. darotropium et béclométhasone
xxii. darotropium et triamcinolone
xxiii. darotropium et flunisolide
xxiv. darotropium et dexaméthasone
xxv. indacatérol et fluticasone
xxvi. indacatérol et ciclesonide
xxvii. indacatérol et budésonide
xxviii. indacatérol et mométasone
xxix. indacatérol et béclométhasone
xxx. indacatérol et triamcinolone
xxxi. indacatérol et flunisolide
xxxii. indacatérol et dexaméthasone
xxxiii. vilantérol et fluticasone
xxxiv. vilantérol et ciclésonide
xxxv. vilantérol et budésonide
xxxvi. vilantérol et mométasone
xxxvii. vilantérol et béclométasone
xxxv. vilantérol et budésonide
xxxvi. vilantérol et mométasone
xxxvii. vilantérol et béclométhasone
xxxviii. vilantérol et triamcinolone
xxxix. vilantérol et flunisolide
xl. vilantérol et dexaméthasone
xli. carmotérol et fluticasone
xlii. carmotérol et ciclésonide
xliii. carmotérol et budésonide
xliv. carmotérol et mométasone
xlv. carmotérol et béclométhasone
xlvi. carmotérol et triamcinolone
xlvii. carmotérol et flunisolide
xlviii. carmotérol et fluticasone
L. olodatérol et ciclésonide
Li. olodatérol et budésonide
Lii. olodatérol et mométasone
Liii. olodatérol et béclaméthasone
Liv. olodatérol et triamcinolone
Lv. olodatérol et flunisolide
Lvi. olodatérol et dexaméthasone
dans laquelle lesdits agents thérapeutiques peuvent être présents en tant que sels ou esters pharmaceutiquement acceptables de ceux-ci ou sous une forme énantiomériquement pure ou en mélange racémique.

16. Composition pharmaceutique selon la revendication 12, comprenant l'une quelconque des combinaisons suivantes, lesdites combinaisons étant appropriées à une administration séparée, séquentielle ou conjointe en quantités efficaces :
i. aclidinum, tiotropium et salmétérol
ii. aclidinum, tiotropium et formotérol
iii. aclidinum, tiotropium et arformotérol
iv. aclidinum, tiotropium et indacatérol
v. aclidinum, tiotropium et olodatérol
vi. aclidinum, tiotropium et vilantérol
vii. aclidinum, tiotropium et carmotérol
viii. aclidinum, tiotropium et bambutérol
ix. aclidinum, glycopyrronium et salmétérol
x. aclidinum, glycopyrronium et formotérol
xi. aclidinum, glycopyrronium et arformotérol
xii. aclidinum, glycopyrronium et indacatérol
xiii. aclidinum, glycopyrronium et olodatérol
xiv. aclidinum, glycopyrronium et vilantérol
xv. aclidinum, glycopyrronium et carmotérol
xvi. aclidinum, glycopyrronium et bambutérol
xvii. aclidinum, oxytropium et salmétérol
xviii. aclidinum, oxytropium et formotérol
xix. aclidinum, oxytropium et arformotérol
xx. aclidinum, oxytropium et indacatérol
xxi. adidinum, oxytropium et olodatérol
xxii. adidinum, oxytropium et vilantérol
xxiii. aclidinum, oxytropium et carmotérol
xxiv. aclidinum, oxytropium et bambutérol
xxv. glycopyrronium, tiotropium et salmétérol
xxvi. glycopyrronium, tiotropium et formotérol
xxvii. glycopyrronium, tiotropium et arformotérol
xxviii. glycopyrronium, tiotropium et indacatérol
xxix. glycopyrronium, tiotropium et olodatérol
xxx. glycopyrronium, tiotropium et vilantérol
xxxi. glycopyrronium, tiotropium et carmotérol
xxxii. glycopyrronium, tiotropium et bambutérol
xxxiii. glycopyrronium, oxytropium et salmétérol
xxxiv. glycopyrronium, oxytropium et formotérol
xxxv. glycopyrronium, oxytropium et arformotérol
xxxvi. glycopyrronium, oxytropium et indacatérol
xxxvii. glycopyrronium, oxytropium et olodatérol
xxxviii. glycopyrronium, oxytropium et vilantérol
xxxix. glycopyrronium, oxytropium et carmotérol
xl. glycopyrronium, oxytropium et bambutérol
xli. daratropium, tiotropium et salmétérol
xlii. daratropium, tiotropium et formotérol
xliii. daratropium, tiotropium et arformotérol
xliv. daratropium, tiotropium et indacatérol
xlv. daratropium, tiotropium et olodatérol
xlvi. daratropium, tiotropium et vilantérol
xlvii. daratropium, tiotropium et carmotérol
xlviii. daratropium, tiotropium et bambutérol
xlix. daratropium, glycopyrronium et salmétérol
L. daratropium, glycopyrronium et formotérol
Li. daratropium, glycopyrronium et arformotérol
Lii. daratropium, glycopyrronium et indacatérol
Liii. daratropium, glycopyrronium et olodatérol
Liv. daratropium, glycopyrronium et vilantérol
Lv. daratropium, glycopyrronium et carmotérol
Lvi. daratropium, glycopyrronium et bambutérol
Lvii. daratropium, aclidinium et salmétérol
Lviii. daratropium, aclidinium et formotérol
Lix. daratropium, aclidinium et arformotérol
Lx. daratropium, aclidinium et indacatérol
Lxi. daratropium, aclidinium et olodatérol
Lxii. daratropium, aclidinium et vilantérol
Lxiii. daratropium, aclidinium et carmotérol
Lxiv. daratropium, aclidinium et bambutérol
Lxv. daratropium, oxytropium et salmétérol
Lxvi. daratropium, oxytropium et formotérol
Lxvii. daratropium, oxytropium et arformotérol
Lxviii. daratropium, oxytropium et indacatérol
Lxix. daratropium, oxytropium et olodatérol
Lxx. daratropium, oxytropium et vilantérol
Lxxii. daratropium, oxytropium et bambutérol
Lxxiii. Indacatérol, tiotropium et salmétérol
Lxxiv. Indacatérol, tiotropium et formotérol
Lxxv. Indacatérol, tiotropium et arformotérol
Lxxvi. Indacatérol, tiotropium et olodatérol
Lxxvii. Indacatérol, tiotropium et vilantérol
Lxxviii. Indacatérol, tiotropium et carmotérol
Lxxix. Indacatérol, tiotropium et bambutérol
Lxxx. Indacatérol, glycopyrronium et salmétérol
Lxxxi. Indacatérol, glycopyrronium et formotérol
Lxxxii. Indacatérol, glycopyrronium et arformotérol
Lxxxiii. Indacatérol, glycopyrronium et olodatérol
Lxxxiv. Indacatérol, glycopyrronium et vilantérol
Lxxxv. Indacatérol, glycopyrronium et carmotérol
Lxxxvi. Indacatérol, glycopyrronium et bambutérol
Lxxxvii. Indacatérol, aclidinium et salmétérol
Lxxxviii. Indacatérol, aclidinium et formotérol
Lxxxix. Indacatérol, aclidinium et arformotérol
xc. Indacatérol, aclidinium et olodatérol
xci. Indacatérol, aclidinium et vilantérol
xcii. Indacatérol, aclidinium et carmotérol
xciii. Indacatérol, aclidinium et bambutérol
xciv. Indacatérol, oxytropium et salmétérol
xcv. Indacatérol, oxytropium et formotérol
xcvi. Indacatérol, oxytropium et arformotérol
xcvii. Indacatérol, oxytropium et olodatérol
xcviii. Indacatérol, oxytropium et vilantérol
xcix. Indacatérol, oxytropium et carmotérol
c. Indacatérol, oxytropium et bambutérol
ci. vilantérol, tiotropium et salmétérol
cii. vilantérol, tiotropium et salmétérol
cii. vilantérol, tiotropium et formotérol
ciii. vilantérol, tiotropium et arformotérol
civ. vilantérol, tiotropium et indacatérol
cv. vilantérol, tiotropium et olodatérol
cvi. vilantérol, tiotropium et carmotérol
cvii. vilantérol, tiotropium et bambutérol
cviii. vilantérol, glycopyrronium et salmétérol
cix. vilantérol, glycopyrronium et formotérol
cx. vilantérol, glycopyrronium et arformotérol
cxi. vilantérol, glycopyrronium et indacatérol
cxii. vilantérol, glycopyrronium et olodatérol
cxiii. vilantérol, glycopyrronium et carmotérol
cxiv. vilantérol, glycopyrronium et bambutérol
cxv. vilantérol, aclidinium et salmétérol
cxvi. vilantérol, aclidinium et formotérol
cxvii. vilantérol, aclidinium et arformotérol
cxviii. vilantérol, aclidinium et indacatérol
cxix. vilantérol, aclidinium et olodatérol
cxx. vilantérol, aclidinium et carmotérol
cxxi. vilantérol, aclidinium et bambutérol
cxxii. vilantérol, oxytropium et salmétérol
cxxiii. vilantérol, oxytropium et formotérol
cxxiv. vilantérol, oxytropium et arformotérol
cxxv. vilantérol, oxytropium et indacatérol
cxxvi. vilantérol, oxytropium et olodatérol
cxxvii. vilantérol, oxytropium et carmotérol
cxxviii. vilantérol, oxytropium et bambutérol
cxxix. carmotérol, tiopropium et salmétérol
cxxx. carmotérol, tiopropium et formotérol
cxxxi. carmotérol, tiopropium et arformotérol
cxxxii. carmotérol, tiopropium et indacatérol
cxxxiii. carmotérol, tiopropium et alodatérol
cxxxiv. carmotérol, tiopropium et vilantérol
cxxxv. carmotérol, tiopropium et bambutérol
cxxxvi. carmotérol, glycopyrronium et salmétérol
cxxxvii. carmotérol, glycopyrronium et formotérol
cxxxviii. carmotérol, glycopyrronium et arformotérol
cxxxix. carmotérol, glycopyrronium et indacatérol
cxl. carmotérol, glycopyrronium et olodatérol
cxli. carmotérol, glycopyrronium et vilantérol
cxlii. carmotérol, aclidinium et salmétérol
cxliv. carmotérol, aclidinium et formotérol
cxlv. carmotérol, aclidinium et arformotérol
cxlvi. carmotérol, aclidinium et indacatérol
cxlvii. carmotérol, aclidinium et olodatérol
cxlviii. carmotérol, aclidinium et vilantérol
cxlix. carmotérol, aclidinium et bambutérol
cl. carmotérol, oxytropium et salmétérol
cli. carmotérol, oxytropium et formotérol
clii. carmotérol, oxytropium et arformotérol
cliii. carmotérol, oxytropium et indacatérol
cliv. carmotérol, oxytropium et olodatérol
clv. carmotérol, oxytropium et vilantérol
clvi. carmotérol, oxytropium et bambutérol
clvii. olodatérol, tiotropium et salmétérol
clviii. olodatérol, tiotropium et formotérol
clix. olodatérol, tiotropium et arformotérol
clx. olodatérol, tiotropium et indacatérol
clxi. olodatérol, tiotropium et vilantérol
clxii. olodatérol, tiotropium et bambutérol
clxiii. olodatérol, glycopyrronium et salmétérol
clxv. olodatérol, glycopyrronium et arformotérol
clxvi. olodatérol, glycopyrronium et indacatérol
clxvii. olodatérol, glycopyrronium et vilantérol
clxviii. olodatérol, glycopyrronium et bambutérol
clxix. olodatérol, aclidinium et salmétérol
clxx. olodatérol, aclidinium et formotérol
clxxi. olodatérol, aclidinium et arformotérol
clxxii. olodatérol, aclidinium et indacatérol
clxxiii. olodatérol, aclidinium et vilantérol
clxxiv. olodatérol, aclidinium et bambutérol
clxxv. olodatérol, oxytropium et salmétérol
clxxvi. olodatérol, oxytropium et formotérol
clxxvii. olodatérol, oxytropium et arformotérol
clxxviii. olodatérol, oxytropium et indacatérol
clxxix. olodatérol, oxytropium et vilantérol
clxxx. olodatérol, oxytropium et bambutérol
dans laquelle lesdits agents thérapeutiques peuvent être présents en tant que sels ou esters pharmaceutiquement acceptables de ceux-ci ou sous une forme énantiomériquement pure ou en mélange racémique.

17. Composition pharmaceutique selon la revendication 13, comprenant l'une quelconque des combinaisons suivantes, lesdites combinaisons étant appropriées à une administration séparée, séquentielle ou conjointe en quantités efficaces :
i. aclidinum, tiotropium et salbutamol
ii. aclidinum, tiotropium et lévosalbutamol
iii. aclidinum, tiotropium et terbutaline
iv. aclidinum, tiotropium et pirbutarol
v. aclidinum, tiotropium et procatérol
vi. aclidinum, tiotropium et fénotérol
vii. aclidinum, tiotropium et ritodrine
viii. aclidinum, tiotropium et bitoltérol
ix. aclidinum, tiotropium et métaprotérénol
x. aclidinum, glycopyrronium et salbutamol
xi. aclidinum, glycopyrronium et lévosalbutamol
xii. aclidinum, glycopyrronium et terbutaline
xiii. aclidinum, glycopyrronium et pirbutérol
xiv. aclidinum, glycopyrronium et procatérol
xv. aclidinum, glycopyrronium et fénotérol
xvi. aclidinum, glycopyrronium et bitoltérol
xvii. aclidinum, glycopyrronium et ritodrine
xviii. aclidinum, glycopyrronium et métaprotérénol
xix. aclidinum, ipratropium et salbutamol
xx. aclidinum, ipratropium et lévosalbutamol
xxi. aclidinum, ipratropium et terbutaline
xxii. aclidinum, ipratropium et pirbutérol
xxiii. aclidinum, ipratropium et procatérol
xxiv. aclidinum, ipratropium et fénotérol
xxv. aclidinum, ipratropium et bitoltérol
xxvi. aclidinum, ipratropium et ritodrine
xxvii. aclidinum, ipratropium et métaprotérénol
xxviii. aclidinum, oxytropium et salbutamol
xxix. aclidinum, oxytropium et lévosalbutamol
xxx. aclidinum, oxytropium et terbutaline
xxxi. aclidinum, oxytropium et pirbutérol
xxxii. aclidinum, oxytropium et procatérol
xxxiii. aclidinum, oxytropium et fénotérol
xxxiv. aclidinum, oxytropium et bitoltérol
xxxv. aclidinum, oxytropium et ritodrine
xxxvi. aclidinum, oxytropium et métaprotérénol
xxxvii. glycopyrronium, tiotropium et salbutamol
xxxviii. glycopyrronium, tiotropium et lévosalbutamol
xxxix. glycopyrronium, tiotropium et terbutaline
xl. glycopyrronium, tiotropium et pirbutérol
xli. glycopyrronium, tiotropium et procatérol
xlii. glycopyrronium, tiotropium et fénotérol
xliii. glycopyrronium, tiotropium et bitoltérol
xliv. glycopyrronium, tiotropium et ritodrine
xlv. glycopyrronium, tiotropium et métaprotérénol
xlvi. glycopyrronium, ipratropium et salbutamol
xlvii. glycopyrronium, ipratropium et lévosalbutamol
xlviii. glycopyrronium, ipratropium et terbutaline
xlix. glycopyrronium, ipratropium et pirbutérol
L. glycopyrronium, ipratropium et procatérol
Li. glycopyrronium, ipratropium et fénotérol
Lii. glycopyrronium, ipratropium et bitoltérol
Liii. glycopyrronium, ipratropium et ritodrine
Liv. glycopyrronium, ipratropium et métaprotérénol
Lv. glycopyrronium, oxytropium et salbutamol
Lvi. glycopyrronium, oxytropium et lévosalbutamol
Lvii. glycopyrronium, oxytropium et terbutaline
Lviii. glycopyrronium, oxytropium et pirbutérol
Lix. glycopyrronium, oxytropium et procatérol
Lx. glycopyrronium, oxytropium et fénotérol
Lxi. glycopyrronium, oxytropium et bitoltérol
Lxii. glycopyrronium, oxytropium et ritodrine
Lxiii. glycopyrronium, oxytropium et métaprotérénol
Lxiv. Daratropium, tiotropium et salbutamol
Lxv. Daratropium, tiotropium et lévosalbutamol
Lxvi. Daratropium, tiotropium et terbutaline
Lxvii. Daratropium, tiotropium et pirbutérol
Lxviii. Daratropium, tiotropium et procatérol
Lxix. Daratropium, tiotropium et fénotérol
Lxx. Daratropium, tiotropium et bitoltérol
Lxxi. Daratropium, tiotropium et ritodrine
Lxxii. Daratropium, tiotropium et métaprotérénol
Lxxiii. Daratropium, aclidinium et salbutamol
Lxxiv. Daratropium, aclidinium et lévosalbutamol
Lxxv. Daratropium, aclidinium et terbutaline
Lxxvi. Daratropium, aclidinium et pirbutérol
Lxxvii. Daratropium, aclidinium et procatérol
Lxxviii. Daratropium, aclidinium et fénotérol
Lxxix. Daratropium, aclidinium et bitoltérol
Lxxx. Daratropium, aclidinium et ritodrine
Lxxxi. Daratropium, aclidinium et métaprotérénol
Lxxxii. Daratropium, glycopyrronium et salbutamol
Lxxxiii. Daratropium, glycopyrronium et lévosalbutamol
Lxxxiv. Daratropium, glycopyrronium et terbutaline
Lxxxv. Daratropium, glycopyrronium et pirbutérol
Lxxxvi. Daratropium, glycopyrronium et procatérol
Lxxxvii. Daratropium, glycopyrronium et fénotérol
Lxxxviii. Daratropium, glycopyrronium et bitoltérol
Lxxxix. Daratropium, glycopyrronium et ritodrine
xc. Daratropium, glycopyrronium et métaprotérénol
xci. Daratropium, iprotropium et salbutamol
xcii. Daratropium, iprotropium et lévosalbutamol
xciii. Daratropium, iprotropium et terbutaline
xciv. Daratropium, iprotropium et pirbutérol
xcv. Daratropium, iprotropium et procatérol
xcvi. Daratropium, iprotropium et fénotérol
xcvii. Daratropium, iprotropium et bitoltérol
xcviii. Daratropium, iprotropium et ritodrine
xcix. Daratropium, iprotropium et métaprotérénol
c. Daratropium, oxytropium et salbutamol
ci. Daratropium, oxytropium et lévosalbutamol
cii. Daratropium, oxytropium et terbutaline
ciii. Daratropium, oxytropium et pirbutérol
civ. Daratropium, oxytropium et procatérol
cv. Daratropium, oxytropium et fénotérol
cvi. Daratropium, oxytropium et bitoltérol
cvii. Daratropium, oxytropium et ritodrine
cviii. Daratropium, oxytropium et métaprotérénol
cix. indacatérol, tirotropium et salbutamol
cx. indacatérol, tirotropium et lévosalbutamol
cxi. indacatérol, tirotropium et terbutaline
cxii. indacatérol, tirotropium et pirbutérol
cxiii. indacatérol, tirotropium et procatérol
cxiv. indacatérol, tirotropium et fénotérol
cxv. indacatérol, tirotropium et bitoltérol
cxvi. indacatérol, tirotropium et ritodrine
cxvii. indacatérol, tirotropium et métaprotérénol
cxviii. indacatérol, glycopyrronium et salbutamol
cxix. indacatérol, glycopyrronium et lévosalbutamol
cxx. indacatérol, glycopyrronium et terbutaline
cxxi. indacatérol, glycopyrronium et pirbutérol
cxxii. indacatérol, glycopyrronium et procatérol
cxxiii. indacatérol, glycopyrronium et fénotérol
cxxiv. indacatérol, glycopyrronium et bitoltérol
cxxv. indacatérol, glycopyrronium et ritodrine
cxxvi. indacatérol, glycopyrronium et métaprotérénol
cxxvii. indacatérol, aclidinium et salbutamol
cxxviii. indacatérol, aclidinium et lévosalbutamol
cxxix. indacatérol, aclidinium et terbutaline
cxxx. indacatérol, aclidinium et pirbutérol
cxxxi. indacatérol, aclidinium et procatérol
cxxxii. indacatérol, aclidinium et fénotérol
cxxxiii. indacatérol, aclidinium et bitoltérol
cxxxiv. indacatérol, aclidinium et ritodrine
cxxxv. indacatérol, aclidinium et métaprotérénol
cxxxvii. indacatérol, ipratropium et salbutamol
cxxxvii. indacatérol, ipratropium et lévosalbutamol
cxxxviii. indacatérol, ipratropium et terbutaline
cxxxix. indacatérol, ipratropium et pirbutérol
cxl. indacatérol, ipratropium et procatérol
cxli. indacatérol, ipratropium et fénotérol
cxlii. indacatérol, ipratropium et bitoltérol
cxliii. indacatérol, ipratropium et ritodrine
cxliv. indacatérol, ipratropium et métaprotérénol
cxlv. indacatérol, oxytropium et salbutamol
cxlvi. indacatérol, oxytropium et lévosalbutamol
cxlvii. indacatérol, oxytropium et terbutaline
cxlviii. indacatérol, oxytropium et pirbutérol
cxlix. indacatérol, oxytropium et procatérol
cl. indacatérol, oxytropium et fénotérol
cli. indacatérol, oxytropium et bitoltérol
clii. indacatérol, oxytropium et ritodrine
cliii. indacatérol, oxytropium et métaprotérénol
cliv. vilantérol, tiotropium et salbutamol
clv. vilantérol, tiotropium et lévosalbutamol
clvi. vilantérol, tiotropium et terbutaline
clvii. vilantérol, tiotropium et pirbutérol
clviii. vilantérol, tiotropium et procatérol
clix. vilantérol, tiotropium et fénotérol
clx. vilantérol, tiotropium et bitoltérol
clxi. vilantérol, tiotropium et ritodrine
clxii. vilantérol, tiotropium et métaprotérénol
clxiii. vilantérol, glycopyrronium et salbutamol
clxiv. vilantérol, glycopyrronium et lévosalbutamol
clxv. vilantérol, glycopyrronium et terbutaline
clxvi. vilantérol, glycopyrronium et pirbutérol
clxvii. vilantérol, glycopyrronium et procatérol
clxviii. vilantérol, glycopyrronium et fénotérol
clxix. vilantérol, glycopyrronium et bitoltérol
clxx. vilantérol, glycopyrronium et ritodrine
clxxi. vilantérol, glycopyrronium et métaprotérénol
clxxii. vilantérol, ipratropium et salbutamol
clxxiii. vilantérol, ipratropium et lévosalbutamol
clxxiv. vilantérol, ipratropium et terbutaline
clxxv. vilantérol, ipratropium et pirbutérol
clxxvi. vilantérol, ipratropium et procatérol
clxxvii. vilantérol, ipratropium et fénotérol
clxxviii. vilantérol, ipratropium et bitoltérol
clxxix. vilantérol, ipratropium et ritodrine
clxxx. vilantérol, ipratropium et métaprotérénol
clxxxi. vilantérol, aclidinium et salbutamol
clxxxii. vilantérol, aclidinium et lévosalbutamol
clxxxiii. vilantérol, aclidinium et terbutaline
clxxxiv. vilantérol, aclidinium et pirbutérol
clxxxv. vilantérol, aclidinium et procatérol
clxxxvi. vilantérol, aclidinium et fénotérol
clxxxvii. vilantérol, aclidinium et bitoltérol
clxxxviii. vilantérol, aclidinium et ritodrine
clxxxix. vilantérol, aclidinium et métaprotérénol
cxc. vilantérol, oxytropium et salbutamol
cxci. vilantérol, oxytropium et lévosalbutamol
cxcii. vilantérol, oxytropium et terbutaline
cxciii. vilantérol, oxytropium et pirbutérol
cxciv. vilantérol, oxytropium et procatérol
cxcv. vilantérol, oxytropium et fénotérol
cxcvi. vilantérol, oxytropium et bitoltérol
cxcvii. vilantérol, oxytropium et ritodrine
cxcviii. vilantérol, oxytropium et métaprotérénol
cxcix. carmotérol, tiotropium et salbutamol
cc. carmotérol, tiotropium et lévosalbutamol
cci. carmotérol, tiotropium et terbutaline
ccii. carmotérol, tiotropium et pirbutérol
cciii. carmotérol, tiotropium et procatérol
cciv. carmotérol, tiotropium et fénotérol
ccv. carmotérol, tiotropium et bitoltérol
ccvi. carmotérol, tiotropium et ritodrine
ccvii. carmotérol, tiotropium et métaprotérénol
ccviii. carmotérol, tiotropium et lévosalbutamol
ccix. carmotérol, ipratropium et salbutamol
ccx. carmotérol, ipratropium et terbutaline
ccxi. carmotérol, ipratropium et pirbutérol
ccxii. carmotérol, ipratropium et procatérol
ccxiii. carmotérol, ipratropium et fénotérol
ccxiv. carmotérol, ipratropium et bitoltérol
ccxv. carmotérol, ipratropium et ritodrine
ccxvi. carmotérol, ipratropium et métaprotérénol
ccxvii. carmotérol, aclidinum et lévosalbutamol
ccxviii. carmotérol, aclidinum et salbutamol
ccxix. carmotérol, aclidinum et terbutaline
ccxx. carmotérol, aclidinum et pirbutérol
ccxxi. carmotérol, aclidinum et procatérol
ccxxii. carmotérol, aclidinum et fénotérol
ccxxiii. carmotérol, aclidinum et bitoltérol
ccxxiv. carmotérol, aclidinum et ritodrine
ccxxv. carmotérol, aclidinum et métaprotérénol
ccxxvi. carmotérol, oxytropium et salbutamol
ccxxvii. carmotérol, oxytropium et lévosalbutamol
ccxxviii. carmotérol, oxytropium et terbutaline
ccxxix. carmotérol, oxytropium et pirbutérol
ccxxx. carmotérol, oxytropium et procatérol
ccxxxi. carmotérol, oxytropium et fénotérol
ccxxxii. carmotérol, oxytropium et bitoltérol
ccxxxiii. carmotérol, oxytropium et ritodrine
ccxxxix. carmotérol, oxytropium et métaprotérénol
ccxxxv. olodatérol, oxytropium et salbutamol
ccxxxvi. olodatérol, oxytropium et lévosalbutamol
ccxxxvii. olodatérol, oxytropium et terbutaline
ccxxxviii. olodatérol, oxytropium et pirbutérol
ccxxxix. olodatérol, tiotropium et procatérol
ccxl. olodatérol, tiotropium et fénotérol
ccxli. olodatérol, tiotropium et bitoltérol
ccxlii. olodatérol, tiotropium et ritodrine
ccxliii. olodatérol, tiotropium et métaprotérénol
ccxliv. olodatérol, ipratropium et salbutamol
ccxlv. olodatérol, ipratropium et lévosalbutamol
ccxlvi. olodatérol, ipratropium et terbutaline
ccxlvii. olodatérol, ipratropium et pirbutérol
ccxlviii. olodatérol, ipratropium et procatérol
ccxlix. olodatérol, ipratropium et fénotérol
ccl. olodatérol, ipratropium et bitoltérol
ccli. olodatérol, ipratropium et ritodrine
cclii. olodatérol, ipratropium et métaprotérénol
ccliii. olodatérol, aclidinum et salbutamol
ccliv. olodatérol, aclidinum et lévolsalbutamol
cclv. olodatérol, aclidinum et terbutaline
cclvi. olodatérol, aclidinum et pirbutérol
cclvii. olodatérol, aclidinum et procatérol
cclviii. olodatérol, aclidinum et fénotérol
cclix. olodatérol, aclidinum et bitoltérol
cclx. olodatérol, aclidinum et ritodrine
cclxi. olodatérol, aclidinum et métaprotérénol
cclxii. olodatérol, glycopyrronium et salbutamol
cclxiii. olodatérol, glycopyrronium et lévosalbutamol
cclxiv. olodatérol, glycopyrronium et terbutaline
cclxv. olodatérol, glycopyrronium et pirbutérol
cclxvi. olodatérol, glycopyrronium et procatérol
cclxvii. olodatérol, glycopyrronium et fénotérol
cclxviii. olodatérol, glycopyrronium et bitoltérol
cclxix. olodatérol, glycopyrronium et ritodrine
cclxx. olodatérol, glycopyrronium et métaprotérénol
cclxxi. olodatérol, oxytropium et salbutamol
cclxxii. olodatérol, oxytropium et lévosalbutamol
cclxxiii. olodatérol, oxytropium et terbutaline
cclxxiv. olodatérol, oxytropium et pirbutérol
cclxxv. olodatérol, oxytropium et procatérol
cclxxvi. olodatérol, oxytropium et fénotérol
cclxxvii. olodatérol, oxytropium et bitoltérol
cclxxviii. olodatérol, oxytropium et ritodrine
cclxxix. olodatérol, oxytropium et métaprotérénol
dans laquelle lesdits agents thérapeutiques peuvent être présents en tant que sels ou esters pharmaceutiquement acceptables de ceux-ci ou sous une forme énantiomériquement pure ou en mélange racémique.

18. Composition pharmaceutique selon la revendication 14, comprenant l'une quelconque des combinaisons suivantes, lesdites combinaisons étant appropriées à une administration séparée, séquentielle ou conjointe en quantités efficaces :
i. aclidinum, tiotropium et fluticasone
ii. aclidinum, tiotropium et ciclésonide
iii. aclidinum, tiotropium et budésonide
iv. aclidinum, tiotropium et mométasone
v. aclidinum, tiotropium et béclométhasone
vi. aclidinum, tiotropium et triamcinolone
vii. aclidinum, tiotropium et flunisolide
viii. aclidinum, tiotropium et dexométhasone
ix. daratropium, tiotropium et fluticasone
x. daratropium, tiotropium et ciclésonide
xi. daratropium, tiotropium et budésonide
xii. daratropium, tiotropium et mométasone
xiii. daratropium, tiotropium et béclamétasone
xiv. daratropium, tiotropium et triamcinolone
xv. daratropium, tiotropium et flunisolide
xvi. daratropium, tiotropium et dexométhasone
xvii. indacatérol, tiotropium et fluticasone
xviii. indacatérol, tiotropium et budésonide
xix. indacatérol, tiotropium et ciclésonide
xx. indacatérol, tiotropium et mométasone
xxi. indacatérol, tiotropium et béclaméthasone
xxii. indacatérol, tiotropium et triamcinolone
xxiii. indacatérol, tiotropium et flunisolide
xxiv. indacatérol, tiotropium et dexométhasone
xxv. vilantérol, tiotropium et ciclésonide
xxvi. vilantérol, tiotropium et fluticasone
xxvii. vilantérol, tiotropium et budésonide
xxviii. vilantérol, tiotropium et mométasone
xxix. vilantérol, tiotropium et béclaméthasone
xxx. vilantérol, tiotropium et triamcinolone
xxxi. vilantérol, tiotropium et flunisolide
xxxii. vilantérol, tiotropium et dexométhasone
xxxiii. carmotérol, tiotropium et budésonide
xxxiv. carmotérol, tiotropium et ciclésonide
xxxv. carmotérol, tiotropium et fluticasone
xxxvi. carmotérol, tiotropium et mométasone
xxxvii. carmotérol, tiotropium et béclaméthasone
xxxviii. carmotérol, tiotropium et triamcinolone
xxxix. carmotérol, tiotropium et flunisolide
xl. carmotérol, tiotropium et dexométhasone
xli. olodatérol, tiotropium et ciclésonide
xlii. olodatérol, tiotropium et fluticasone
xliii. olodatérol, tiotropium et budésonide
xliv. olodatérol, tiotropium et mométasone
xlv. olodatérol, tiotropium et béclaméthasone
xlvi. olodatérol, tiotropium et triamcinolone
xlvii. olodatérol, tiotropium et flunisolide
xlviii. olodatérol, tiotropium et déxométhasone
dans laquelle lesdits agents thérapeutiques peuvent être présents en tant que sels ou esters pharmaceutiquement acceptables de ceux-ci ou sous une forme énantiomériquement pure ou en mélange racémique.

19. Composition pharmaceutique selon la revendication 15, comprenant l'une quelconque des combinaisons suivantes, lesdites combinaisons étant appropriées à une administration séparée, séquentielle ou conjointe en quantités efficaces :
i. aclidnium, salmétérol et salbutamol
ii. aclidnium, salmétérol et lévosalbutamol
iii. aclidnium, formotérol et salbutamol
iv. aclidnium, formotérol et lévosalbutamol
v. aclidnium, arformotérol et salbutamol
vi. aclidnium, arformotérol et lévosalbutamol
vii. aclidnium, indacatérol et salbutamol
viii. aclidnium, indacatérol et lévosalbutamol
ix. aclidnium, olodatéral et salbutamol
x. aclidnium, olodatéral et lévosalbutamol
xi. aclidnium, vilantérol et salbutamol
xii. aclidnium, vilantérol et lévosalbutamol
xiii. aclidnium, carmotérol et salbutamol
xiv. aclidnium, carmotérol et lévosalbutamol
xv. aclidnium, bambutérol et salbutamol
xvi. aclidnium, bambutérol et lévosalbutamol
xvii. glycopyrronium, indacatérol et salbutamol
xviii. glycopyrronium, indacatérol et lévosalbutamol
xix. glycopyrronium, salmétérol et salbutamol
xx. glycopyrronium, salmétérol et lévosalbutamol
xxi. glycopyrronium, formotérol et salbutamol
xxii. glycopyrronium, formotérol et lévosalbutamol
xxiii. glycopyrronium, arformotérol et salbutamol
xxiv. glycopyrronium, arformotérol et lévosalbutamol
xxv. glycopyrronium, carmotérol et salbutamol
xxvi. glycopyrronium, carmotérol et lévosalbutamo
xxvii. glycopyrronium, olodatérol et salbutamol
xxviii. glycopyrronium, olodatérol et lévosalbutamol
xxix. glycopyrronium, vilantérol et salbutamol
xxx. glycopyrronium, vilantérol et lévosalbutamol
xxxi. glycopyrronium, bambutérol et salbutamol
xxxii. glycopyrronium, bambutérol et lévosalbutamol
xxxiii. daratropium, indacatérol et salbutamol
xxxiv. daratropium, indacatérol et lévosalbutamol
xxxv. daratropium, salmétérol et salbutamol
xxxvi. daratropium, salmétérol et lévosalbutamol
xxxvii. daratropium, formotérol et salbutamol
xxxviii. daratropium, formotérol et lévosalbutamol
xxxix. daratropium, carmotérol et salbutamol
xl. daratropium, carmotérol et lévosalbutamol
xli. daratropium, olodatérol et salbutamol
xlii. daratropium, olodatérol et lévosalbutamol
xliii. daratropium, vilantérol et salbutamol
xliv. daratropium, vilantérol et lévosalbutamol
xlv. daratropium, bambutérol et salbutamol
xlvi. daratropium, bambutérol et lévosalbutamol
xlvii. daratropium, arformotérol et salbutamol
xlviii. daratropium, arformotérol et lévosalbutamol
dans laquelle lesdits agents thérapeutiques peuvent être présents en tant que sels ou esters pharmaceutiquement acceptables de ceux-ci ou sous une forme énantiomériquement pure ou en mélange racémique.

20. Composition pharmaceutique selon la revendication 16, comprenant l'une quelconque des combinaisons suivantes, lesdites combinaisons étant appropriées à une administration séparée, séquentielle ou conjointe en quantités efficaces :
i. aclidinium, salmétérol et mométasone
ii. aclidinium, salmétérol et fluticasone
iii. aclidinium, salmétérol et budésonide
iv. aclidinium, formotérol et mométasone
v. aclidinium, formotérol et fluticasone
vi. aclidinium, formotérol et budésonide
vii. aclidinium, arformotérol et mométasone
viii. aclidinium, arformotérol et fluticasone
ix. aclidinium, arformotérol et budésonide
x. aclidinium, indacatérol et mométasone
xi. aclidinium, indacatérol et fluticasone
xii. aclidinium, indacatérol et budésonide
xiii. aclidinium, olodatérol et mométasone
xiv. aclidinium, olodatérol et fluticasone
xv. aclidinium, olodatérol et budésonide
xvi. aclidinium, vilantérol et mométasone
xvii. aclidinium, vilantérol et fluticasone
xviii. aclidinium, vilantérol et budésonide
xix. aclidinium, carmotérol et mométasone
xx. aclidinium, carmotérol et fluticasone
xxi. aclidinium, carmotérol et budésonide
xxii. aclidinium, bambutérol et mométasone
xxiii. aclidinium, bambutérol et fluticasone
xxiv. aclidinium, bambutérol et budésonide
xxv. glycopyrronium, indacatérol et mométasone
xxvi. glycopyrronium, indacatérol et fluticasone
xxvii. glycopyrronium, indacatérol et budésonide
xxviii. glycopyrronium, salmétérol et mométasone
xxix. glycopyrronium, salmétérol et fluticasone
xxx. glycopyrronium, salmétérol et budésonide
xxxi. glycopyrronium, formotérol et mométasone
xxxii. glycopyrronium, formotérol et fluticasone
xxxiii. glycopyrronium, formotérol et budésonide
xxxiv. glycopyrronium, arformotérol et mométasone
xxxv. glycopyrronium, arformotérol et fluticasone
xxxvi. glycopyrronium, arformotérol et budésonide
xxxvii. glycopyrronium, carmotérol et mométasone
xxxviii. glycopyrronium, carmotérol et fluticasone
xxxix. glycopyrronium, carmotérol et budésonide
xl. glycopyrronium, olodatérol et mométasone
xli. glycopyrronium, olodatérol et fluticasone
xlii. glycopyrronium, olodatérol et budésonide
xliii. glycopyrronium, vilantérol et mométasone
xliv. glycopyrronium, vilantérol et fluticasone
xlv. glycopyrronium, vilantérol et budésonide
xlvi. glycopyrronium, bambutérol et mométasone
xlvii. glycopyrronium, bambutérol et fluticasone
xlviii. glycopyrronium, bambutérol et budésonide
xlix. daratropium, indacatérol et mométasone
l. daratropium, indacatérol et fluticasone
li. daratropium, indacatérol et budésonide
lii. daratropium, salmétérol et mométasone
liii. daratropium, salmétérol et fluticasone
liv. daratropium, salmétérol et budésonide
lv. daratropium, formétérol et mométasone
lvi. daratropium, formétérol et fluticasone
lvii. daratropium, formétérol et budésonide
lviii. daratropium, carmotérol et mométasone
lix. daratropium, carmotérol et fluticasone
lx. daratropium, carmotérol et budésonide
lxi. daratropium, olodatérol et mométasone
lxii. daratropium, olodatérol et fluticasone
lxiii. daratropium, olodatérol et budésonide
lxiv. daratropium, vilantérol et mométasone
lxv. daratropium, vilantérol et fluticasone
lxvi. daratropium, vilantérol et budésonide
lxvii. daratropium, bambutérol et mométasone
lxviii. daratropium, bambutérol et fluticasone
lxix. daratropium, bambutérol et budésonide
lxx. daratropium, arformotérol et mométasone
lxxi. daratropium, arformotérol et fluticasone
lxxii. daratropium, arformotérol et budésonide
lxxiii. indacatérol, salmétérol et mométasone
lxxiv. indacatérol, salmétérol et fluticasone
lxxv. indacatérol, salmétérol et budésonide
lxxvi. indacatérol, formotérol et mométasone
lxxvii. indacatérol, formotérol et fluticasone
lxxviii. indacatérol, formotérol et budésonide
lxxix. indacatérol, arformotérol et mométasone
lxxx. indacatérol, arformotérol et fluticasone
lxxxi. indacatérol, arformotérol et budésonide
lxxxii. indacatérol, olodatérol et mométasone
lxxxiii. indacatérol, olodatérol et fluticasone
lxxxiv. indacatérol, olodatérol et budésonide
lxxxv. indacatérol, vilantérol et mométasone
lxxxvi. indacatérol, vilantérol et fluticasone
lxxxvii. indacatérol, vilantérol et budésonide
lxxxviii. indacatérol, carmétérol et mométasone
lxxxix. indacatérol, carmétérol et fluticasone
xc. indacatérol, carmétérol et budésonide
xci. indacatérol, bambutérol et mométasone
xcii. indacatérol, bambutérol et fluticasone
xciii. indacatérol, bambutérol et budésonide
xciv. vilantérol, salmétérol et mométasone
xcv. vilantérol, salmétérol et fluticasone
xcvi. vilantérol, salmétérol et budésonide
xcvii. vilantérol, formotérol et mométasone
xcviii. vilantérol, formotérol et fluticasone
xcix. vilantérol, formotérol et budésonide
c. vilantérol, arformotérol et mométasone
ci. vilantérol, arformotérol et fluticasone
cii. vilantérol, arformotérol et budésonide
ciii. vilantérol, olodatérol et mométasone
civ. vilantérol, olodatérol et fluticasone
cv. vilantérol, olodatérol et budésonide
cvi. vilantérol, carmétérol et mométasone
cvii. vilantérol, carmétérol et fluticasone
cviii. vilantérol, carmétérol et budésonide
cix. vilantérol, bambutérol et mométasone
cx. vilantérol, bambutérol et fluticasone
cxi. vilantérol, bambutérol et budésonide
cxii. vilantérol, indacatérol et mométasone
cxiii. vilantérol, indacatérol et fluticasone
cxiv. vilantérol, indacatérol et budésonide
cxv. carmétérol, salmétérol et mométasone
cxvi. carmétérol, salmétérol et fluticasone
cxvii. carmétérol, salmétérol et budésonide
cxviii. carmétérol, formotérol et mométasone
cxix. carmétérol, formotérol et fluticasone
cxx. carmétérol, formotérol et budésonide
cxxi. carmétérol, arformotérol et mométasone
cxxii. carmétérol, arformotérol et fluticasone
cxxiii. carmétérol, arformotérol et budésonide
cxxiv. carmétérol, indacétérol et mométasone
cxxv. carmétérol, indacétérol et fluticasone
cxxvi. carmétérol, indacétérol et budésonide
cxxvii. carmétérol, olodatérol et mométasone
cxxviii. carmétérol, olodatérol et fluticasone
cxxix. carmétérol, olodatérol et budésonide
cxxx. carmétérol, vilantérol et mométasone
cxxxi. carmétérol, vilantérol et fluticasone
cxxxii. carmétérol, vilantérol et budésonide
cxxxiii. carmétérol, bambutérol et mométasone
cxxxiv. carmétérol, bambutérol et fluticasone
cxxxv. carmétérol, bambutérol et budésonide
cxxxvi. colodatérol, salmétérol et mométasone
cxxxvii.colodatérol, salmétérol et fluticasone
cxxxviii. colodatérol, salmétérol et budésonide
cxxxix. olodatérol, formotérol et mométasone
cxl. olodatérol, formotérol et fluticasone
cxli. olodatérol, formotérol et budésonide
cxlii. olodatérol, arformotérol et mométasone
cxliii. olodatérol, arformotérol et fluticasone
cxliv. olodatérol, arformotérol et budésonide
cxlv. olodatérol, indacatérol et mométasone
cxlvi. olodatérol, indacatérol et fluticasone
cxlvii. olodatérol, indacatérol et budésonide
cxlviii. olodatérol, vilantérol et mométasone
cxlix. olodatérol, vilantérol et fluticasone
cl. olodatérol, vilantérol et budésonide
cli. olodatérol, carmétérol et mométasone
clii. olodatérol, carmétérol et fluticasone
cliii. olodatérol, carmétérol et budésonide
cliv. olodatérol, bambutérol et mométasone
clv. olodatérol, bambutérol et mométasone
clvi. olodatérol, bambutérol et budésonide
dans laquelle lesdits agents thérapeutiques peuvent être présents en tant que sels ou esters pharmaceutiquement acceptables de ceux-ci ou sous une forme énantiomériquement pure ou en mélange racémique.

21. Composition pharmaceutique selon la revendication 17, comprenant l'une quelconque des combinaisons suivantes, lesdites combinaisons étant appropriées à une administration séparée, séquentielle ou conjointe en quantités efficaces :
i. aclidinium, salbutamol et fluticasone
ii. aclidinium, lévosalbutamol et fluticasone
iii. aclidinium, salbutamol et ciclésonide
iv. aclidinium, lévosalbutamol et ciclésonide
v. aclidinium, salbutamol et budésonide
vi. aclidinium, lévosalbutamol et budésonide
vii. aclidinium, salbutamol et mométasone
viii. aclidinium, lévosalbutamol et mométasone
ix. aclidinium, salbutamol et béclométasone
x. aclidinium, lévosalbutamol et béclométasone
xi. aclidinium, salbutamol et triamcinolone
xii. aclidinium, lévosalbutamol et triamcinolone
xiii. aclidinium, salbutamol et flunisolide
xiv. aclidinium, lévosalbutamol et flunisolide
xv. aclidinium, salbutamol et dexaméthasone
xvi. aclidinium, lévosalbutamol et dexaméthasone
xvii. glycopyrronium, salbutamol et fluticasone
xviii. glycopyrronium, lévosalbutamol et fluticasone
xix. glycopyrronium, salbutamol et ciclésonide
xx. glycopyrronium, lévosalbutamol et ciclésonide
xxi. glycopyrronium, salbutamol et budésonide
xxii. glycopyrronium, lévosalbutamol et budésonide
xxiii. glycopyrronium, salbutamol et mométasone
xxiv. glycopyrronium, lévosalbutamol et mométasone
xxv. glycopyrronium, salbutamol et béclométhasone
xxvi. glycopyrronium, lévosalbutamol et béclométhasone
xxvii. glycopyrronium, salbutamol et triamcinolone
xxviii. glycopyrronium, lévosalbutamol et triamcinolone
xxix. glycopyrronium, salbutamol et flunisolide
xxx. glycopyrronium, lévosalbutamol et flunisolide
xxxi. glycopyrronium, salbutamol et dexaméthasone
xxxii. glycopyrronium, lévosalbutamol et dexaméthasone
xxxiii. daratropium, salbutamol et fluticasone
xxxiv. daratropium, lévosalbutamol et fluticasone
xxxv. daratropium, salbutamol et ciclésonide
xxxvi. daratropium, lévosalbutamol et ciclésonide
xxxvii. daratropium, salbutamol et budésonide
xxxviii. daratropium, lévosalbutamol et budésonide
xxxix. daratropium, salbutamol et mométasone
xl. daratropium, lévosalbutamol et mométasone
xli. daratropium, salbutamol et béclométhasone
xlii. daratropium, lévosalbutamol et béclométhasone
xliii. daratropium, salbutamol et triamcinolone
xliv. daratropium, lévosalbutamol et triamcinolone
xlv. daratropium, salbutamol et flunisolide
xlvi. daratropium, lévosalbutamol et flunisolide
xlvii. daratropium, salbutamol et dexaméthasone
xlviii. daratropium, lévosalbutamol et dexaméthasone
xlix. indacatérol, salbutamol et fluticasone
l. indacatérol, lévosalbutamol et fluticasone
li. indacatérol, salbutamol et ciclésonide
lii. indacatérol, lévosalbutamol et ciclésonide
liii. indacatérol, salbutamol et budésonide
liv. indacatérol, lévosalbutamol et budésonide
lv. indacatérol, salbutamol et mométasone
lvi. indacatérol, lévosalbutamol et mométasone
lvii. indacatérol, salbutamol et béclométhasone
lviii. indacatérol, lévosalbutamol et béclométhasone
lix. indacatérol, salbutamol et triamcinolone
lx. indacatérol, lévosalbutamol et triamcinolone
lxi. indacatérol, salbutamol et flunisolide
lxii. indacatérol, lévosalbutamol et flunisolide
lxiii. indacatérol, salbutamol et dexaméthasone
lxiv. indacatérol, lévosalbutamol et dexaméthasone
lxv. vilantérol, salbutamol et fluticasone
lxvi. vilantérol, lévosalbutamol et fluticasone
lxvii. vilantérol, salbutamol et budésonide
lxviii. vilantérol, lévosalbutamol et budésonide
lxix. vilantérol, salbutamol et ciclésonide
lxx. vilantérol, lévosalbutamol et ciclésonide
lxxi. vilantérol, salbutamol et mométasone
lxxii. vilantérol, lévosalbutamol et mométasone
lxxiii. vilantérol, salbutamol et béclométhasone
lxxiv. vilantérol, lévosalbutamol et béclométhasone
lxxv. vilantérol, salbutamol et triamcinolone
lxxvi. vilantérol, lévosalbutamol et triamcinolone
lxxvii. vilantérol, salbutamol et flunisolide
lxxviii. vilantérol, lévosalbutamol et flunisolide
lxxix. vilantérol, salbutamol et dexaméthasone
lxxx. vilantérol, lévosalbutamol et dexaméthasone
lxxxi. carmétérol, salbutamol et fluticasone
lxxxii. carmétérol, lévosalbutamol et fluticasone
lxxxiii. carmétérol, salbutamol et ciclésonide
lxxxiv. carmétérol, lévosalbutamol et ciclésnide
lxxxv. carmétérol, salbutamol et budésonide
lxxxvi. carmétérol, lévosalbutamol et budésnide
lxxxvii. carmétérol, salbutamol et moméasone
lxxxviii.carmétérol, lévosalbutamol et mométasone
lxxxix. carmétérol, salbutamol et béclométhasone
xc. carmétérol, lévosalbutamol et béclométhasone
xci. carmétérol, salbutamol et triamcinolone
xcii. carmétérol, lévosalbutamol et triamcinolone
xciii. carmétérol, salbutamol et flunisolide
xciv. carmétérol, lévosalbutamol et flunisolide
xcv. carmétérol, salbutamol et dexaméthasone
xcvi. carmétérol, lévosalbutamol et dexaméthasone
xcvii. olodatérol, salbutamol et fluticasone
xcviii. olodatérol, lévosalbutamol et fluticasone
xcix. olodatérol, salbutamol et ciclésonide
c. olodatérol, lévosalbutamol et ciclésonide
ci. olodatérol, salbutamol et budésonide
cii. olodatérol, lévosalbutamol et budésonide
ciii. olodatérol, salbutamol et mométasone
civ. olodatérol, lévosalbutamol et mométasone
cv. olodatérol, salbutamol et béclométhasone
cvi. olodatérol, lévosalbutamol et béclométhasone
cvii. olodatérol, salbutamol et triamcinolone
cviii. olodatérol, lévosalbutamol et triamcinolone
cix. olodatérol, salbutamol et flunisolide
cx. olodatérol, lévosalbutamol et flunisolide
cxi. olodatérol, salbutamol et dexaméthasone
cxii. olodatérol, lévosalbutamol et dexaméthasone
dans laquelle lesdits agents thérapeutiques peuvent être présents en tant que sels ou esters pharmaceutiquement acceptables de ceux-ci ou sous une forme énantiomériquement pure ou en mélange racémique.

22. Composition pharmaceutique selon les revendications 12 à 17, comprenant l'une quelconque des combinaisons suivantes, lesdites combinaisons étant appropriées à une administration séparée, séquentielle ou conjointe en quantités efficaces :
i. formotérol, budésonide et tiotropium
ii. salmétérol, fluticasone et tiotropium
iii. carmotérol, tiotropium et fluticasone
iv. salbutamol, formotérol et budésonide
v. salbutamol, salmétérol et fluticasone
vi. salbutamol, arformotérol et fluticasone
dans laquelle lesdits agents thérapeutiques peuvent être présents en tant que sels ou esters pharmaceutiquement acceptables de ceux-ci ou sous une forme énantiomériquement pure ou en mélange racémique.

23. Composition pharmaceutique selon l'une quelconque des revendications précédentes, dans laquelle la quantité thérapeutiquement efficace de ladite composition pharmaceutique est administrée une fois par jour ou ladite composition pharmaceutique est administrée deux fois par jour.

24. Composition pharmaceutique selon l'une quelconque des revendications précédentes, pour son utilisation dans le traitement d'une affection respiratoire choisie parmi l'asthme et une maladie pulmonaire obstructive chronique et autres maladies obstructives des voies respiratoires.

25. Composition pharmaceutique selon l'une quelconque des revendications précédentes, ladite composition pharmaceutique étant appropriée à une administration séparée, séquentielle ou conjointe dans des quantités efficaces, conjointement avec un blister scellé formant une barrière étroite et élevée contre l'humidité.

26. Composition pharmaceutique selon l'une quelconque des revendications précédentes, ladite composition pharmaceutique étant appropriée à une administration séparée, séquentielle ou conjointe dans des quantités efficaces, conjointement avec une capsule.

27. Composition pharmaceutique selon la revendication 25 ou 26, ladite composition pharmaceutique étant appropriée à une administration séparée, séquentielle ou conjointe dans des quantités efficaces, conjointement avec un dispositif pour inhalation de poudre sèche.

28. Composition pharmaceutique selon la revendication 25, ladite composition pharmaceutique étant appropriée à une administration séparée, séquentielle ou conjointe dans des quantités efficaces, conjointement avec un dispositif d'inhalation, **caractérisée en ce que** ledit dispositif comprend au moins un mécanisme de verrouillage permettant au dispositif de rester verrouillé dans les deux positions dans lesquelles le dispositif est prêt pour l'inhalation et le couvercle est en position fermée, et permettant en outre au dispositif de se réamorcer automatiquement lorsque le couvercle est fermé.

29. Kit pharmaceutique comprenant les médicaments ayant une amine tels que définis dans la revendication 1 et un ou plusieurs agents actifs supplémentaires tels que définis dans la revendication 7 sous la forme d'une poudre sèche en mélange avec un véhicule pharmaceutiquement acceptable, autre que le lactose tel que défini dans la revendication 1, en formes galéniques unitaires séparées, lesdites formes étant appropriées à une administration séparée, séquentielle ou conjointe dans des quantités efficaces, conjointement avec un ou plusieurs dispositifs d'inhalation pour l'administration de médicaments ayant une amine et un ou plusieurs agents actifs supplémentaires.
